# EUROPEAN PATENT SPECIFICATION

(11) **EP 2 586 764 B1**
(45) Date of publication and mention of the grant of the patent: **18.09.2019**
(21) Application number: 11798028.4
(22) Date of filing: 15.06.2011
(51) Int. Cl.: C07C 43/225, C09K 19/12, C09K 19/14, C09K 19/16, C09K 19/18, C09K 19/20, C09K 19/32, C09K 19/34, C09K 19/54, G02F 1/13, G02F 1/1334, C09K 19/44, C09K 19/58, G02F 1/1343, C09K 19/04

(54) **COMPOUND HAVING BRANCHED ALKYL OR BRANCHED ALKENYL, OPTICALLY ISOTROPIC LIQUID CRYSTAL MEDIUM AND OPTICAL ELEMENT**
VERBINDUNG MIT EINEM VERZWEIGTEN ALKYL ODER EINEM VERZWEIGTEN ALKENYL, OPTISCH ISOTROPES FLÜSSIGKRISTALLMEDIUM UND OPTISCHES ELEMENT
COMPOSÉ PRÉSENTANT UN GROUPE ALKYLE RAMIFIÉ OU UN GROUPE ALCÉNYLE RAMIFIÉ, MILIEU DE CRISTAUX LIQUIDES ISOTROPES ET ÉLÉMENT OPTIQUE

(30) Priority: 22.06.2010 JP 2010141450
(43) Date of publication of application: 01.05.2013
(73) Proprietor: JNC Corporation, Chiyoda-ku Tokyo 100-8105 (JP); JNC Petrochemical Corporation, Tokyo 100-0004 (JP)
(72) Inventor: SAGOU, Kouki, Ichihara-shi Chiba 290-8551 (JP); HASEBA, Yasuhiro, Ichihara-shi Chiba 290-8551 (JP)
(74) Representative: Thurston, Joanna
(86) International application number: PCT/JP2011/063700
(87) International publication number: WO 2011/162142

(56) References cited:
- WO-A1-96/37451
- WO-A1-2005/080529
- WO-A1-2010/058681
- DE-A1- 10 251 016
- JP-A- 9 221 435
- JP-A- 2007 291 046
- JP-A- 2007 291 046

## Description

### BACKGROUND OF THE INVENTION

### Technical Field

The invention relates to a liquid crystal compound and a liquid crystal medium useful as materials for optical elements, particularly a liquid crystal compound having a large dielectric anisotropy, a large optical anisotropy and a low melting point, and a liquid crystal medium having a broad temperature range of a liquid crystal phase, a large dielectric anisotropy and a large optical anisotropy. The invention also relates to an optical element utilizing the liquid crystal medium, particularly an optical element that can be used in a broad temperature range and driven at a low voltage and can produce a rapid electrooptical response.

### Description of Related Art

Liquid crystal display (LCD) elements using liquid crystal compositions are widely used for display of clocks, calculators, word processors and so on. These LCD elements utilize the optical anisotropy and the dielectric anisotropy of liquid crystal compounds. The operation modes of LCD elements mainly include phase change (PC), twisted nematic (TN), super twisted nematic (STN), bistable twisted nematic (BTN), electrically controlled birefringence (ECB), optically compensated bend (OCB), in-plane switching (IPS), vertical alignment (VA) and so on, which use one or more polarizers for display purposes. Also, many studies have recently been done to the mode where an electric field is applied to an optically isotropic liquid crystal phase to induce electric birefringence (Patent Documents 1-16 and Non-Patent Documents 1-3).

Moreover, wavelength tuneable filters, wavefront control elements, liquid crystal lenses, aberrational correction elements, aperture control elements, optical head elements and so on that utilize the electric birefringence of a blue phase as one of the optically isotropic liquid crystal phases have been proposed (Patent Documents 10-12).

According to the driving mode, LCD elements can be classified into passive matrix (PM) and active matrix (AM) types. The PM type is further classified into static type, multiplex type and so on, and the AM type is classified into thin film transistor (TFT) type and metal insulator metal (MIM) type, etc.

Each of these LCD elements contains a liquid crystal composition with suitable properties. To improve the characteristics of an LCD element, it is preferred that the liquid crystal composition has suitable properties. General properties necessary for a liquid crystal compound as a component of a liquid crystal composition include:
1) chemical and physical stability,
2) a high clearing point (liquid crystal phase-isotropic phase transition temperature),
3) a low lower-limit temperature of liquid crystal phase (nematic phase, cholesteric phase, smectic phase, and optically isotropic liquid crystal phases like blue phase, etc.),
4) good compatibility with other liquid crystal compounds,
5) a suitable dielectric anisotropy, and
6) a suitable optical anisotropy.

Particularly, in view of lowering the driving voltage, a liquid crystal compound having large dielectric anisotropy and optical anisotropy is preferred for an optically isotropic liquid crystal phase.

When a liquid crystal composition including a liquid crystal compound with chemical and physical stability (the 1^{st} property) is used in an LCD element, the voltage holding ratio can be improved.

In addition, a liquid crystal composition including a liquid crystal compound having a high clearing point or a low lower-limit temperature of a liquid crystal phase (2^{nd} and 3^{rd} properties) can have a broad temperature range of nematic phase or optically isotropic liquid crystal phase, and therefore can be used in display elements in a broad temperature range. To exhibit better properties that are difficult to exhibit by a single compound, a liquid crystal compound is usually mixed with a number of other liquid crystal compounds to prepare a liquid crystal composition for use. Therefore, a liquid crystal compound used in an LCD element preferably has good compatibility with other liquid crystal compounds (4^{th} property). In recent years, LCD elements with superior properties, especially display performances like contrast, display capacity, response time and so on, are required. In addition, regarding the used liquid crystal material, a liquid crystal composition with a low driving voltage is required. Also, in order to drive, at a low voltage, an optical element driven in an optically isotropic liquid crystal phase, it is preferred to use a liquid crystal compound with large dielectric anisotropy and optical anisotropy.

Patent Document 14 reported that a compound having straight alkyl, which is similar to the compound of this application, has a large dielectric anisotropy and a large optical anisotropy. However, the compound described in Patent Document 14 exhibits a high melting point. Therefore, for a composition using the compound described in Patent Document 14, the compatibility with other liquid crystal compounds is not sufficient at low temperatures, and the amount of the compound used is limited.

On the other hand, the compound of the invention, which has branched alkyl and branched alkenyl, has a large dielectric anisotropy and a large optical anisotropy, and also features the exhibition of a quite low melting point as compared to the compound described in Patent Document 14. For example, when the compounds (ref. 01) and (ref. 02) described in Patent Document 14 are compared, with respect to the melting point, with the compound (S1) of the invention having a similar skeleton, the melting point of the compound of the invention is lower by about 20°C.

### (Comparative Compound 1)

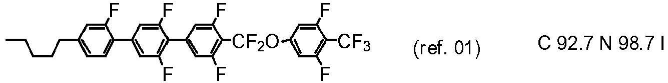

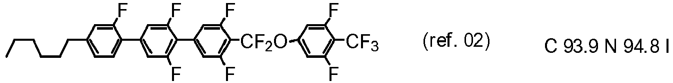

### (Compound of the invention)

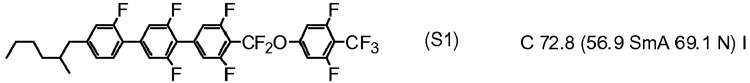

Patent Document 14 does not disclose any synthesis method of a liquid crystal compound having branched alkyl or branched alkenyl, or any specific examples of such compound, and describes nothing about the effect of lowering the melting point of the invention. It is noted that such effect is firstly discovered in the invention.

Moreover, Patent Documents 15 and 16 reported on branched alkyl and branched alkenyl.

### (Comparative Compound 2)

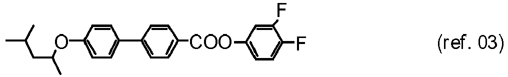

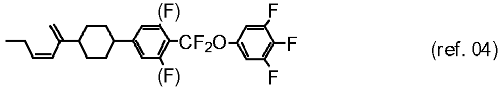

The three-ring compound (ref. 03) described in Patent Document 15 does not have a sufficiently large dielectric anisotropy because any of its left positioned rings is 1,4-phenylene. The three-ring compound (ref. 04) described in Patent Document 16 exhibits a large dielectric anisotropy, but does not have a sufficiently large optical anisotropy, because its most left positioned ring is 1,4-cyclohexylene. Moreover, a compound having an alkenyl group with two directly bonded double bonds, such as the compound (ref. 04), has low chemical stability. Particularly, the compound has a low stability to UV, and has a demerit of being remarkably degraded in the voltage holding ratio after UV irradiation.

Other compounds presenting analogous shortcomings to the comparative compounds discussed above are disclosed in Patent Documents 17 and 18.

### [Prior-Art Documents]

### [Patent Documents]

[Patent Document 1] Japanese Patent Publication No. 2003-327966
[Patent Document 2] International Publication No. 2005/90520
[Patent Document 3] Japanese Patent Publication No. 2005-336477
[Patent Document 4] Japanese Patent Publication No. 2006-89622
[Patent Document 5] Japanese Patent Publication No. 2006-299084
[Patent Document 6] Japanese Patent Publication No. 2006-506477
[Patent Document 7] Japanese Patent Publication No. 2006-506515
[Patent Document 8] International Publication No. 2006/063662
[Patent Document 9] Japanese Patent Publication No. 2006-225655
[Patent Document 10] Japanese Patent Publication No. 2005-157109
[Patent Document 11] International Publication No. 2005/80529
[Patent Document 12] Japanese Patent Publication No. 2006-127707
[Patent Document 13] International Publication No. 1998/023561
[Patent Document 14] International Publication No. 2010/058681
[Patent Document 15] Japanese Patent Publication No. 2007-291046
[Patent Document 16] DE 10251016
[Patent Document 17] International Publication No. 1996/37451
[Patent Document 18] International Publication No. 2005/080529

### [Non-Patent Documents]

[Non-patent Document 1] Nature Materials, 1, 64, (2002)
[Non-patent Document 2] Adv. Mater., 17, 96, (2005)
[Non-patent Document 3] Journal of the SID, 14, 551, (2006)

### SUMMARY OF THE INVENTION

### [Problems to Be Solved by the Invention]

The first object of the invention is to provide a liquid crystal compound that is stable to heat, light and so on, and has a large optical anisotropy, a large dielectric anisotropy and a low melting point. The second object is to provide a liquid crystal medium that is stable to heat, light and so on, has a broad temperature range of a liquid crystal phase, a large optical anisotropy and a large dielectric anisotropy, and exhibits an optically isotropic liquid crystal phase. The third object is to provide a variety of optical elements containing the liquid crystal medium, which can be used in a broad temperature range and has a short response time, a high contrast and a low driving voltage.

### [Means for Solving the Problems]

The invention provides a liquid crystal compound, a liquid crystal medium (a liquid crystal composition or a polymer/liquid crystal composite), and an optical element containing the liquid crystal medium as claimed.
[1] A compound as claimed in any one of claims 1 to 4.
[2] A liquid crystal composition, as specified in claim 5.
[3] The liquid crystal composition as specified in claim 5, further comprising at least one compound selected from the group consisting of compounds represented by formulae (2), (3) and (4), 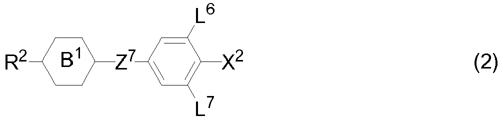 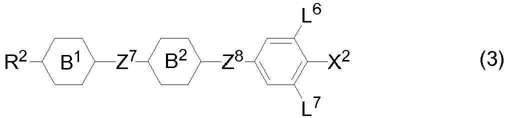 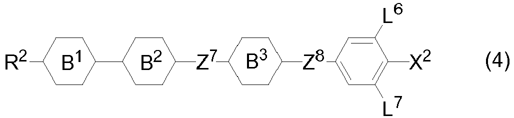 wherein R² is straight alkyl of C₁₋₁₀ or straight alkenyl of C₂₋₁₀, and in the alkyl and the alkenyl, arbitrary hydrogen is optionally replaced by fluorine and arbitrary -CH₂-optionally replaced by -O-; X² is fluorine, chlorine, -OCF₃, -OCHF₂, -CF₃, -CHF₂, -CH₂F, -OCF₂CHF₂ or -OCF₂CHFCF₃; ring B¹, ring B² and ring B³ are independently 1,4-cyclohexylene, 1,3-dioxane-2,5-diyl, pyrimidine-2,5-diyl, tetrahydropyran-2,5-diyl, 1,4-phenylene, naphthalene-2,6-diyl, 1,4-phenylene in which arbitrary hydrogen is replaced by fluorine, or naphthalene-2,6-diyl in which arbitrary hydrogen is replaced by fluorine or chlorine; Z⁷ and Z⁸ are independently -(CH₂)₂-, -(CH₂)₄-, -COO-, -CF₂O-, -OCF₂-, -CH=CH-, -C≡C-, -CH₂O- or a single bond; and L⁶ and L⁷ are independently hydrogen or fluorine.
[4] The liquid crystal composition as specified in claims 5 or 6, further comprising at least one compound selected from the group consisting of compounds represented by formula (5), or at least one compound selected from the group consisting of compounds represented by formula (6), 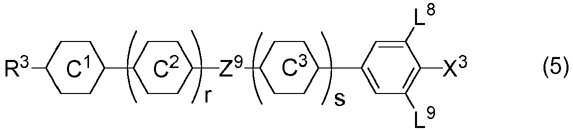 wherein R³ is straight alkyl of C₁₋₁₀ or straight alkenyl of C₂₋₁₀, and in the alkyl and the alkenyl, arbitrary hydrogen is optionally replaced by fluorine and arbitrary -CH₂- is optionally replaced by -O-; X³ is -C≡N or -C≡C-C≡N; ring C¹, ring C² and ring C³ are independently 1,4-cyclohexylene, 1,4-phenylene, 1,4-phenylene in which arbitrary hydrogen is replaced by fluorine, naphthalene-2,6-diyl, naphthalene-2,6-diyl in which arbitrary hydrogen is replaced by fluorine or chlorine, 1,3-dioxane-2,5-diyl, tetrahydropyran-2,5- diyl or pyrimidine-2,5-diyl; Z⁹ is -(CH₂)₂-, -COO-, -CF₂O-, -OCF₂-, -C≡C-, -CH₂O- or a dingle bond; L⁸ and L⁹ are independently hydrogen or fluorine; and r is 1 or 2, s is 0 or 1, and r+s is 0, 1 or 2, 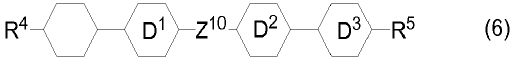 wherein R⁴ and R⁵ are independently straight alkyl of C₁₋₁₀ or straight alkenyl of C₂₋₁₀, and in the alkyl and the alkenyl, arbitrary hydrogen is optionally replaced by fluorine and arbitrary -CH₂- is optionally replaced by -O-; ring D¹, ring D² and ring D³ are independently 1,4-cyclohexylene, pyrimidine-2,5-diyl, 1,4-phenylene, 2-fluoro-1,4-phenylene, 3-fluoro-1,4-phenylene or 2,5-difluoro-1,4-phenylene; and Z¹⁰ is -C≡C-, -COO-, -(CH₂)₂-, -CH=CH- or a single bond.
[5] The liquid crystal composition of claim 5, further comprising at least one compound selected from the group consisting of compounds represented by formula (5) of claim 7.and at least one compound selected from the group consisting of compounds represented by formula (6) of claim 7.
[6] The liquid crystal composition of any one of claims 5 to 8, further comprising at least one compound selected from the group consisting of compounds represented by formulae (7), (8), (9) and (10), at least one compound selected from the group consisting of compounds represented by formula (11), or at least one compound selected from the group consisting of compounds represented by formulae (7), (8), (9) and (10) and at least one compound selected from the group consisting of compounds represented by formula (11), 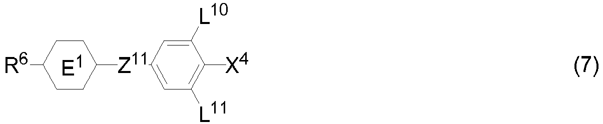 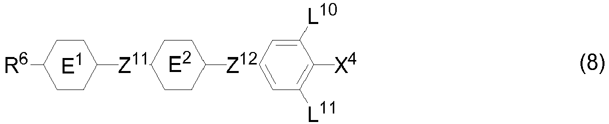 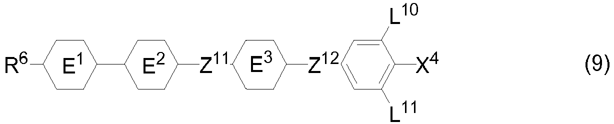 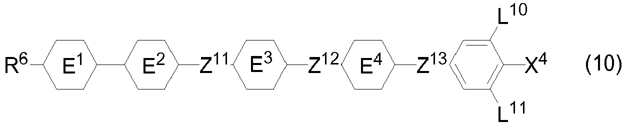 wherein R⁶ is straight alkyl of C₁₋₁₀, straight alkenyl of C₂₋₁₀ or straight alkynyl of C₂₋₁₀, and in the alkyl, the alkenyl and the alkynyl, arbitrary hydrogen is optionally replaced by fluorine and arbitrary -CH₂- is optionally replaced by -O-; X⁴ is fluorine, chlorine, -SF₅, -OCF₃, -OCHF₂, -CF₃, -CHF₂, -CH₂F, -OCF₂CHF₂ or -OCF₂CHFCF₃; ring E¹, ring E², ring E³ and ring E⁴ are independently 1,4-cyclohexylene, 1,3-dioxane-2,5-diyl, pyrimidine-2,5-diyl, tetrahydropyran-2,5-diyl, 1,4-phenylene, naphthalene-2,6-diyl, 1,4-phenylene in which arbitrary hydrogen is replaced by fluorine or chlorine, or naphthalene-2,6-diyl in which arbitrary hydrogen is replaced by fluorine or chlorine; Z¹¹, Z¹² and Z¹³ are independently -(CH₂)₂-, -(CH₂)₄-, -COO-, -CF₂O-, -OCF₂-, -CH=CH-, -C≡C-, -CH₂O- or a single bond; L¹⁰ and L¹¹ are independently hydrogen or fluorine; 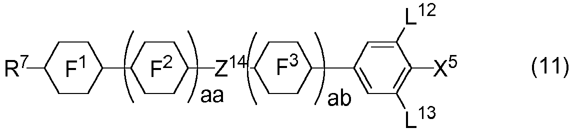 wherein R⁷ is straight alkyl of C₁₋₁₀, straight alkenyl of C₂₋₁₀ or straight alkynyl of C₂₋₁₀, and in the alkyl, the alkenyl and the alkynyl, arbitrary hydrogen is optionally replaced by fluorine and arbitrary -CH₂- is optionally replaced by -O-; X⁵ is -C≡N, -N=C=S or -C≡C-C≡N; ring F¹, ring F² and ring F³ are independently 1,4-cyclohexylene, 1,4-phenylene, 1,4-phenylene in which arbitrary hydrogen is replaced by fluorine or chlorine, naphthalene-2,6-diyl, naphthalene-2,6-diyl in which arbitrary hydrogen is replaced by fluorine or chlorine, 1,3-dioxane-2,5-diyl, tetrahydropyran-2,5-diyl or pyrimidine-2,5- diyl; Z¹⁴ is -(CH₂)₂-, -COO-, -CF₂O-, -OCF₂-, -C≡C-, -CH₂O- or a single bond; L¹² and L¹³ are independently hydrogen or fluorine; and aa is 0, 1 or 2, ab is 0 or 1, and aa+ab is 0, 1 or 2.
[7] The liquid crystal composition of claims 5 or 9, which exhibits a chiral nematic phase at any temperature in the range of 70°C to -20°C and has a helical pitch of 700 nm or less at a temperature in at least a part of the range of 70°C to -20°C.
[8] The liquid crystal composition of any one of claims 5 to 10, wherein the chiral dopant is at least one compound selected from the group consisting of compounds represented by formulae (K1)-(K5), 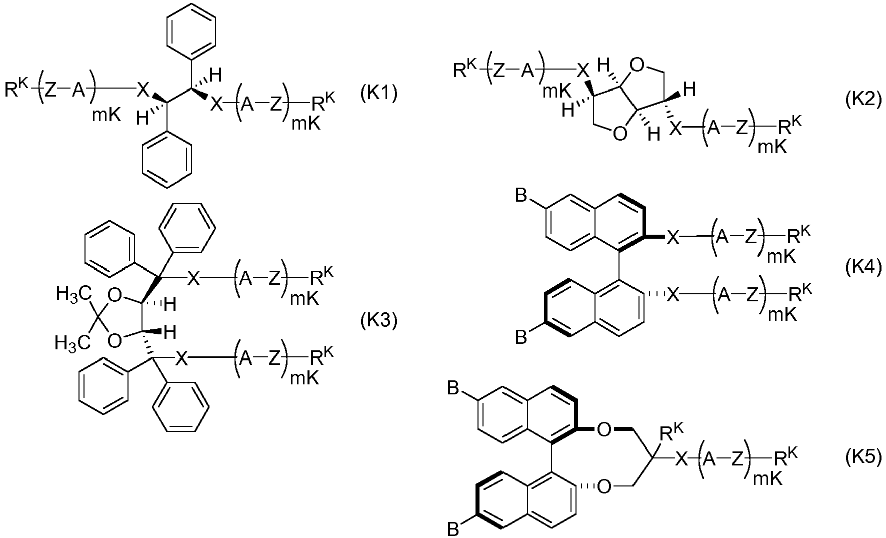
   wherein in formulae (K1) to (K5), each R^{K} is independently hydrogen, halogen, -C≡N, -N=C=O, -N-C=S, or C₁₋₂₀ alkyl in which arbitrary -CH₂- is optionally replaced by -O-, -S-, -COO- or -OCO-, arbitrary -CH₂-CH₂- is optionally replaced by -CH=CH-, -CF=CF- or -C≡C-, and arbitrary hydrogen is optionally replaced by halogen; each A is independently an aromatic or non-aromatic, three- to eight-membered ring, or a fused ring of 9 or more carbons, and in these rings arbitrary hydrogen is optionally replaced by halogen, C₁₋₃ alkyl or C₁₋₃ haloalkyl, -CH₂- is optionally replaced by -O-, -S- or -NH-, and -CH= is optionally replaced by -N=; each B is independently hydrogen, halogen, C₁₋₃ alkyl, C₁₋₃ haloalkyl, an aromatic or non-aromatic, three- to eight-membered ring, or a fused ring of 9 or more carbons, and in these rings arbitrary hydrogen is optionally replaced by halogen, C₁₋₃ alkyl or C₁₋₃ haloalkyl, -CH₂- is optionally replaced by -O-, -S- or -NH-, and -CH= is optionally replaced by -N=; each Z is independently a single bond, or C₁₋₈ alkylene in which arbitrary -CH₂- is optionally replaced by -O-, -S-, -COO-, -OCO-, -CSO-, -OCS-, -N=N-, -CH=N- or -N=CH-, arbitrary -CH₂-CH₂- is optionally replaced by -CH=CH-, -CF=CF- or -C≡C-, and arbitrary hydrogen is optionally replaced by halogen;
   X is a single bond, -COO-, -OCO-, -CH₂O-, -OCH₂-, -CF₂O-, -OCF₂- or -CH₂CH₂-; and mK is an integer of 1-4.
[9] A mixture, comprising the liquid crystal composition of any one of claims 5 to 11, and a polymerizable monomer.
[10] A polymer/liquid crystal composite material, as specified in claim 13 or 14.
[11] An optical element, as claimed in claim 15.

In the invention, "liquid crystal compound" means a compound with a mesogen but is not limited to a compound with a liquid crystal phase. "Liquid crystal medium" is a generic term of a liquid crystal composition and a polymer/liquid crystal composite. Moreover, "optical element" refers to various elements utilizing electrooptical effects to achieve light modulation or optical switching, etc., for example, display elements (LCD elements), light modulation elements used in optical communication systems, optical signal processing or various sensor systems. For light modulation utilizing a change in the refractive index of an optically isotropic liquid crystal medium caused by a voltage application, the Kerr effect is known. The Kerr effect is that the electric birefringence Δ*n*(*E*) is proportional to the square of the electric field *E,* i.e., Δ*n*(*E*) = *K·λ·E*² (*K*= Kerr constant, *λ*= wavelength) for a material exhibiting the Kerr effect. Herein, the "electric birefringence" is the optical anisotropy induced by application of an electric field to the isotropic medium.

The terms in the specification are defined below. "Liquid crystal compound" is a generic term of compounds having a liquid crystal phase, such as nematic phase or smectic phase etc., and compounds having no liquid crystal phase but being useful as components of liquid crystal compositions. "Chiral dopant" is an optically active compound, which is added to give a desired twisted molecular arrangement to the liquid crystal composition. "LCD element" is the generic term of LCD panels and LCD modules. "Liquid crystal compound", "liquid crystal composition" and "LCD element" are sometimes simply called "compound", "composition" and "element", respectively. Further, e.g., the upper-limit temperature of a liquid crystal phase is the phase transition temperature from the liquid crystal phase to the isotropic phase, and often simply called "clearing point" or "upper-limit temperature". The lower-limit temperature of a liquid crystal phase is often simply called "lower-limit temperature". A compound represented by formula (1) is often just called a compound (1). This rule also applies to a compound represented by formula (2), etc. In formulae (1)-(11), the symbols B, D and E, etc. surrounded by hexagons respectively correspond to ring B, ring D and ring E, etc. A compound content expressed by a percentage is a weight percentage (wt%) relative to the total weight of the composition. Numerous identical symbols, such as the rings A¹, Y¹ or B, etc., are included in the same or different formulae, but the groups represented by the same symbol can be identical or different from each other.

"Arbitrary" denotes not only arbitrary position but also arbitrary number, except for the case where the number is zero. The expression "arbitrary A may be replaced by B, C or D" not only means arbitrary A may be replaced by B, arbitrary A may be replaced by C or arbitrary A may be replaced by D, but also means that a plurality of A's may be replaced by at least two of B, C and D. For example, the scope of "alkyl in which arbitrary -CH₂- is optionally replaced by -O- and arbitrary -CH₂-CH₂- optionally replaced by -CH=CH-" includes alkyl, alkenyl, alkoxy, alkoxyalkyl, alkenyloxy and alkenyloxyalkyl, etc. Further, in the invention, two contiguous -CH₂- being replaced by -O- to form -O-O- is not preferable, so is the terminal -CH₂- of alkyl being replaced by -O-. The invention will be further described below. The terminal groups, rings and linking groups, etc. of the compound of formula (1) will also be illustrated by way of preferred examples.

### [Effects of the Invention]

The liquid crystal compound of the invention is stable to heat, light and so on, and has a large optical anisotropy, a large dielectric anisotropy and a low melting point. Because the liquid crystal compound has a low melting point, it is feasible that a liquid crystal composition has a high content thereof. The liquid crystal composition is stable to heat, light and so on, exhibits a high upper-limit temperature and a low lower-limit temperature of an optically isotropic liquid crystal phase, and has a low driving voltage in an element driven in an optically isotropic liquid crystal phase. The polymer//liquid crystal composite material has an optically isotropic liquid crystal phase, exhibits a high upper-limit temperature and a low lower-limit temperature of the optically isotropic liquid crystal phase, and has a low driving voltage in an element driven in an optically isotropic liquid crystal phase.

The optical element of the invention driven in an optically isotropic liquid crystal phase has a broad temperature range for use, a short response time, a high contrast and a low driving voltage.

### BRIEF DESCRIPTION OF THE DRAWINGS

Fig. 1 shows a comb-like electrode substrate used in an embodiment.
Fig. 2 shows an optical system used in an embodiment.

### DESCRIPTION OF THE EMBODIMENTS

### 1-1. Compound (1)

The liquid crystal compound of the invention is a compound represented by the above formula (1). The liquid crystal composition having an optically isotropic liquid crystal phase of the invention contains, as a component A, the compound represented by the above formula (1). The first aspect of the liquid crystal composition of the invention relates to a composition of the component A only, or a composition containing the component A and other components not specifically mentioned in this specification. The compound of formula (1) is described first. 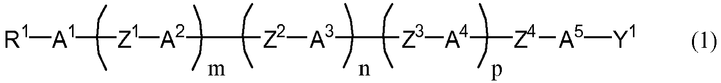 In formula (1), R¹ is branched C₄₋₂₀ alkyl or alkenyl branching at a carbon of 2-position thereof. Specific examples of the branched alkyl are expressed by the following formulae (CHN1-1), (CHN1-3), and (CHN1-6) to (CHN1-7), wherein R^{1a} is C₁₋₁₀ alkyl in which arbitrary -CH₂- is optionally replaced by -O- and arbitrary -CH₂-CH₂- is optionally replaced by -CH=CH-; R^{1b} is hydrogen, or C₁₋₁₀ alkyl in which arbitrary -CH₂-is optionally replaced by -O- and arbitrary -CH₂-CH₂- optionally replaced by -CH=CH-. The formulae (CHN1-2), (CHN1-4), and (CHN1-5), (CHN1-8) and (CHN1-9) are not claimed in the present application.

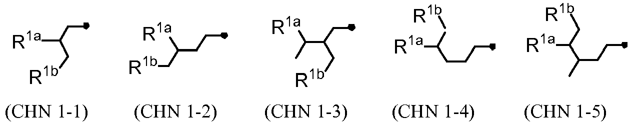

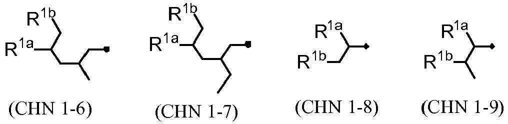

The characteristics of a compound with branched alkyl depend on the branching position. As compared to the straight-chain compounds, a compound having an alkyl group branching at 2-, 3-, 4- or 1-position, such as the groups (CHN 1-1) to (CHN 1-9), has a quite low melting point.

Specific examples of branched alkenyl are expressed by the following formulae (CHN 2-1) to (CHN 2-32), wherein R^{1a} and R^{1b} represent the same structures as above. The formulae (CHN2-7) to (CHN2-12), (CHN2-15) to (CHN2-21), (CHN2-23) to (CHN2-26), (CHN2-28) and (CHN2-29) are not claimed in the present application.

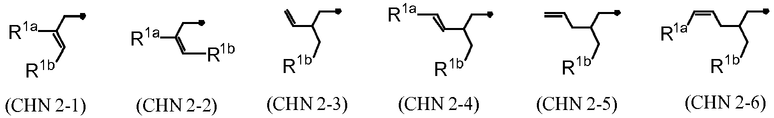

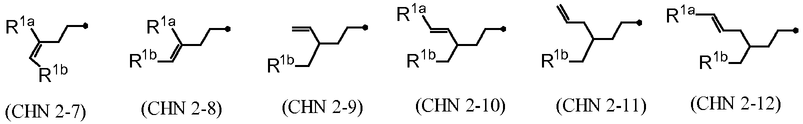

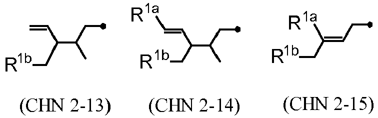

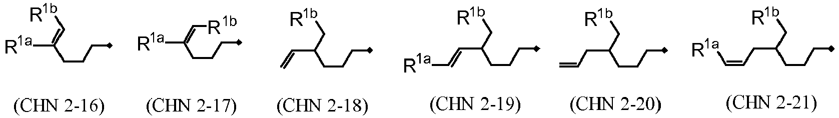

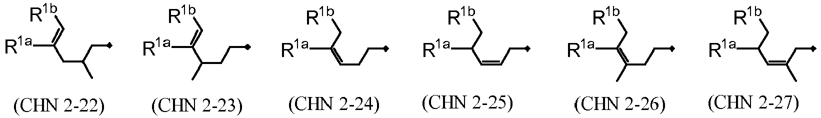

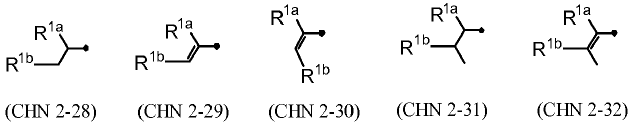

The characteristics of a compound having branched alkenyl depend on the branching position. As compared to the straight-chain compounds, a compound having an alkenyl group branching at 2-, 3-, 4- or 1-position, such as the groups (CHN 2-1) to (CHN 2-32), has a quite low melting point.

The preferred stereo configuration of -CH=CH- in an alkenyl depends on the position of the double bond. For example, a *trans*-configuration is generally preferred for an alkenyl having a double bond at an odd position, such as formula (CHN 2-1), and a *cis*-configuration is generally preferred for alkenyl having a double bond at an even position, such as (CHN 2-5). An alkenyl compound having a preferred stereo configuration has a broad temperature range of a liquid crystal phase. This is detailed in Mol. Cryst. Liq. Cryst., 1985, 131, 109 and Mol. Cryst. Liq. Cryst., 1985, 131, 327. Moreover, it is desired that the position of the double bond does not make the double bond conjugate with another double bond or a ring such as 1,4-phenylene.
Examples unclaimed in the present application of branched alkoxy and branched alkenyloxy are expressed by the following formulae (CHN 3-1) to (CHN 3-15), wherein R^{1a} and R^{1b} represent the same structures as above.

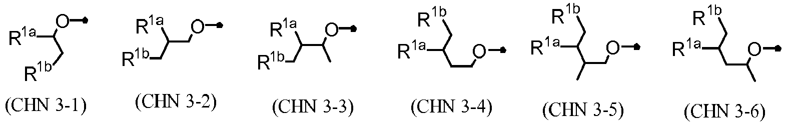

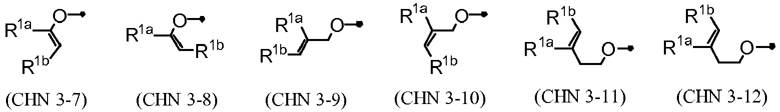

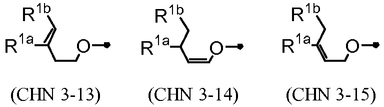

The characteristics of a compound having branched alkoxy or branched alkenyloxy depend on the branching position. As compared to straight-chain compounds, a compound having an alkoxy or alkenyloxy group branching at 2-, 3- or 4-position, such as the groups (CHN 3-1) to (CHN 3-15), have a quite low melting point. Moreover, it is desired that the position of the double bond in the branched alkenyloxy does not make the double bond conjugate with another double bond or a ring such as 1,4-phenylene.

Preferred examples of R^{1a} and R^{1b} are hydrogen, -CH₃, -C₂H₅, -C₃H₇, -C₄H₉, -C₅H₁₁, -C₆H₁₃, -C₇H₁₅, -C₈H₁₇, -C₉H₁₉ and -C₁₀H₂₁. It is more preferred that arbitrary one of R^{1a} and R^{1b} is -CH₃, -C₂H₅ or -C₃H₇.

Examples unclaimed in the present application of the group derived from R^{1a} and R^{1b} by replacing arbitrary -CH₂- by -O- or replacing arbitrary -CH₂-CH₂- by -CH=CH-are CH₃(CH₂)₂O-, CH₃-O-(CH₂)₂-, CH₃-O-CH₂-O-, CH₂=CH-(CH₂)₃-, CH₃-CH=CH-(CH₂)₂- and CH₃-CH=CH-CH₂O-, etc.

Moreover, examples unclaimed in the present application of the group derived from R^{1a} by replacing arbitrary -CH₂- by -O- or replacing arbitrary -CH₂-CH₂- by -C≡C- or -CH=CH- and replacing arbitrary hydrogen by halogen are CF₂=CH-, CH₂F(CH₂)₂O- and CH₂FCH₂C≡C-.

In formula (1), the rings A¹, A², A³, A⁴ and A⁵ are independently 1,4-phenylene, 1,3-dioxane-2,5-diyl, tetrahydropyran-2,5-diyl, tetrahydropyran-3,6-diyl, pyrimidine-2,5-diyl, pyridine-2,5-diyl or naphthalene-2,6-diyl, and in these rings arbitrary hydrogen is optionally replaced by fluorine or chlorine.

Preferred examples of the rings A¹, A², A³, A⁴ and A⁵ are expressed by formulae (RG-1) to (RG-16).

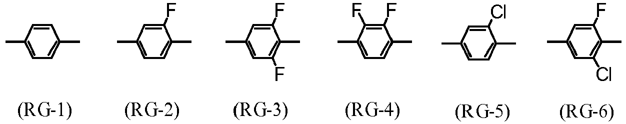

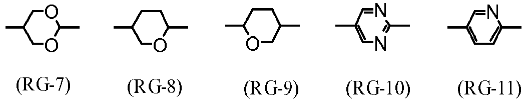

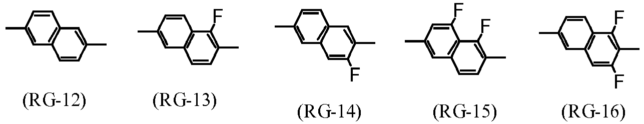

More preferred examples of the rings A¹, A², A³, A⁴ and A⁵ are the structures expressed by formulae (RG-1) to (RG-3), (RG-5) to (RG-7) and (RG-12) to (RG-16). Particularly, a compound having the group (RG-2) or (RG-3) as 1,4-phenylene in which one or two hydrogens are replaced by fluorine has a large dielectric anisotropy. Moreover, a compound having two or more of the ring expressed by (RG-2) or (RG-3) particularly has a large dielectric anisotropy.

Moreover, a compound with the ring A¹ of formula (RG-2) or (RG-3) has a low melting point.

In formula (1), Z¹, Z², Z³ and Z⁴ are independently a single bond, or C₁₋₄ alkylene in which arbitrary -CH₂- is optionally replaced by -O-, -COO-, -OCO- or -CF₂O- and arbitrary -CH₂-CH₂- is optionally replaced by -CH=CH-, -CF=CF- or -C≡C-.

Preferred examples of Z¹, Z², Z³ and Z⁴ are a single bond, -CH₂-, -(CH₂)₂-, -COO-, -CF₂O- or -CH=CH-. It is more preferred that arbitrary one of Z¹, Z², Z³ and Z⁴ is -COO- or -CF₂O-.

In formula (1), Y¹ is fluorine, chlorine, -SF₅, -C≡N, -N=C=S, or C₁₋₃ alkyl in which arbitrary hydrogen is replaced by fluorine or chlorine, and in the alkyl arbitrary -CH₂- is optionally replaced by -O- and arbitrary -CH₂-CH₂- is optionally replaced by -CH=CH- or -C≡C-.

Specific examples of the alkyl in which arbitrary hydrogen is replaced by fluorine or chlorine are -CHF₂, -CF₃, -CF₂CH₂F, -CF₂CHF₂, -CH₂CF₃, -CF₂CF₃, -(CH₂)₃-F, -(CF₂)₃-F, -CF₂CHFCF₃ and -CHFCF₂CF₃.

Specific examples of the alkoxy in which arbitrary hydrogen is replaced by fluorine or chlorine are -OCHF₂, -OCF₃, -OCF₂CH₂F, -OCF₂CHF₂, -OCH₂CF₃, -O-(CF₂)₃-F, -OCF₂CHFCF₃ and -OCHFCF₂CF₃.

Specific examples of the alkenyl in which arbitrary hydrogen is replaced by fluorine or chlorine are -CH=CF₂, -CF=CHF, -CH=CHCH₂F, -CH=CHCF₃, -(CH₂)₂-CH=CF₂, -CH₂CH=CHCF₃ and -CH=CHCF₂CF₃.

Preferred specific examples of Y¹ are fluorine, chlorine, -C≡N, -CF₃, -CHF₂, -OCF₃ and -OCHF₂. More preferred examples of Y¹ are fluorine, chlorine, -C≡N, -CF₃ and -OCF₃. When Y¹ is fluorine or chlorine, the melting point is low, and the compatibility with other liquid crystal compounds is particularly good. When Y¹ is -C≡N, -CF₃, -CHF₂, -OCF₃ or -OCHF₂, the dielectric anisotropy is particularly large.

In formula (1), m is 1, n is 1, and p is 0 or 1.

Among the structures of formula (1), the preferred structures are expressed by formulae (1-1) and (1-2). 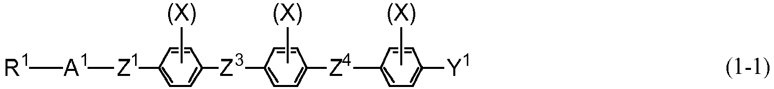 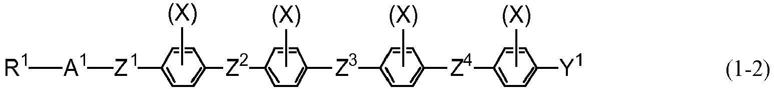 In formulae (1-1) to (1-2), R¹ is C₄₋₂₀ alkyl or alkenyl branching at a carbon of 2-position thereof; the ring A¹ is 1,4-phenylene, 1,3-dioxane-2,5-diyl, tetrahydropyran-2,5-diyl, tetrahydro- pyran-3,6-diyl, pyrimidine-2,5-diyl or pyridine-2,5-diyl, and in these rings arbitrary hydrogen is optionally replaced by fluorine; Z¹, Z², Z³ and Z⁴ are independently a single bond, -CH₂CH₂-, -COO- or -CF₂O-, with a proviso that arbitrary one of Z¹, Z², Z³ and Z⁴ is -COO- or -CF₂O-; Y¹ is fluorine, chlorine, -C≡N, or C₁₋₃ alkyl in which arbitrary hydrogen is replaced by fluorine, and in the alkyl arbitrary -CH₂- is optionally replaced by -O- and arbitrary -CH₂-CH₂- is optionally replaced by -CH=CH-; X is fluorine or chlorine, wherein the symbol of 1,4-phenylene and (X) connected by a straight line, 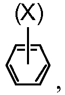 represents 1,4-phenylene in which one or two hydrogens are optionally replaced by X.

Among the compounds, the particularly preferred ones have R¹ being a structure expressed by arbitrary one of formulae (CHN1-1), (CHN1-3), and (CHN1-6) to (CHN1-7), Z¹, Z², Z³ and Z⁴ being independently a single bond, -(CH₂)₂-, -COO- or -CF₂O- with a proviso that at least one of Z¹, Z², Z³ and Z⁴ is -COO- or -CF₂O-, X being fluorine or chlorine, and Y¹ being fluorine, chlorine, -C≡N, -CF₃ or -OCF₃.

Among the compounds of formulae (1-1) to (1-2), the more preferred ones are the compounds of the following formulae (1-1-1), (1-1-2), (1-2-1), (1-2-2), (1-2-9) and (1-2-10), wherein those of formulae (1-1-1) to (1-1-2) and (1-2-1) to (1-2-2) are even more preferred. 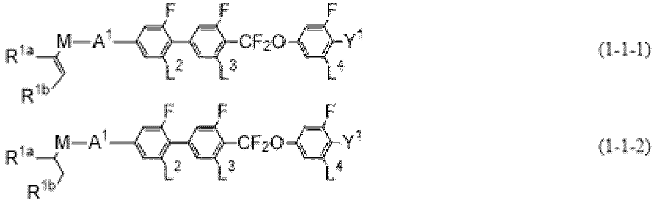 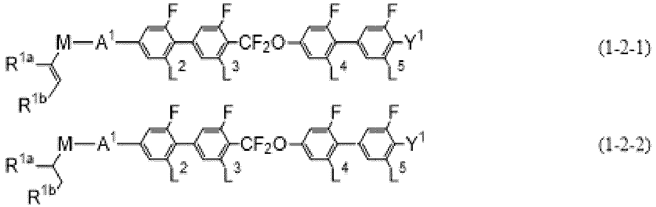 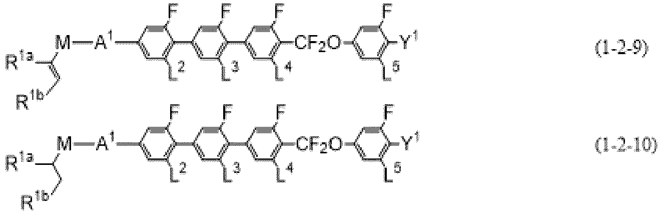 In these formulae, R^{1a} is C₁₋₁₀ alkyl or C₂₋₁₀ alkenyl; R^{1b} is hydrogen or C₁₋₁₀ alkyl; M is -CH₂-; A¹ is 1,4-phenylene, 1,3-dioxane-2,5-diyl, tetrahydropyran- 2,5-diyl, tetrahydropyran-3,6-diyl, pyrimidine-2,5-diyl or pyridine-2,5-diyl, and in these rings arbitrary hydrogen is optionally replaced by fluorine; L², L³, L⁴ and L⁵ are independently hydrogen, fluorine or chlorine; Y¹ is fluorine, chlorine, -C≡N, C₁₋₃ alkyl in which arbitrary hydrogen is replaced by fluorine, alkenyl in which arbitrary hydrogen is replaced by fluorine, or alkoxy in which arbitrary hydrogen is replaced by fluorine.

In formulae (1-1-1), (1-1-2), (1-2-1), (1-2-2), (1-2-9) and (1-2-10), L², L³, L⁴ and L⁵ are independently hydrogen, fluorine or chlorine. When at least one of L², L³, L⁴ and L⁵ is chlorine or fluorine, the compound has a large dielectric anisotropy, a low melting point, and a good compatibility with other liquid crystal compounds. It is preferred that arbitrary one of them is hydrogen or fluorine.

### 1-2. Properties of Compound (1)

The compound (1) used in this invention is further detailed below. A compound (1) is a liquid crystal compound having branched alkyl or branched alkenyl. This compound has very stable physical and chemical properties under the conditions where the element is usually used, and has a good compatibility with other liquid crystal compounds. The compound is difficult to exhibit a smectic phase. A composition containing such a compound is stable under the conditions where the element is usually used. Therefore, the composition has a broader temperature range of optically isotropic liquid crystal phase, and thus can be used in a display element in a broad temperature range. Moreover, because the compound has large dielectric anisotropy and optical anisotropy, it is useful as a component for lowering the driving voltage of a composition driven in an optically isotropic liquid crystal phase. Moreover, when the composition prepared from the compound (1) and the chiral dopant exhibits a blue phase, a uniform blue phase without co-existence of the N* phase and the isotropic phase is easily formed. That is, the compound (1) is a compound easily exhibiting a uniform blue phase.

For the compound (1), by suitably selecting the combination of m, n and p, the left terminal group R¹, the right terminal group Y¹ or the linking groups Z¹ to Z⁴, the physical properties such as clearing point, optical anisotropy and dielectric anisotropy, etc., can be adjusted arbitrarily. The respective effects of the combination of m, n and p, the species of the left terminal group R¹, the species of the right terminal group Y¹, the species of the linking groups Z¹ to Z⁴, and the species of L² to L⁵ in 1,4-phenylene to the physical properties of the compound (1) are described below.

In general, a compound with m+n+p=3 has a higher clearing point and a large optical anisotropy. A compound with m+n+p=2 has a lower melting point and a good compatibility with other liquid crystal compounds. A not claimed compound with m+n+p=1 has a very low melting point.

A compound with R¹ being an optically active group is useful as a chiral dopant. A compound with R¹ not being an optically active group is useful as a component of a composition. When R¹ is alkenyl, the preferred stereo configuration depends on the position of the double bond. An alkenyl compound having a preferred stereo configuration has a broader temperature range of a liquid crystal phase.

When the linking group Z¹, Z², Z³ or Z⁴ is a single bond, -CH₂CH₂-, -CH=CH-, -CF₂O-, -OCF₂-, -CH₂O-, -OCH₂-, -CF=CF-, -(CH₂)₃-O-, -O-(CH₂)₃-, -(CH₂)₂-CF₂O-, -OCF₂-(CH₂)₂- or -(CH₂)₄-, the viscosity is low. When the linking group is a single bond, -(CH₂)₂-, -CF₂O-, -OCF₂- or-CH=CH-, the viscosity is even lower. When the linking group is -CH=CH-, the temperature range of the liquid crystal phase is broad, and the elastic constant ratio K₃₃/K₁₁ (K₃₃: bend elastic constant, K₁₁: splay elastic constant) is large. When the linking group is -C≡C-, the optical anisotropy is large. When the linking group is -COO- or -CF₂O-, the dielectric anisotropy is large. When Z¹, Z², Z³ or Z⁴ is a single bond, -(CH₂)₂-, -CH₂O-, -CF₂O-, -OCF₂- or -(CH₂)₄-, the compound is chemically stable and is not easily degraded.

When the right terminal group Y¹ is fluorine, chlorine, -C≡N, -SF₅, -CF₃, -OCF₃ or -CH=CH-CF₃, the dielectric anisotropy is large. When Y¹ is fluorine, -CF₃ or -OCF₃, the compound is chemically stable.

When L², L³, L⁴ or L⁵ is hydrogen or chlorine, the melting point is low. When L², L³, L⁴ or L⁵ is fluorine, the dielectric anisotropy is large. When at least two of L² to L⁵ are fluorine, the dielectric anisotropy is very large. Moreover, when at least one of L² to L⁵ is chlorine, the compatibility with other liquid crystal compounds is good.

As mentioned above, by properly selecting the species of the ring structures, the terminal groups and the linking groups, etc., a compound with target physical properties can be obtained.

### 1-3. Specific Examples of Compound (1)

Preferred examples of the compound (1) are the compounds of formulae (1-1) to (1-2). More preferred examples are the compounds of formulae (1-1-1), (1-1-2), (1-2-1), (1-2-2), (1-2-9) and (1-2-10), wherein those of formulae (1-1-1) to (1-1-2) and (1-2-1) to (1-2-2) are even more preferred.

The specific compounds can be exemplified by the compounds of the following formulae (1-1-1a) to (1-2-16j) of which (1-1-1g), (1-1-2g), (1-1-1h), (1-1-2h), (1-1-1i), (1-1-2i), (1-1-1j), (1-1-2j), (1-1-1k), (1-1-2k), (1-1-11), (1-1-2l), (1-2-1d), (1-2-2d), (1-2-1e), (1-2-2e), (1-2-1f), (1-2-2f), (1-2-1g), (1-2-2g), (1-2-1h), (1-2-2h), (1-2-1i), (1-2-2i), (1-2-9d), (1-2-10d), (1-2-9e), (1-2-10e), (1-2-9f), (1-2-10f), (1-2-9g), (1-2-10g), (1-2-9h), (1-2-10h), (1-2-9i), (1-2-10i), (1-2-9j) and (1-2-10j) are within the scope of the claims, wherein R^{1a} or R^{1b} is hydrogen or C₁₋₁₀ alkyl, L¹, L², L³ or L⁴ is hydrogen, fluorine or chlorine, and Y¹ is fluorine, chlorine, -SF₅, -C≡N, -N=C=S, -CF₃, -CF₂H, -OCF₃ or -OCF₂H.

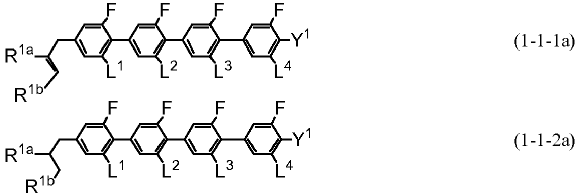

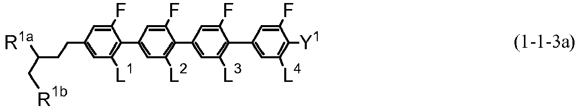

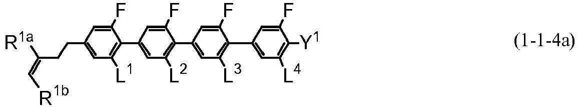

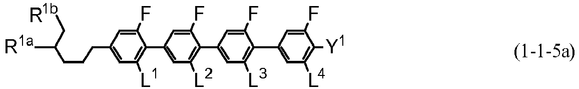

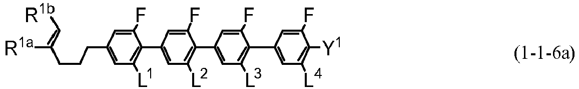

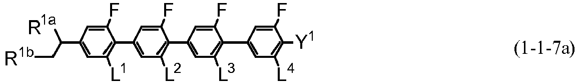

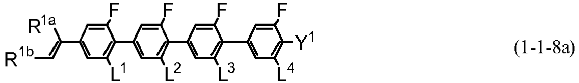

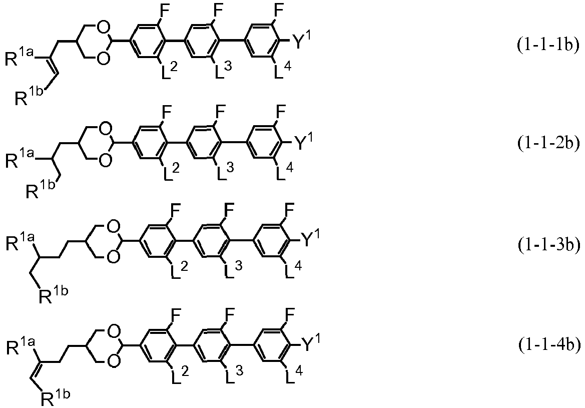

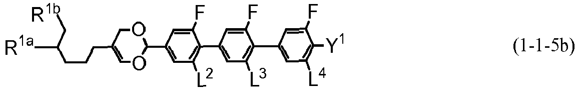

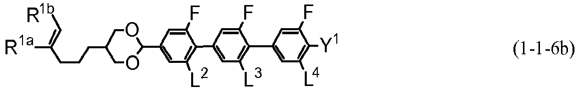

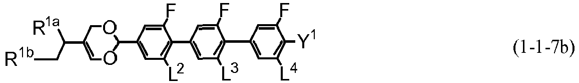

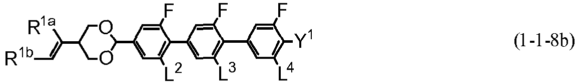

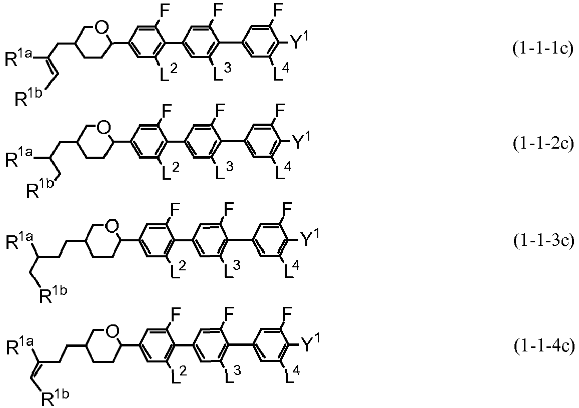

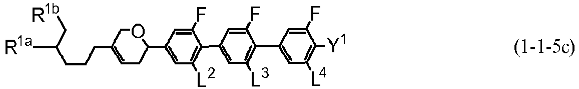

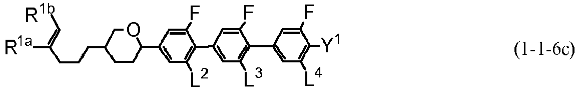

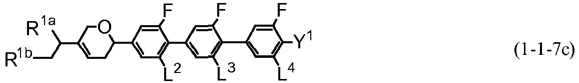

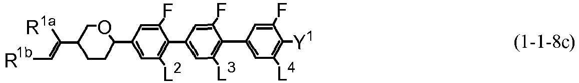

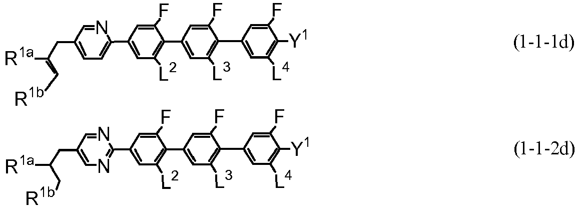

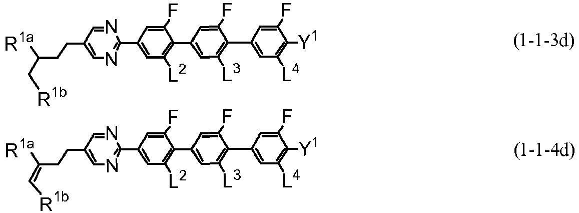

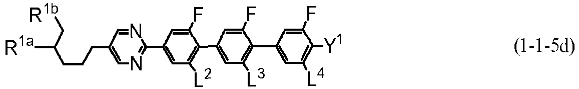

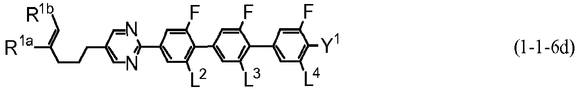

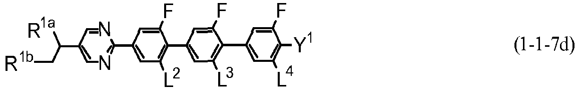

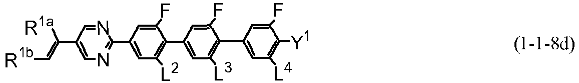

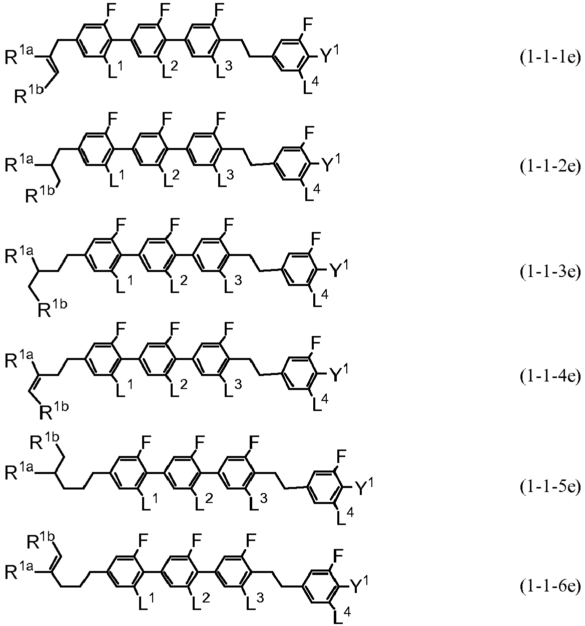

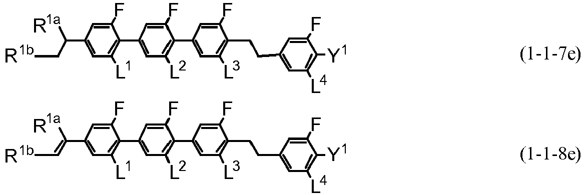

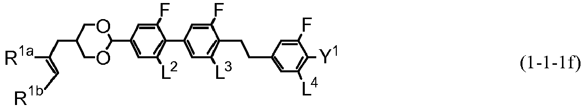

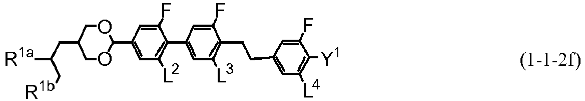

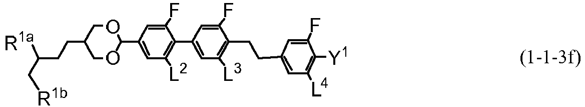

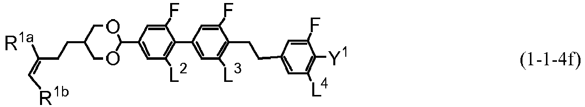

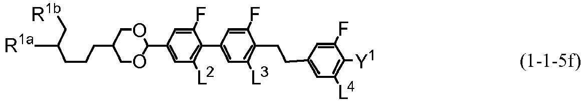

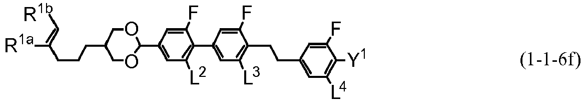

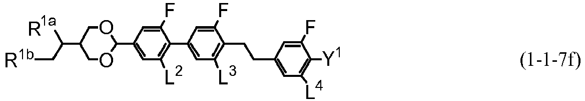

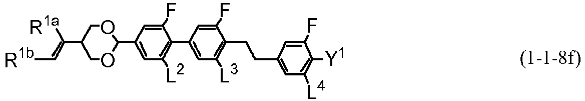

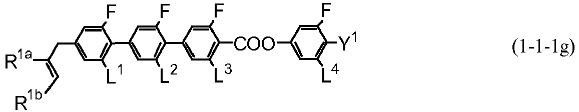

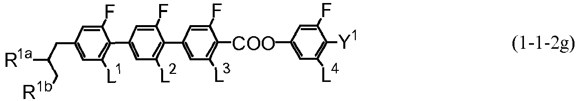

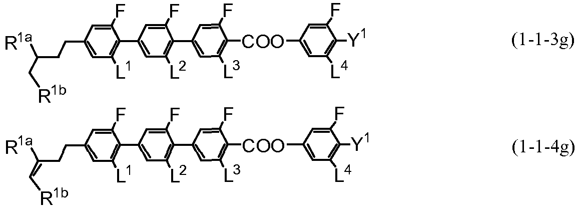

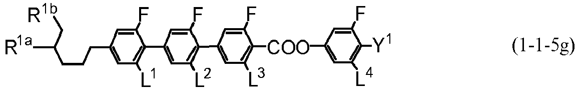

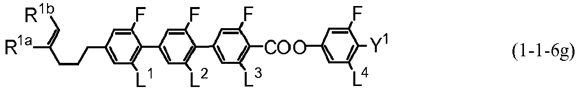

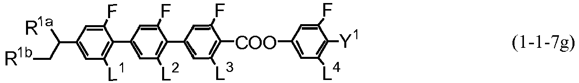

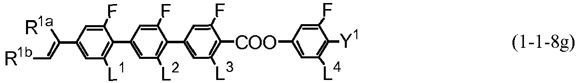

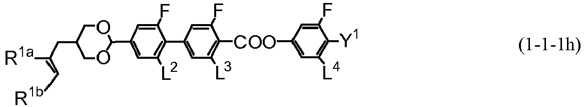

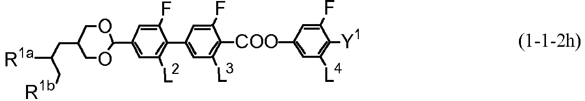

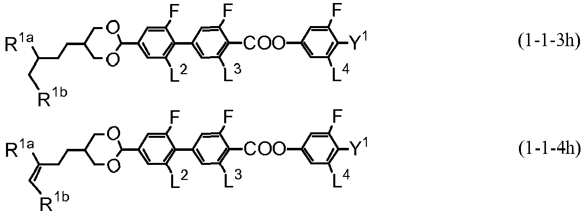

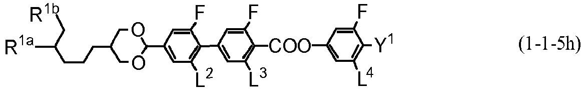

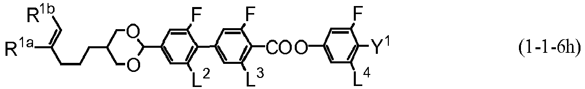

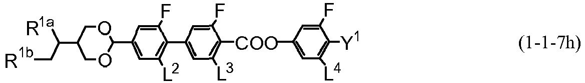

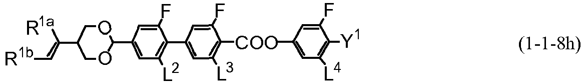

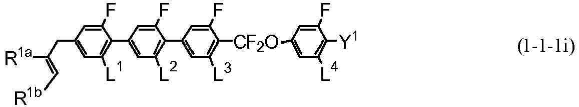

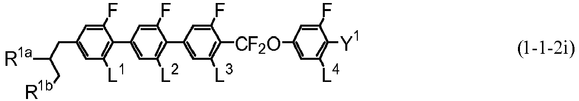

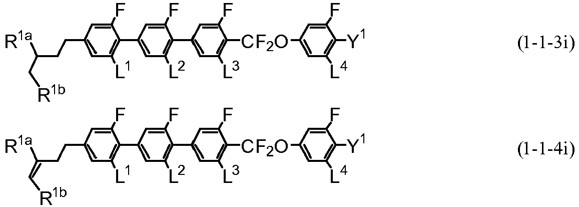

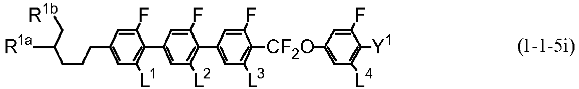

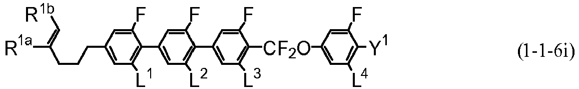

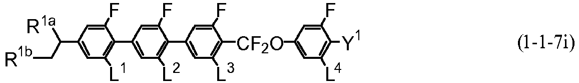

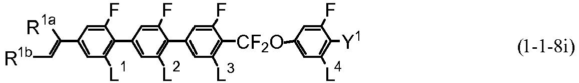

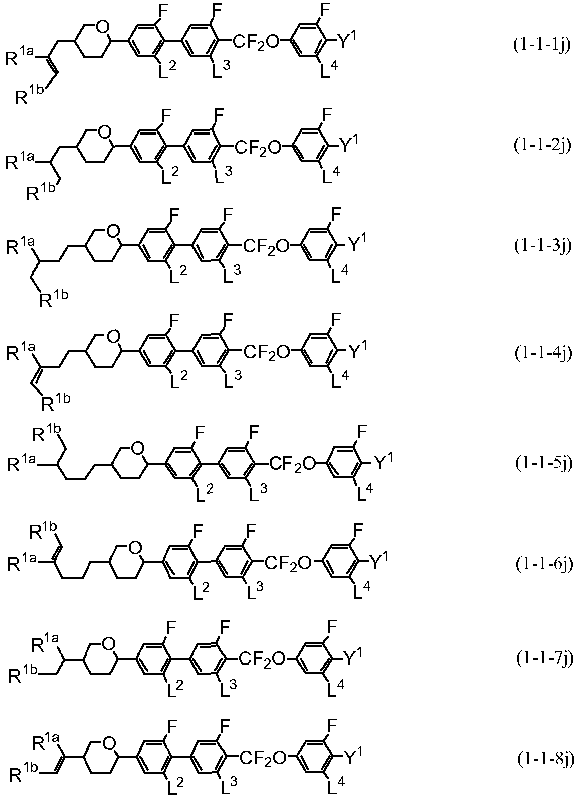

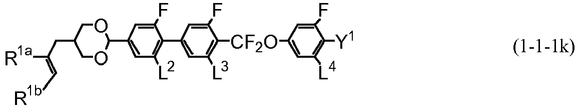

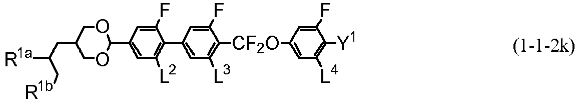

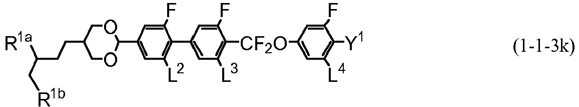

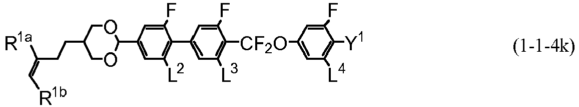

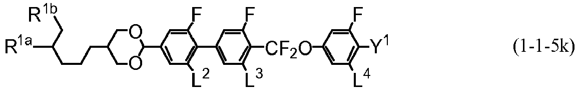

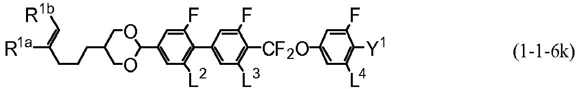

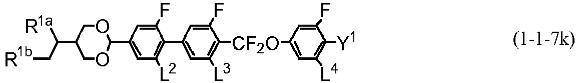

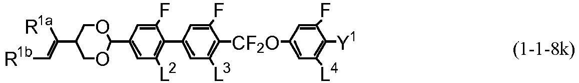

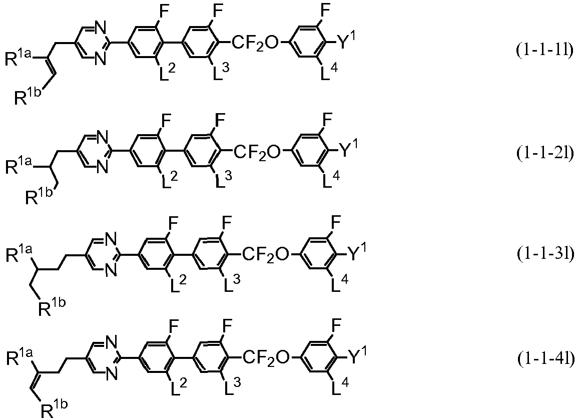

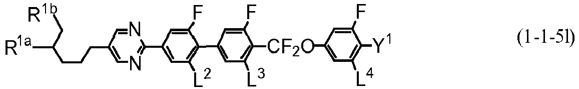

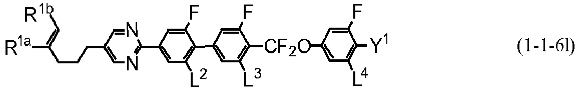

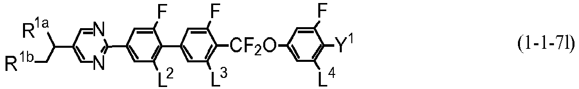

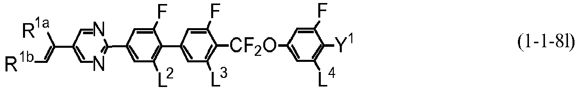

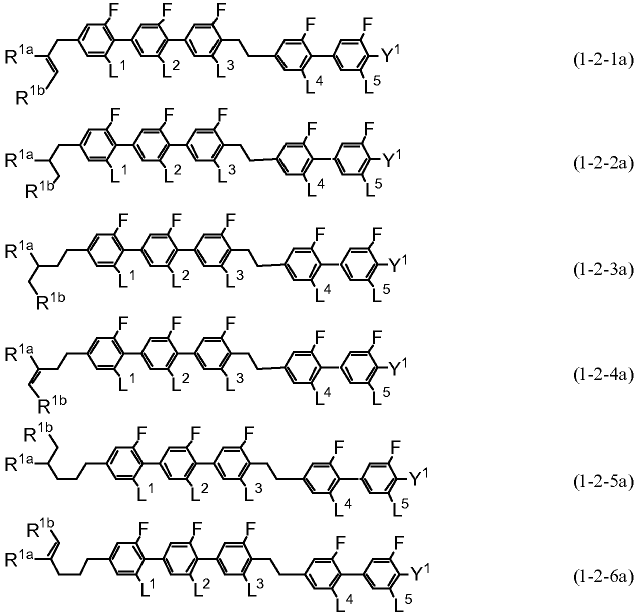

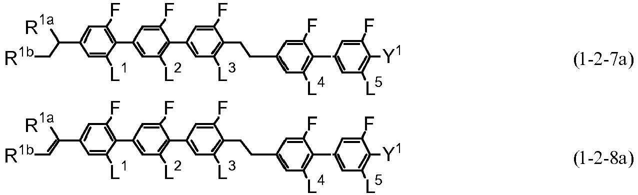

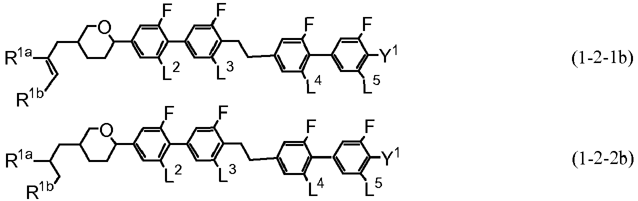

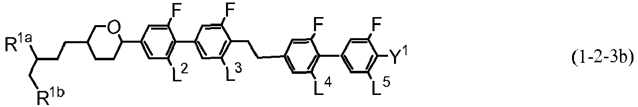

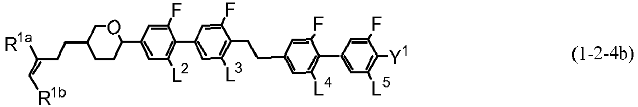

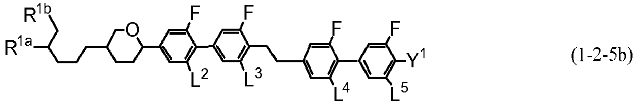

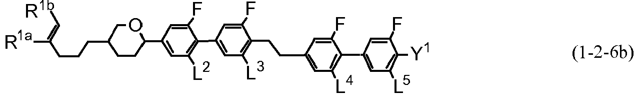

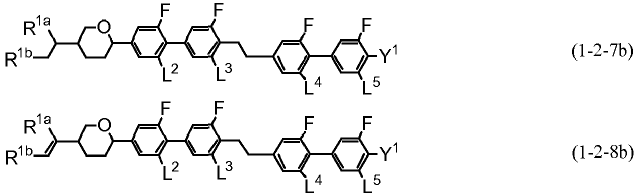

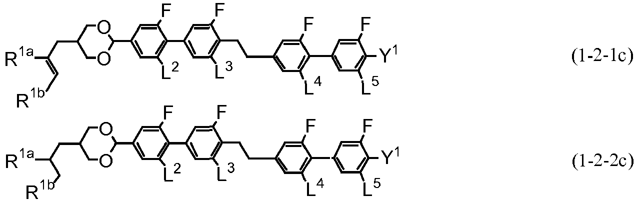

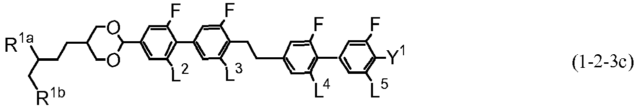

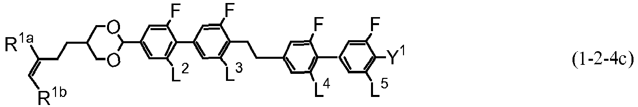

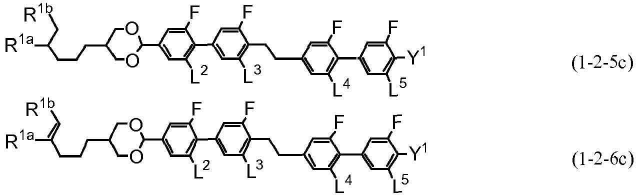

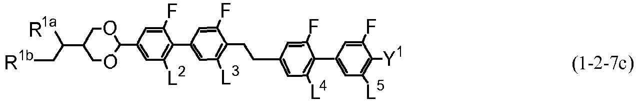

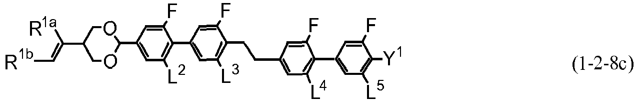

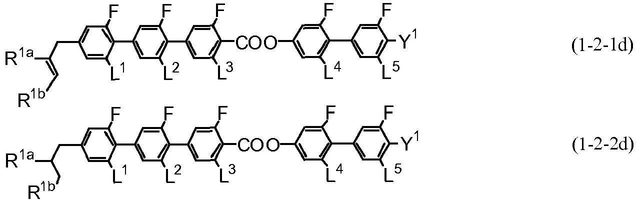

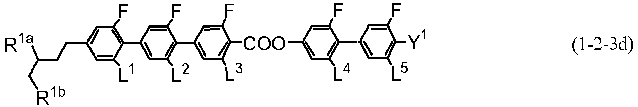

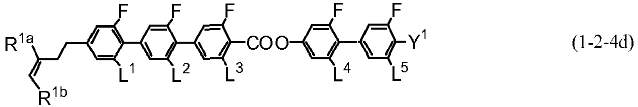

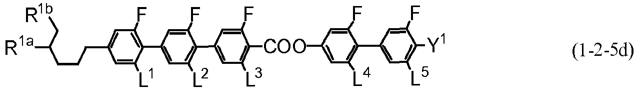

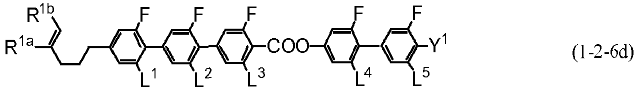

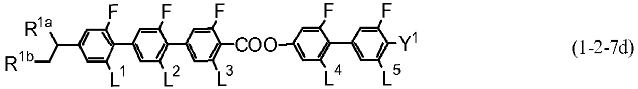

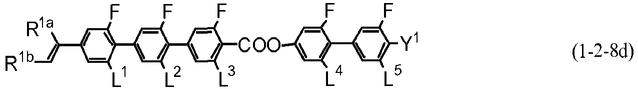

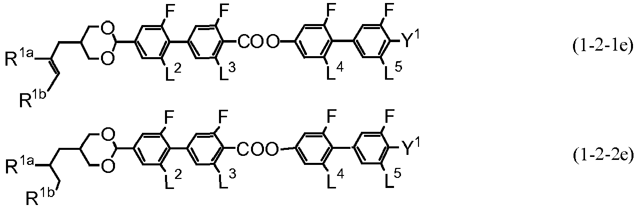

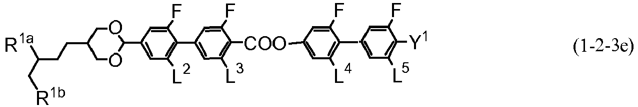

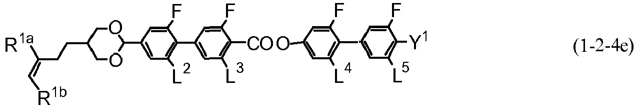

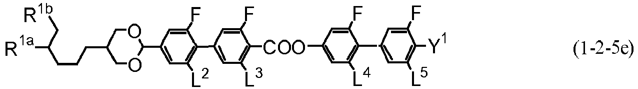

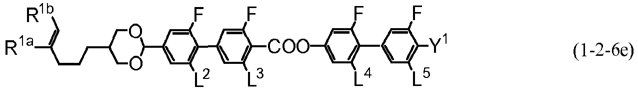

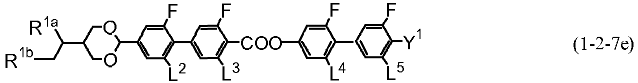

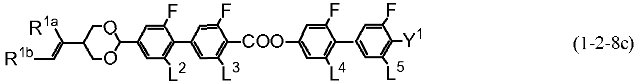

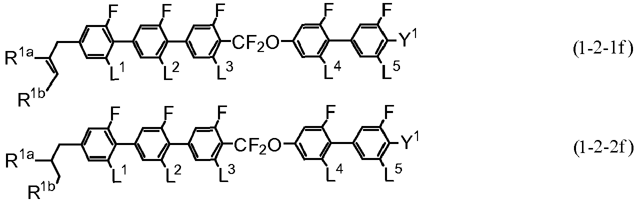

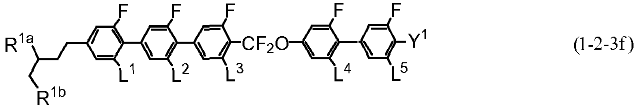

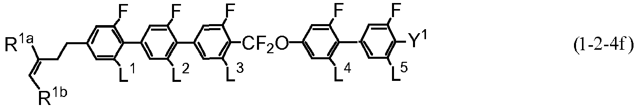

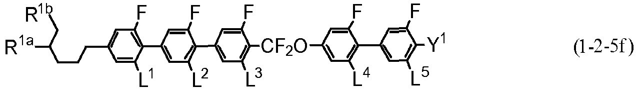

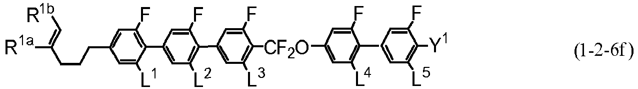

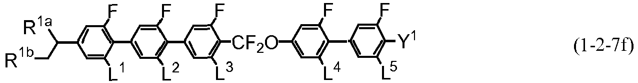

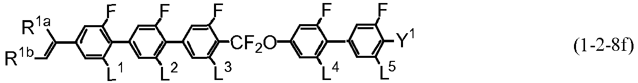

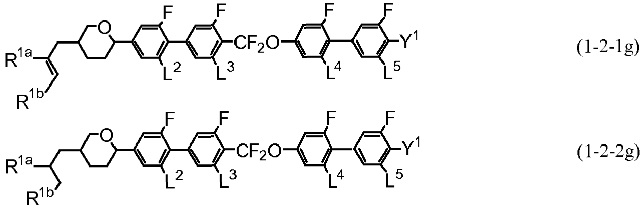

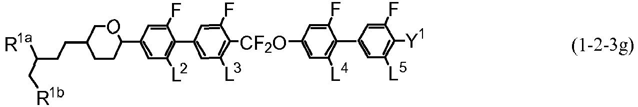

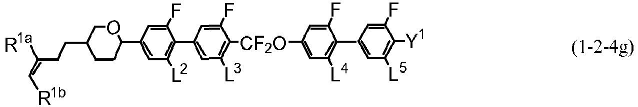

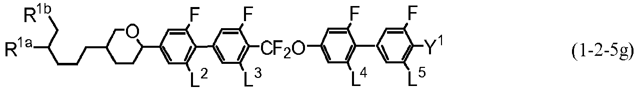

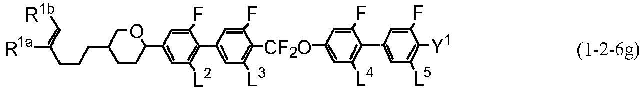

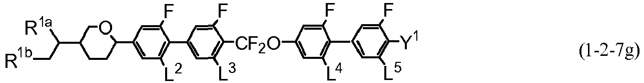

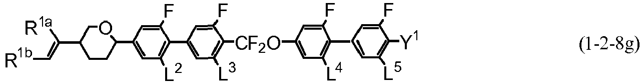

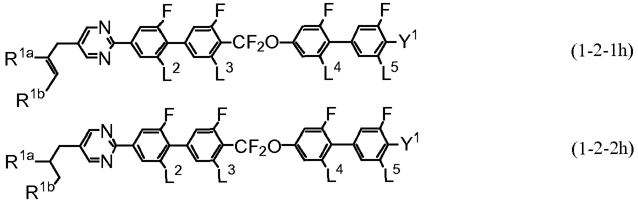

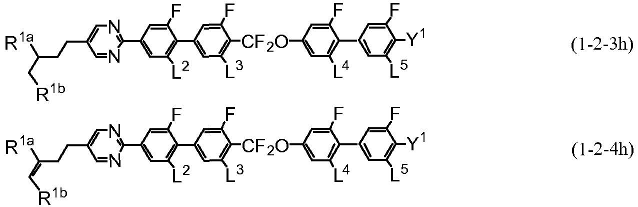

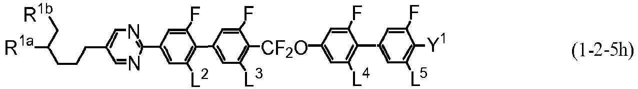

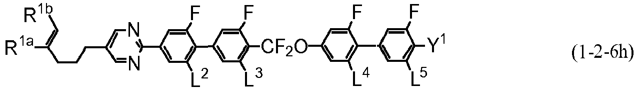

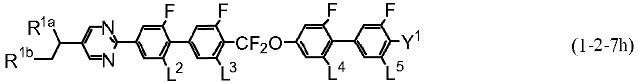

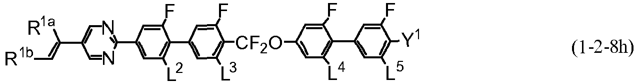

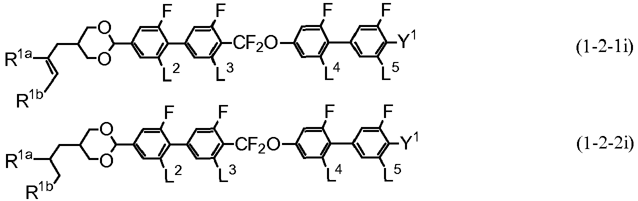

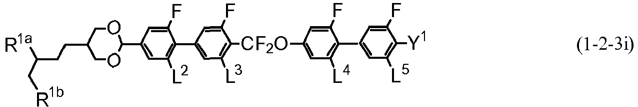

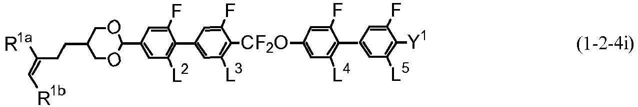

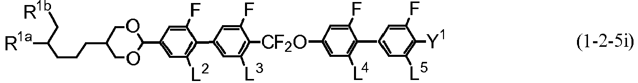

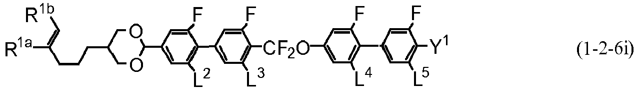

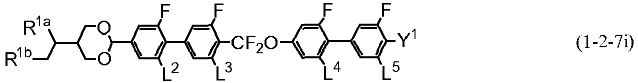

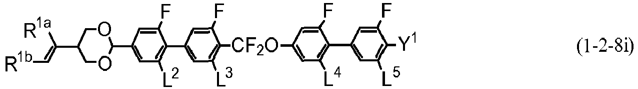

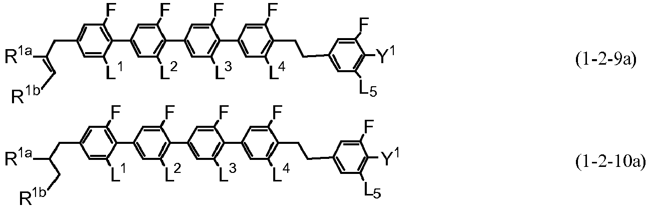

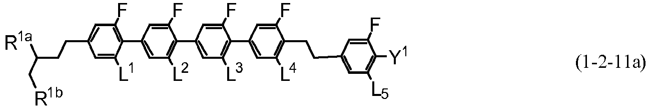

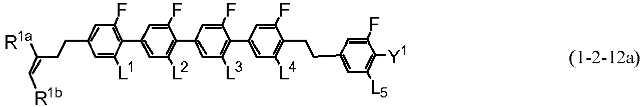

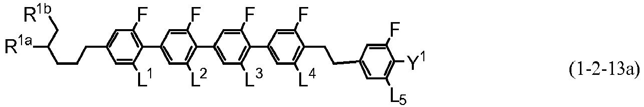

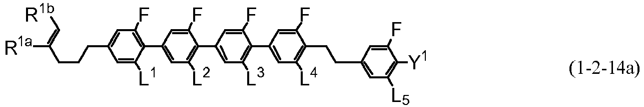

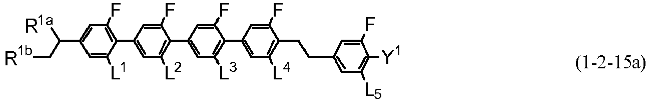

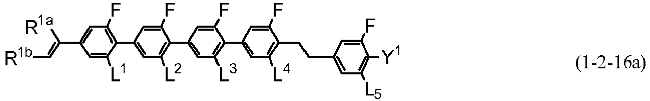

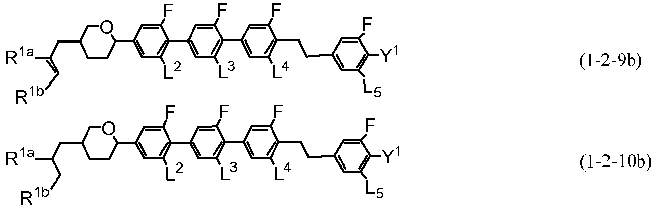

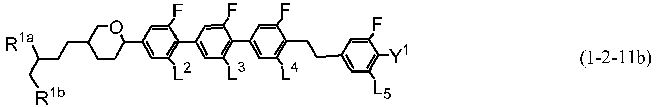

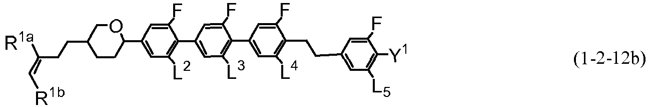

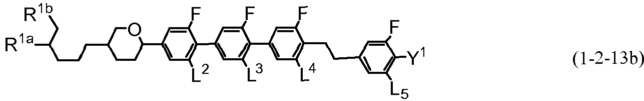

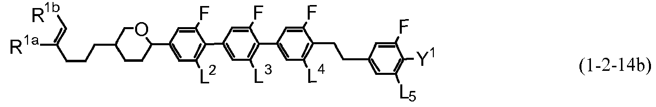

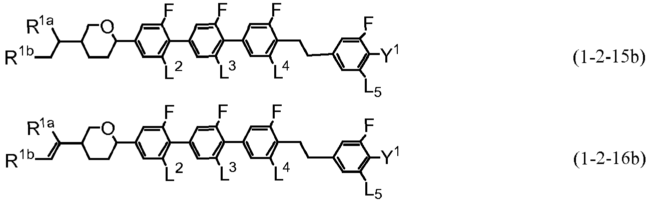

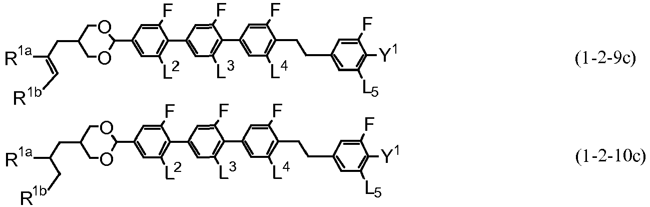

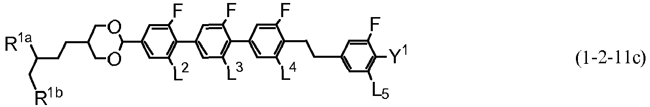

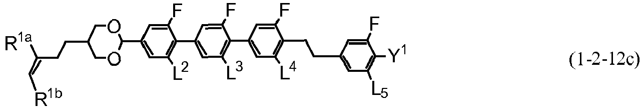

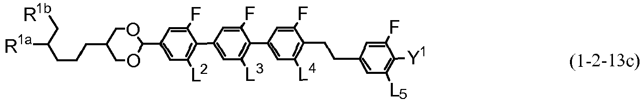

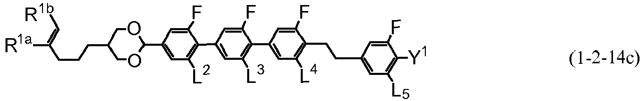

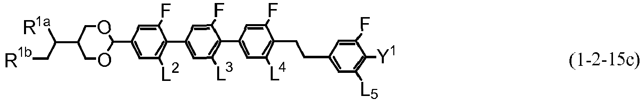

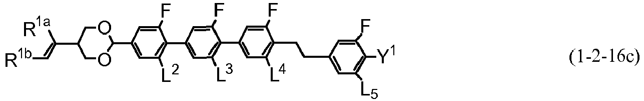

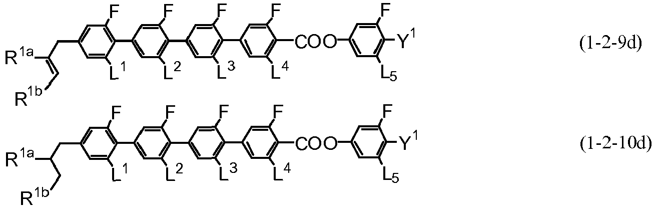

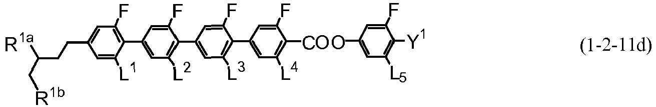

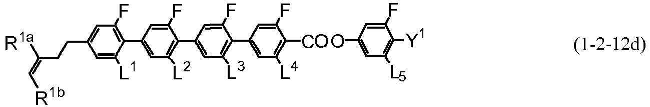

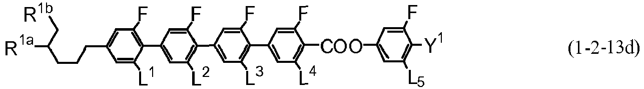

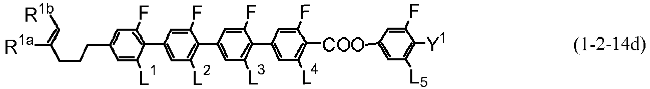

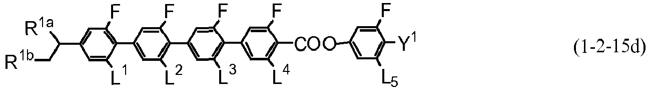

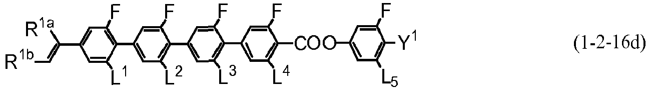

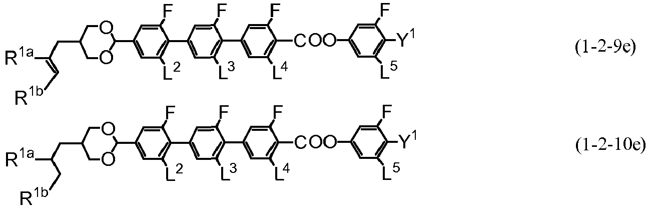

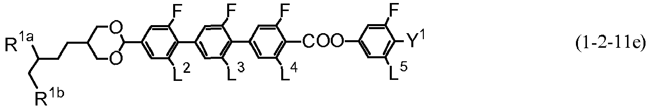

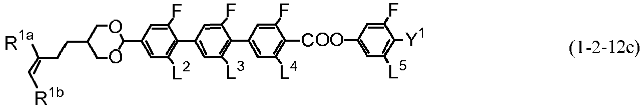

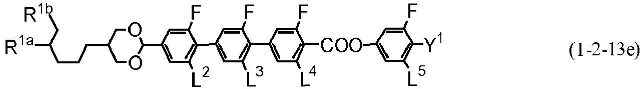

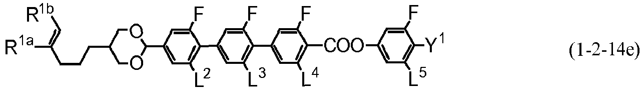

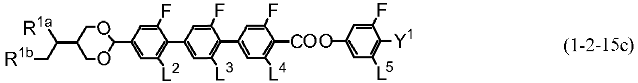

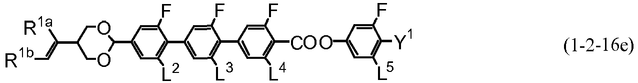

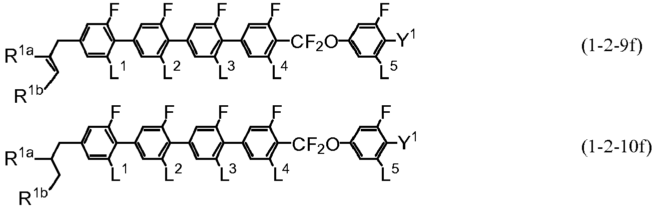

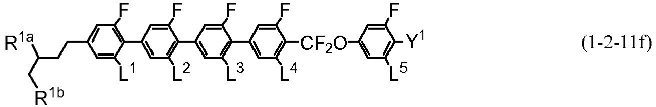

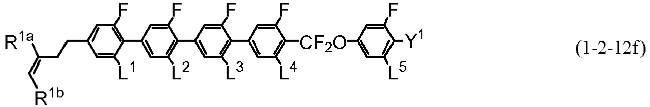

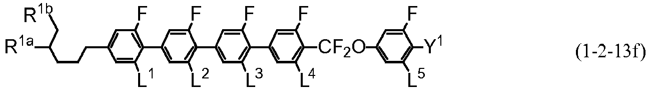

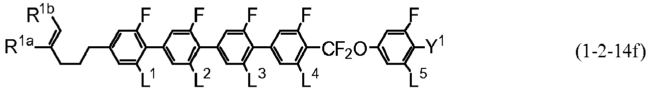

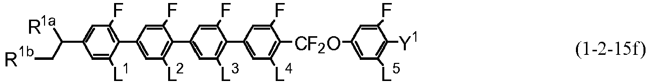

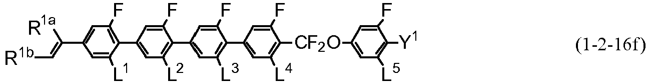

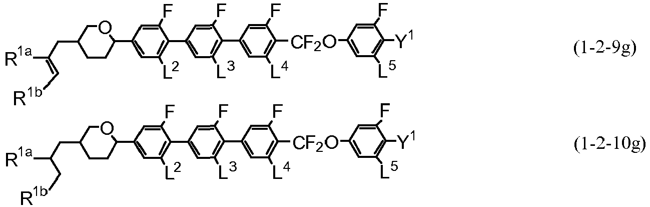

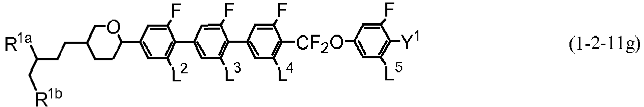

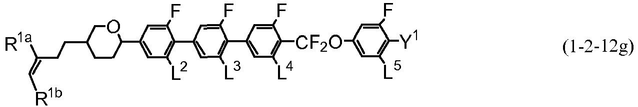

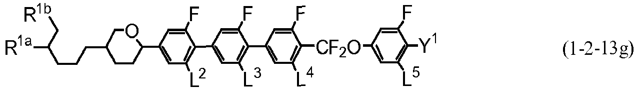

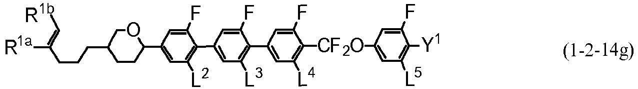

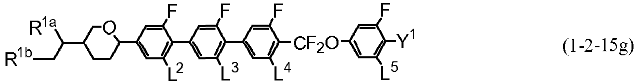

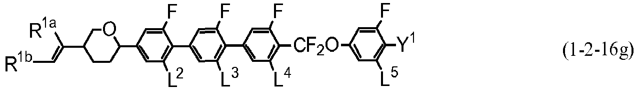

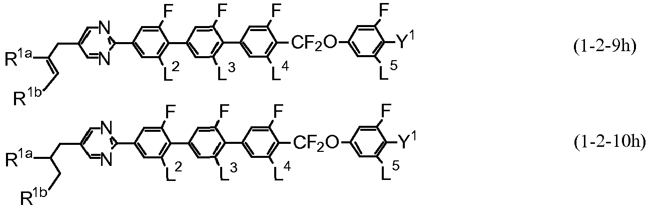

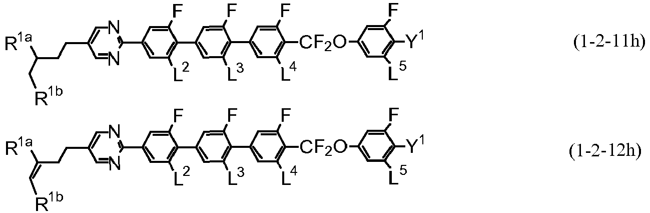

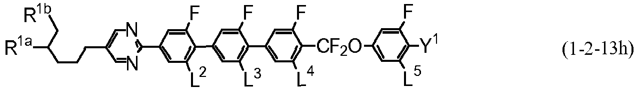

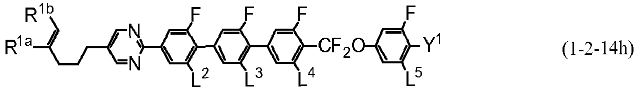

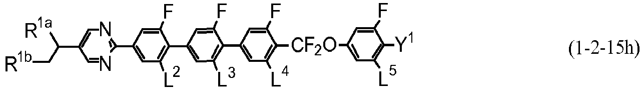

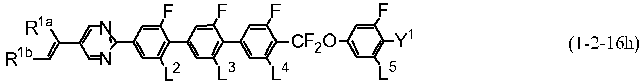

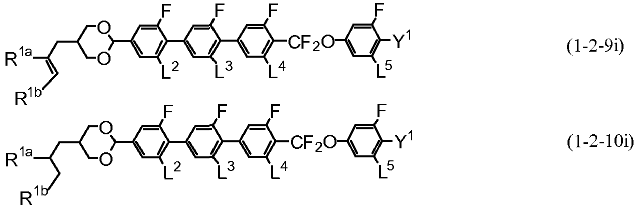

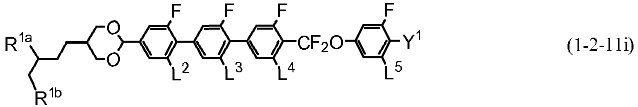

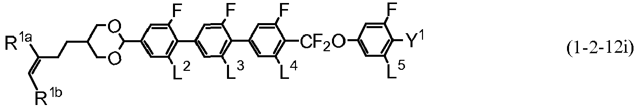

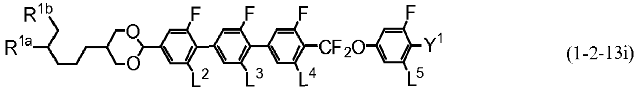

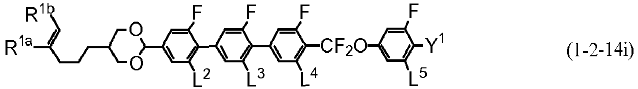

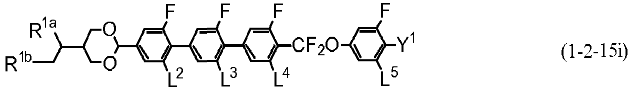

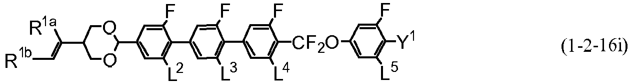

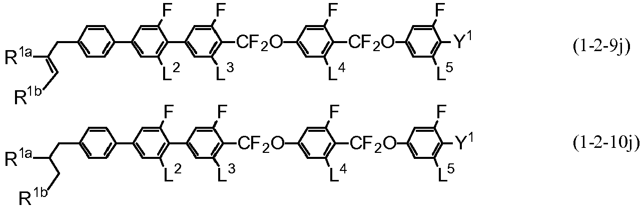

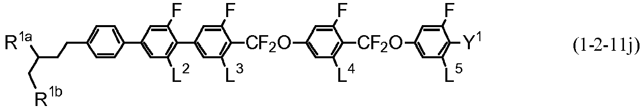

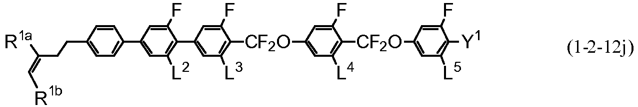

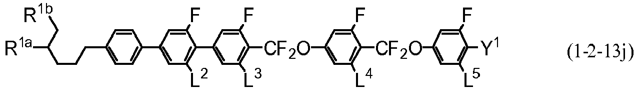

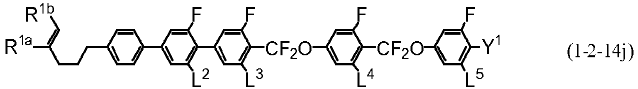

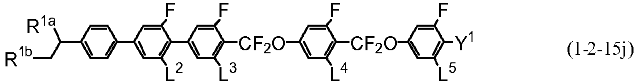

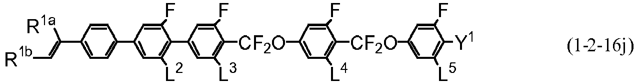

### 1-4. Synthesis of Compound (1)

The synthesis of the compound (1) is described below. A compound (1) can be synthesized by a suitable combination of organic synthesis methods. The methods for introducing target terminal groups, rings and linking groups in the starting compound are described in, for example, Organic Syntheses (John Wiley & Sons, Inc.), Organic Reactions (John Wiley & Sons, Inc.), Comprehensive Organic Synthesis (Pergamon Press), and New Lecture on Experimental Chemistry (Maruzen).

### 1 -4-1. Synthesis of Branched Alkyl

Ther are many methods for introducing a branched alkyl to the left terminal group. However, typically, a branched alkyl is introduced by preparing a halogen derivative (93) of the branched alkyl with the following method, and then conducting a cross coupling reaction of the Grignard reagent of the halogen derivative and a halogen derivative (94) of an aromatic compound or the like in the presence of a palladium catalyst.

In the formula, "Alkyl" represents branched alkyl, X¹ represents halogen, a triflate group, a mesyl group or a tosyl group, and "Core" represents an organic group having a ring structure, an alcohol derivative into which a protective group (Pro) is introduced, or an ester derivative.

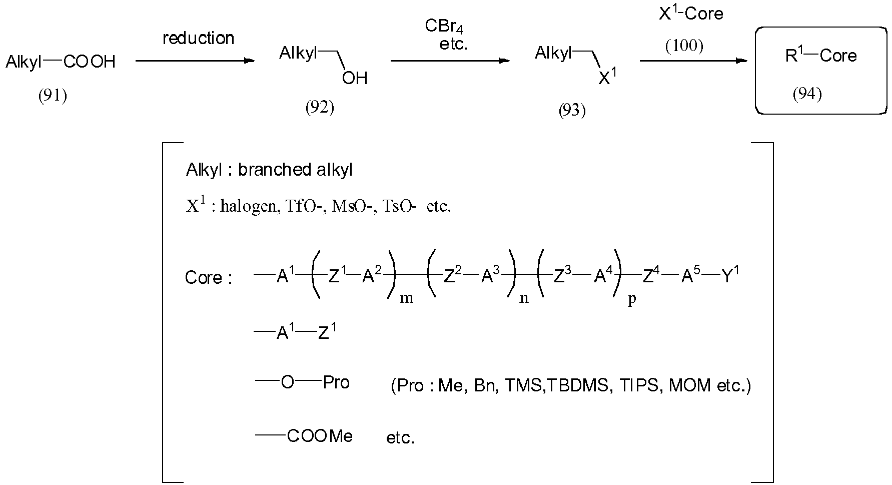

The halogen derivative (93) of the branched alkyl can be a commercially available product, or can be obtained by converting a corresponding carboxylic derivative (91) of the branched alkyl or a corresponding alcohol derivative (92) of the branched alkyl, etc., with a known method.

Moreover, the branched alkyl can be constructed with various methods from general synthesis reagents. An example is described as follows, wherein in the formula, X¹ represents halogen, a triflate group, a mesyl group or a tosyl group, and X² represents MgBr, MgCl or Li.

### [Alkyl, Alkenyl]

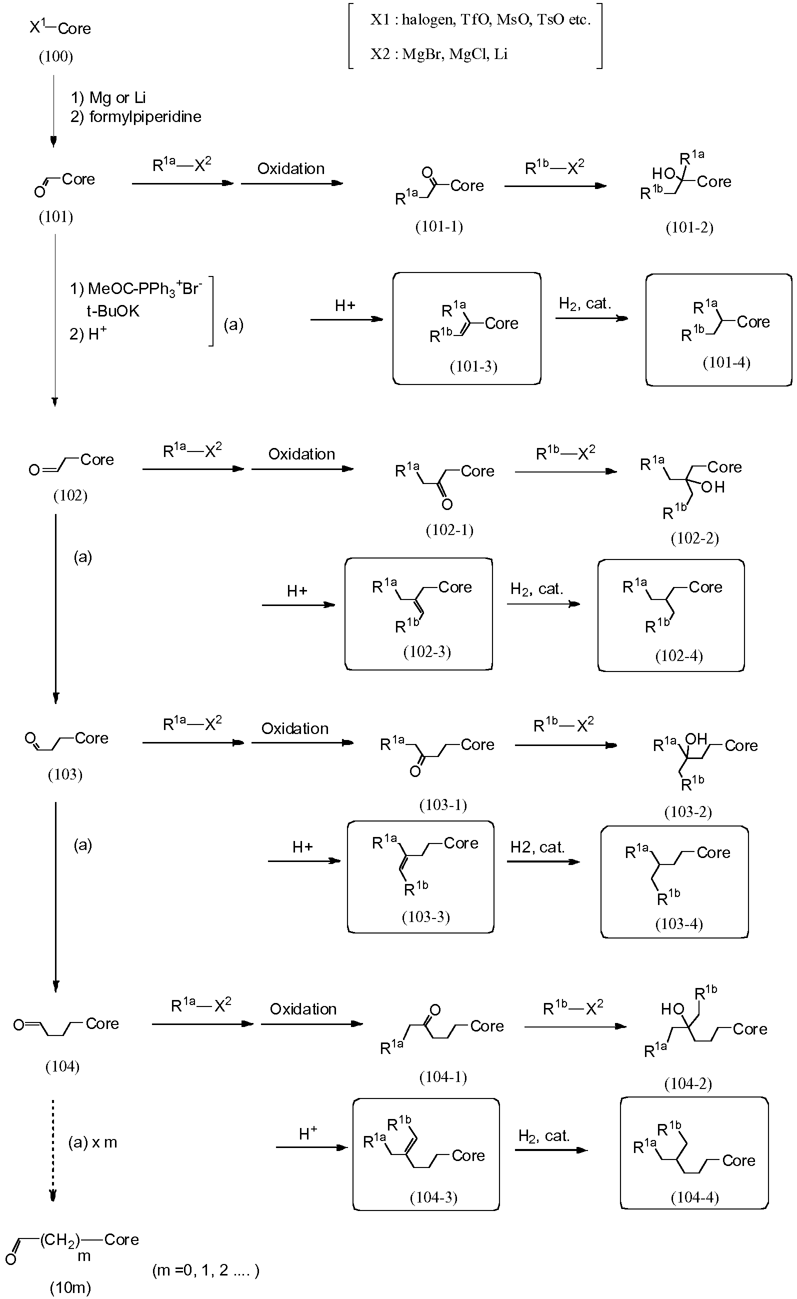

The aldehyde derivative (101) can be derived by preparing a Grignard reagent possibly by reacting magnesium with the corresponding halogen derivative (100), and adding a formylating agent. By repeating the Wittig reaction using (methoxymethyl)-triphenylphosphine bromide and a base, and a subsequent hydrolysis reaction [(a)], an aldehyde derivative [(102), (103), (104) or (10m)] having a required chain length can be prepared from the aldehyde derivative (101).

When an alkyl or alkenyl branching at 2-position is to be synthesized, the compound (102) is reacted with a corresponding alkyl Grignard reagent, etc., and then an oxidation reaction is conducted to derive the compound (102-1). Furthermore, based on the reaction with a corresponding alkyl Grignard reagent, etc., the alcohol derivative (102-2) of the branched alkyl is obtained.

The branched alkenyl (102-3) is obtained by conducting a dehydration reaction with an acid and so on to the alcohol derivative (102-2). Moreover, the branched alkyl (102-4) is obtained by conducting hydrogen reduction to the copound (102-3).

The branched alkenyl (103-3) and the branched alkyl (103-4) each branching at 3-position and not claimed in the present application, the branched alkenyl (104-3) and the branched alkyl (104-4) each branching at 4-position and not claimed in the present application, and the branched alkenyl (101-3) and the branched alkyl (101-4) each branching at 1-position and not claimed in the present application can be synthesized with the same methods.

Moreover, from an aforementioned derivative, such as the compound (103-1), it is also possible to synthesize a branched alkenyl [(103-5) or (103-6)] using the Tebbe reaction, Wittig reaction or olefin metathesis reaction shown below.

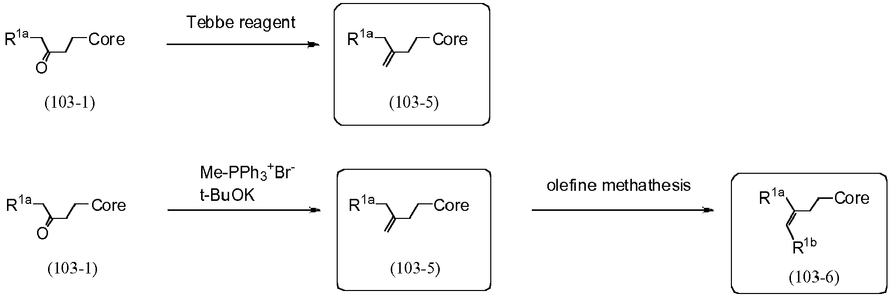

Moreover, a reference example of the method of introducing oxygen into a branched alkyl or a branched alkenyl is shown beow.

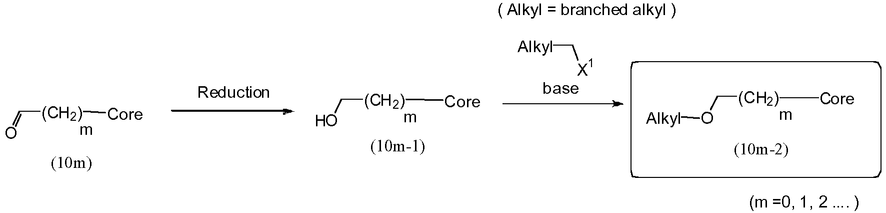

An ether bond can be introduced into a branched alkyl by deriving the alcohol derivative (10m-1) from a corresponding derivative, such as the compound (10m), with a reduction reaction, and then reacting the same with a halogen derivative and a base for etherification. An ether bond can also be introduced into a branched alkenyl with the same method.

In addition, a reference example of the method of introducing an alkyne moiety into a branched alkyl or a branched alkenyl is shown beow.

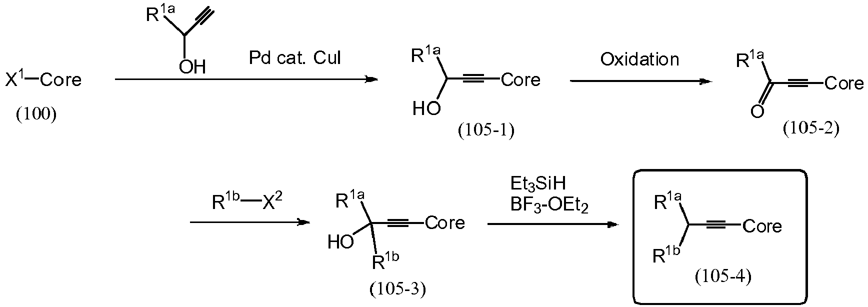

A compound (105-1), not claimed in the present application, is obtained from the corresponding halogen derivative (100) by reacting the same with an alkyne derivative through a Sonogashira reaction using a palladium catalyst and Cul. Then, an oxidation reaction and a Grignard reaction are conducted to convert the compound (105-1) to the compound (105-3). A branched compound (105-4) can be obtained by reacting the compound (105-3) with triethylsilane and boron halide.

### 1-4-2. Method for Forming Linking Groups Z¹ to Z⁴

An example of the method for forming the linking groups Z¹-Z⁴ in the compound (1) is shown in the following scheme. In this scheme, MSG¹ or MSG² is a monovalent organic group having at least one ring. The multiple MSG¹'s (or MSG²'s) used in the scheme can be identical or different. The compounds (1A)-(1J) are within the scope of compound (1).

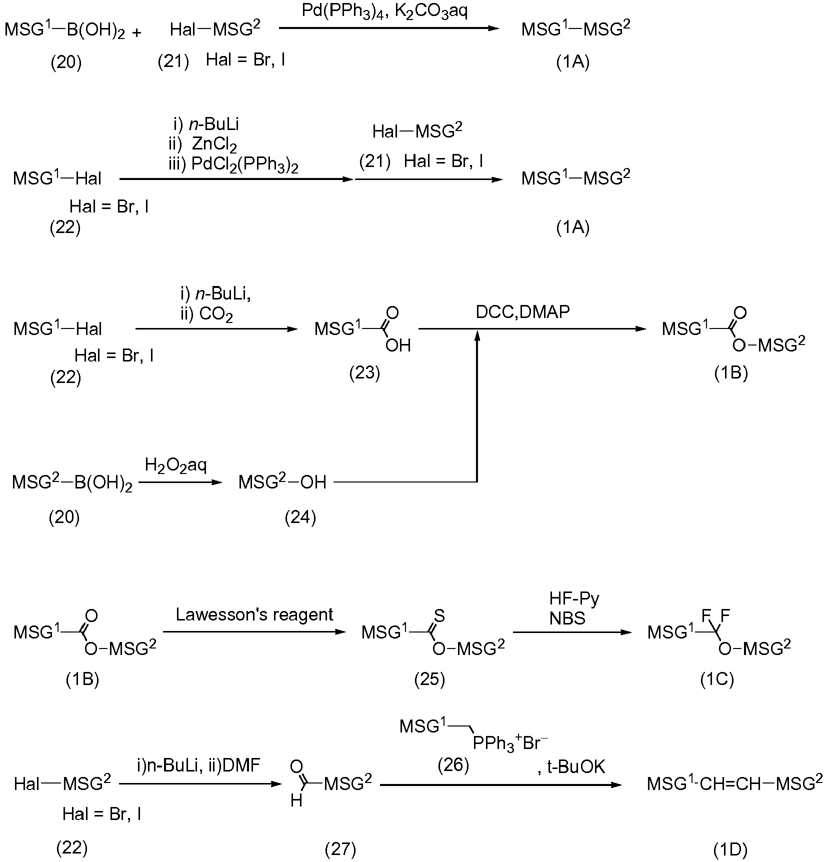

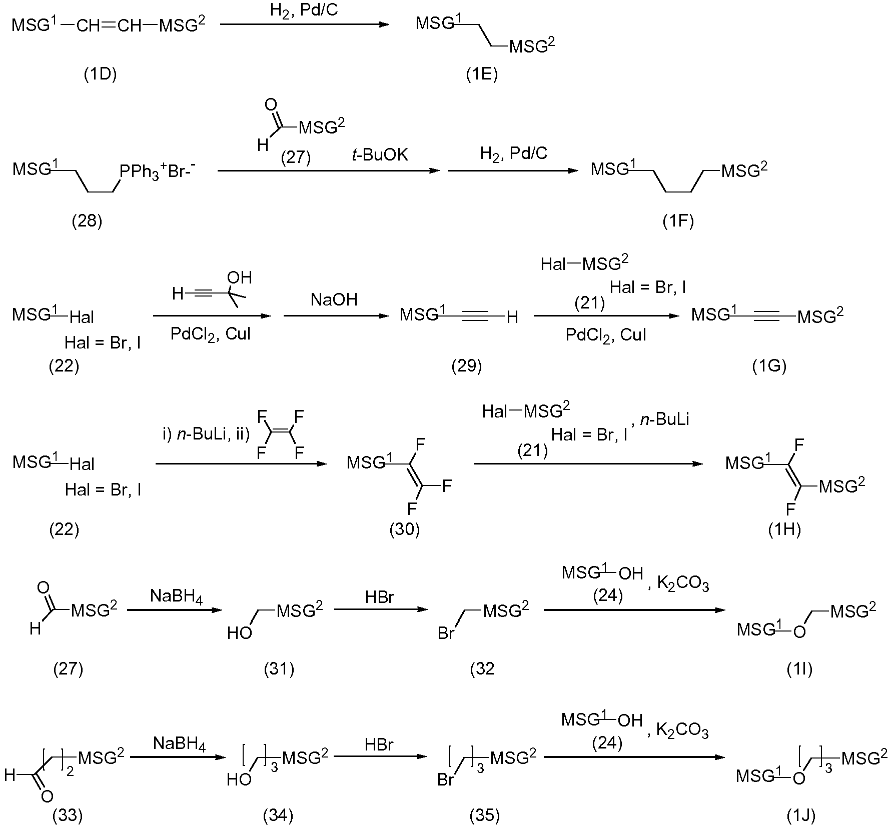

Next, the methods for forming each of the linking groups Z¹ to Z⁴ in the compound (1) are described in Items (I)-(X) below.

### (I) Formation of Single Bond

Arylboric acid (20) is reacted with a compound (21) synthesized with a well- known method, in the presence of an aqueous carbonate solution and a catalyst such as tetrakis(triphenylphosphine)palladium, to synthesize a compound (1A). The compound (1A) could alternatively be synthesized by reacting *n*-butyl lithium with a compound (22) synthesized by a well-known method, with zinc chloride, and then with the compound (21) in the presence of a catalyst such as bis(triphenylphosphine)palladium dichloride.

### (II) Formation of -COO- and -OCO-

The compound (22) is reacted with *n*-butyl lithium and then with carbon dioxide to produce a carboxylic acid (23). Next, the compound (23) and a phenol compound (24) synthesized with a well-known method are subjected to dehydration in the presence of DCC (1,3-dicyclohexylcarbodiimide) and DMAP (4-dimethylaminopyridine) to synthesize a compound (1B) having -COO-. A compound having -OCO- could also be synthesized through this process.

### (III) Formation of -CF₂O- and -OCF₂-

The compound (1B) is treated with a vulcanizing agent such as Lawesson's reagent to produce a compound (25). Then the compound (25) is fluorinated with hydrogen fluoride pyridine complex and NBS (N-bromosuccinimide) to synthesize a compound (1C) having -CF₂O-, as described in M. Kuroboshi et al., Chem. Lett., 1992, 827. The compound (1C) could alternatively be synthesized by fluorinating the compound (25) with (diethylamino)sulfur trifluoride (DAST), as described in W. H. Bunnelle et al., J. Org. Chem. 1990, 55, 768. A compound having -OCF₂- could also be synthesized through this process. These linking groups could alternatively be formed through the process described in Peer. Kirsch et al., Angew. Chem. Int. Ed. 2001, 40, 1480.

### (IV) Formation of -CH=CH-

The compound (22) is treated with *n*-butyl lithium and then reacted with a formamide such as N,N-dimethylformamide (DMF) to produce an aldehyde (27). Then, a phosphosium salt (26) synthesized with a well-known method is treated with a base such as potassium *t*-butoxide, so as to produce a phosphorus ylide, which is then reacted with the aldehyde (27) to produce a compound (1D). A *cis*-compound is produced under the reaction conditions, and, if necessary, may be isomerized to a *trans-* compound by a well-known method.

### (V) Formation of -(CH₂)₂-

The compound (1D) is hydrogenated in the presence of a catalyst, such as Pd/C, to produce a compound (IE).

### (VI) Formation of -(CH₂)₄-

A compound having -(CH₂)₂-CH=CH- is obtained following the process of (IV) using a phosphonium salt (28) instead of the phosphonium salt (26), which is then catalytically hydrogenated to synthesize a compound (IF).

### (VII) Formation of -C≡C-

2-Methyl-3-butyn-2-ol is reacted with the compound (22) in the presence of a catalyst containing palladium dichloride and copper halide, and then deprotected under a basic condition to produce a compound (29). Next, the compound (29) is reacted with the compound (21), in the presence of a catalyst containing bis(triphenylphosphine) palladium dichloride and copper halide, to produce a compound (1G).

### (VIII) Formation of -CF=CF-

The compound (22) is treated with *n*-butyl lithium and then reacted with tetrafluoroethylene to produce a compound (30). The compound (21) is treated with n-butyl lithium and then reacted with the compound (30) to produce a compound (1H).

### (IX) Formation of -CH₂O- or -OCH₂-

The compound (27) is reduced with a reductant such as sodium borohydride to produce a compound (31), which is then halogenated with, for example, hydrobromic acid, to produce a compound (32). The compound (32) is reacted with the compound (24) in the presence of potassium carbonate and so on to produce a compound (1I).

### (X) Formation of -(CH₂)₃O- or -O(CH₂)₃-

A compound (1J) is synthesized according to the same process as (IX), using a compound (33) instead of the compound (27).

### 1-4-3. Method for Synthesizing Rings A¹ to A⁵

For rings such as 1,4-phenylene, 1,3-dioxane-2,5-diyl, 2-fluoro-1,4-phenylene, 2,3-difluoro-1,4-phenylene, 2,5-difluoro-1,4-phenylene, 2,6-difluoro-1,4-phenylene, 2,3,5,6-tetrafluoro-1,4-phenylene, pyrimidine-2,5-diyl and pyridine-2,5-diyl, the starting materials are commercially available or can be synthesized with well-known methods.

### 1-4-4. Method for Synthesizing Compound (1)

There are numerous processes for synthesizing a compound of formula (1), suitably from commercially available reagents, in reference to the embodiments in this specification, or literatures and books. An example of the processes is shown below. The method for synthesizing the compound (1) is not limited to this synthesis example.

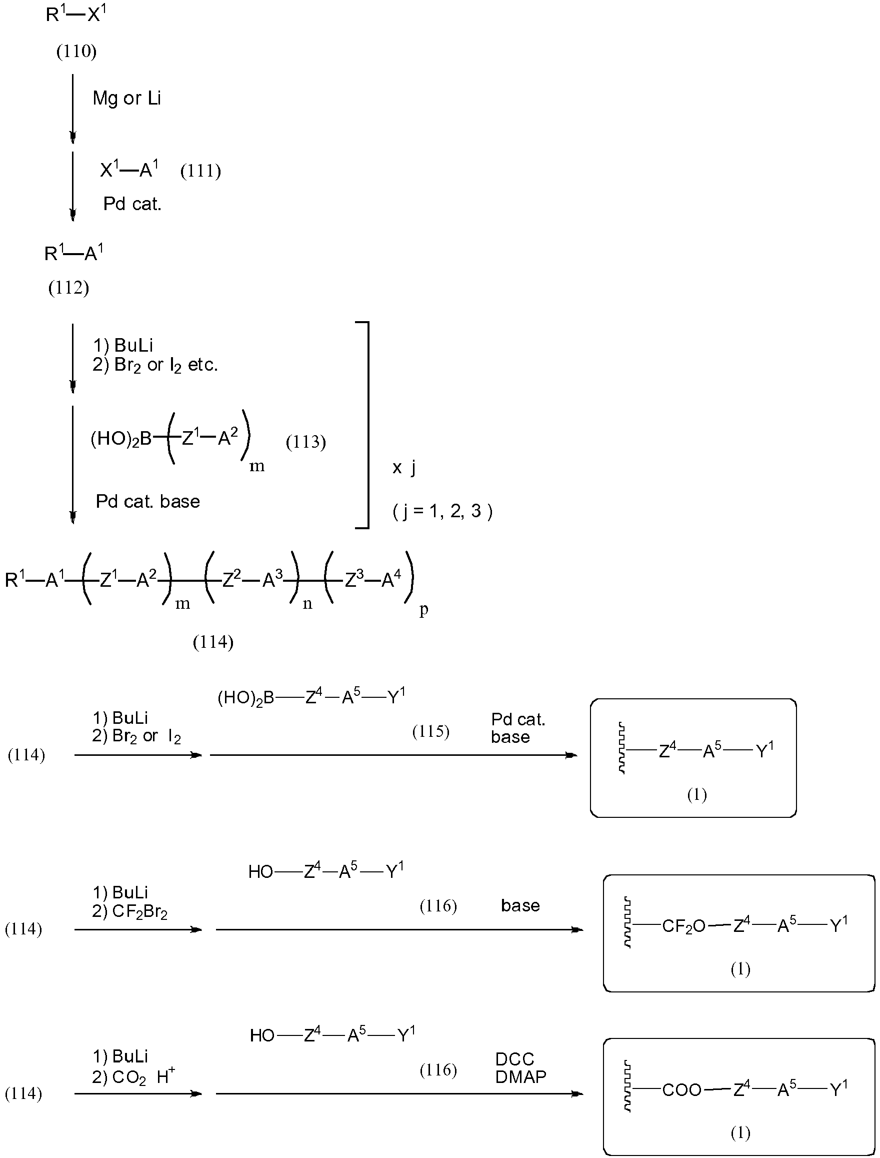

A compound (112) can be obtained by firstly preparing the halogen derivative (110) of branch alkyl with the method shown in 1-4-1, reacting the same with magnesium or the like, and conducting a cross coupling reaction to the product with a corresponding halogen derivative (111). After the compound (112) is converted to a halogen derivative or the like, a coupling reaction using a palladium catalyst is conducted, and an intermediate (114) can be obtained by repeating the conversion and the coupling for a required number of cycles.

### Starting from the intermediate (114) as a raw material, a variety of compounds (1) can be obtained.

For example, by further conducting a coupling reaction with a boric acid derivative (115), a compound (1) having a single bond can be obtained.

Moreover, by reacting the intermediate (114) with alkyl lithium and dibromodifluoromethane and then conducting an etherification reaction with a phenol derivative (116), a compound (1) having a CF₂O bonding can be obtained.

Further, by converting the intermediate (114) to a carboxylic derivative using alkyl lithium and dry ice, and then conducting an esterification reaction using DCC (1,3-dicyclohexylcarbodiimide) and DMAP (4-dimethylaminopyridine), a compound (1) having an ester bond can be obtained.

### 2. Compounds (2)-(11)

A second aspect of this invention relates to a liquid crystal composition obtained by adding a component selected from the components B, C, D and E shown below to the compound of formula (1) as the component A. As compared with a composition containing the component A alone, the liquid crystal composition can be readily adjusted for the driving voltage, temperature range of liquid crystal phase, optical anisotropy, dielectric anisotropy, viscosity and so on.

Preferably, the component added to the component A is a mixture obtained by mixing a component B, C, D, E or F, wherein the component B includes at least one compound selected from the group consisting of the compounds of formulae (2), (3) and (4) above, the component C includes at least one compound selected from the group consisting of the compounds of formula (5) above, the component D includes at least one compound selected from the group consisting of the compounds of formula (6), the component E includes at least one compound selected from the group consisting of the compounds of formulae (7) to (10), and the component F includes at least one compound selected from the group consisting of the compounds of formula (11).

Moreover, for each component of the liquid crystal composition used in this invention, even an analogue containing isotopes of the elements can be used due to the little difference in the physical properties.

In the component B above, suitable examples of the compound of formula (2) are expressed by formulae (2-1)-(2-16), suitable examples of the compound of formula (3) are expressed by formulae (3-1)-(3-112), and suitable examples of the compound of formula (4) are expressed by formulae (4-1)-(4-52).

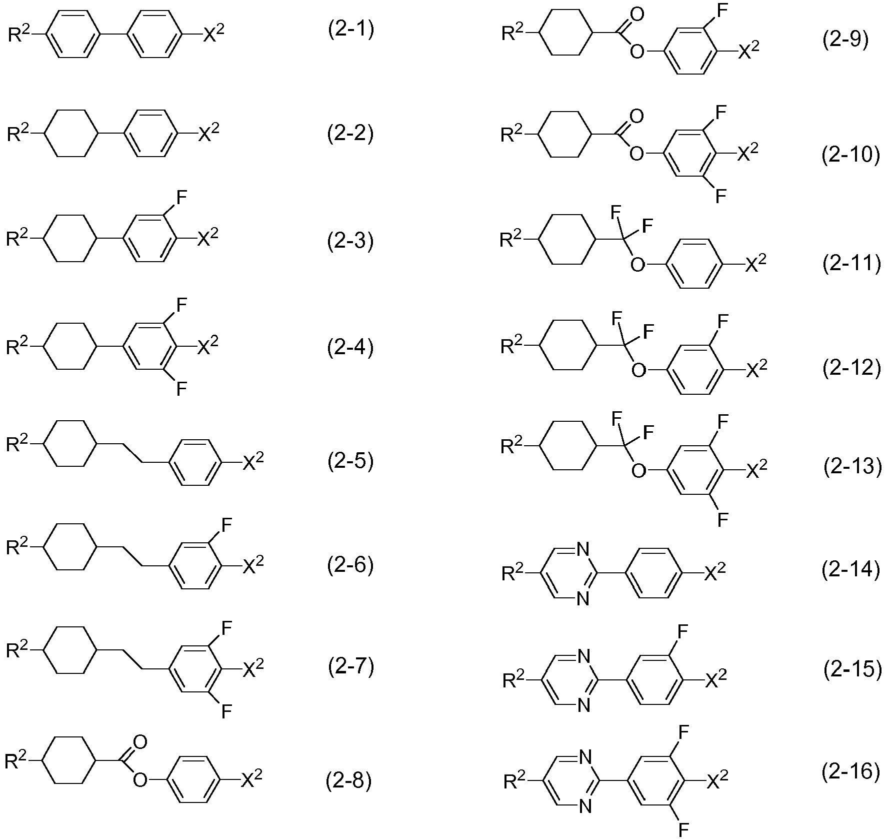

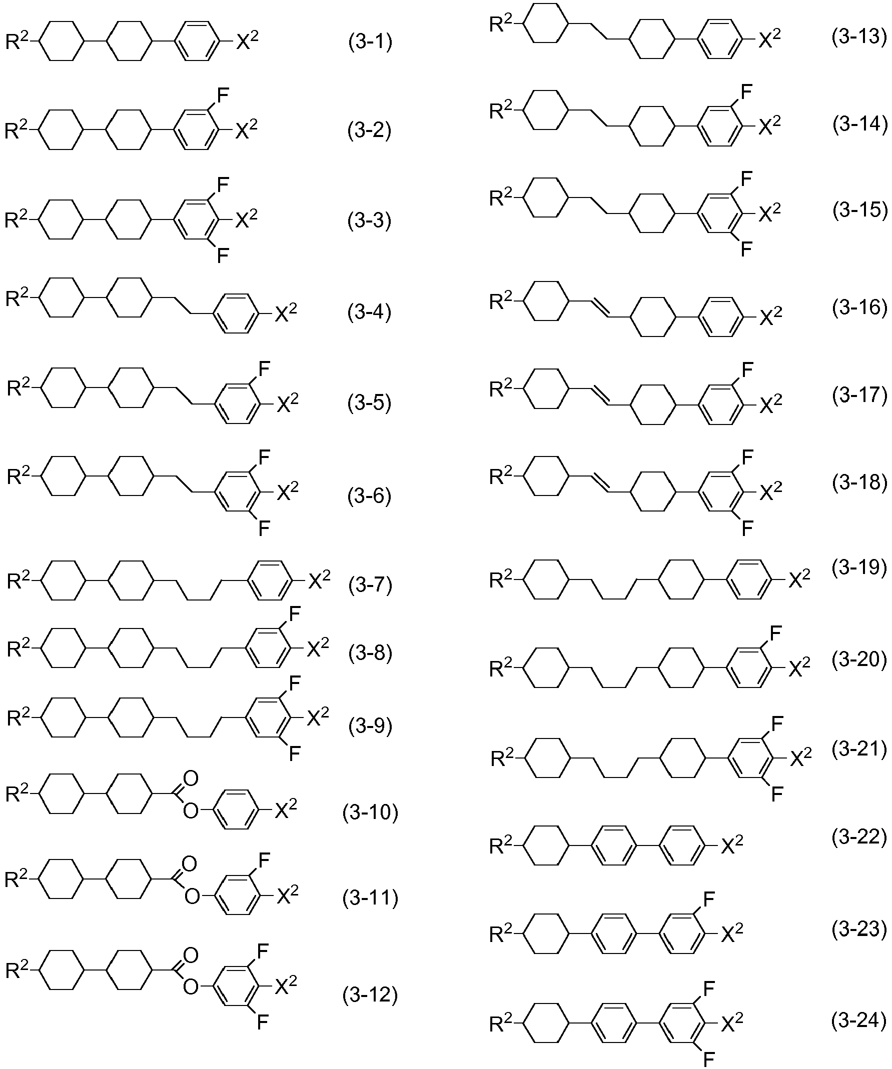

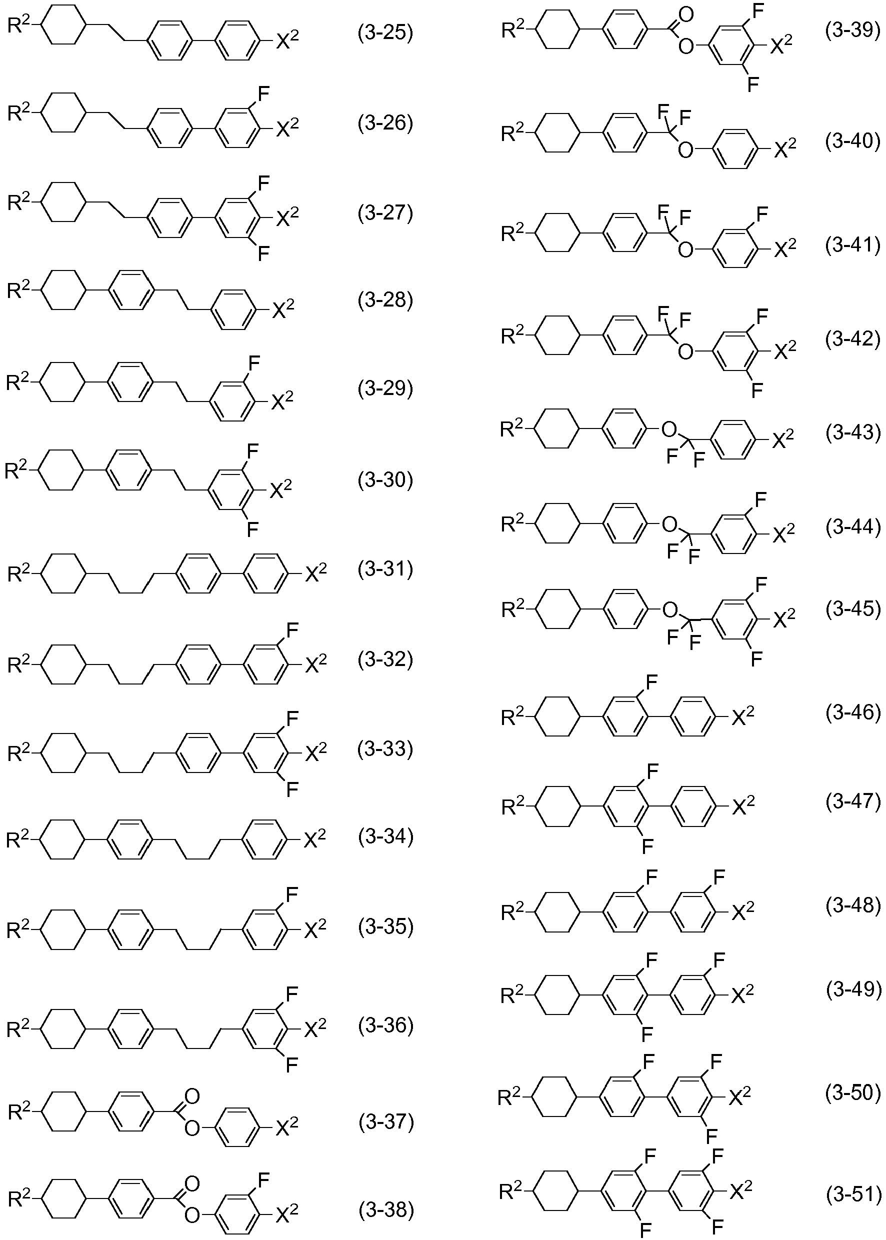

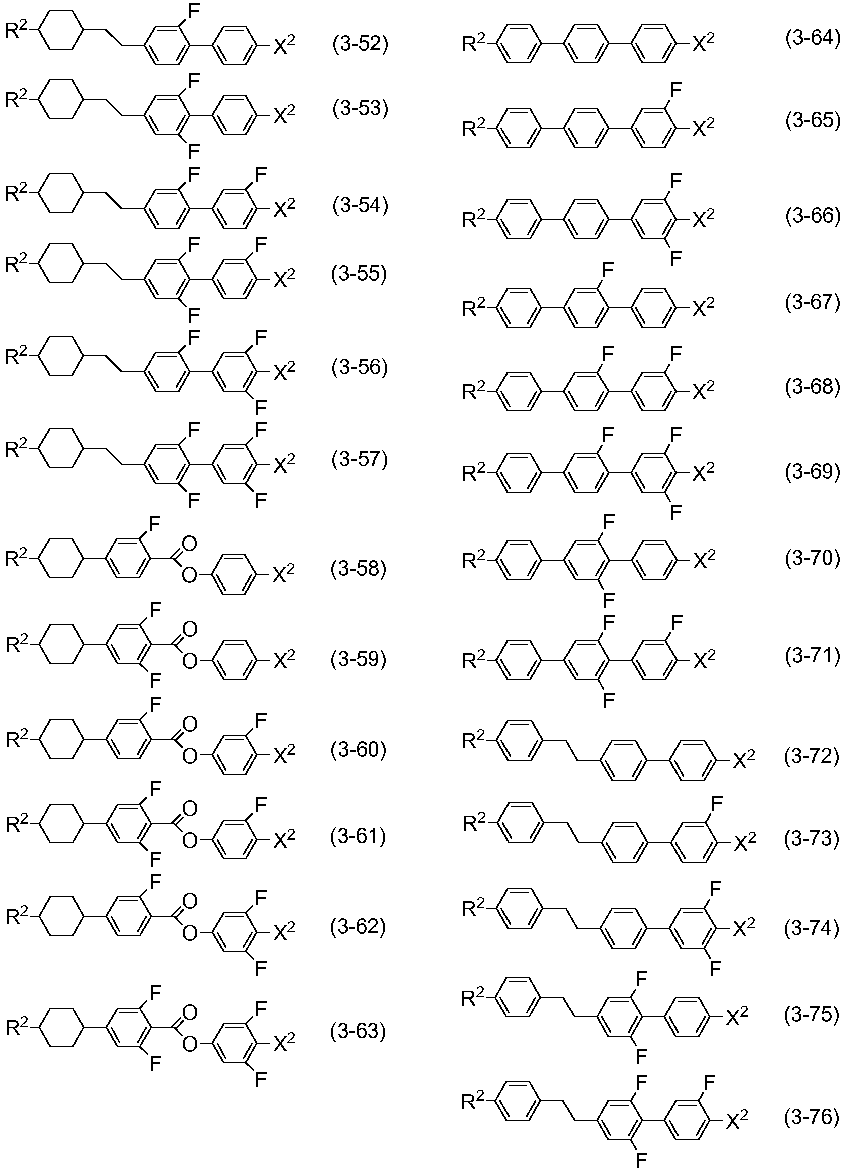

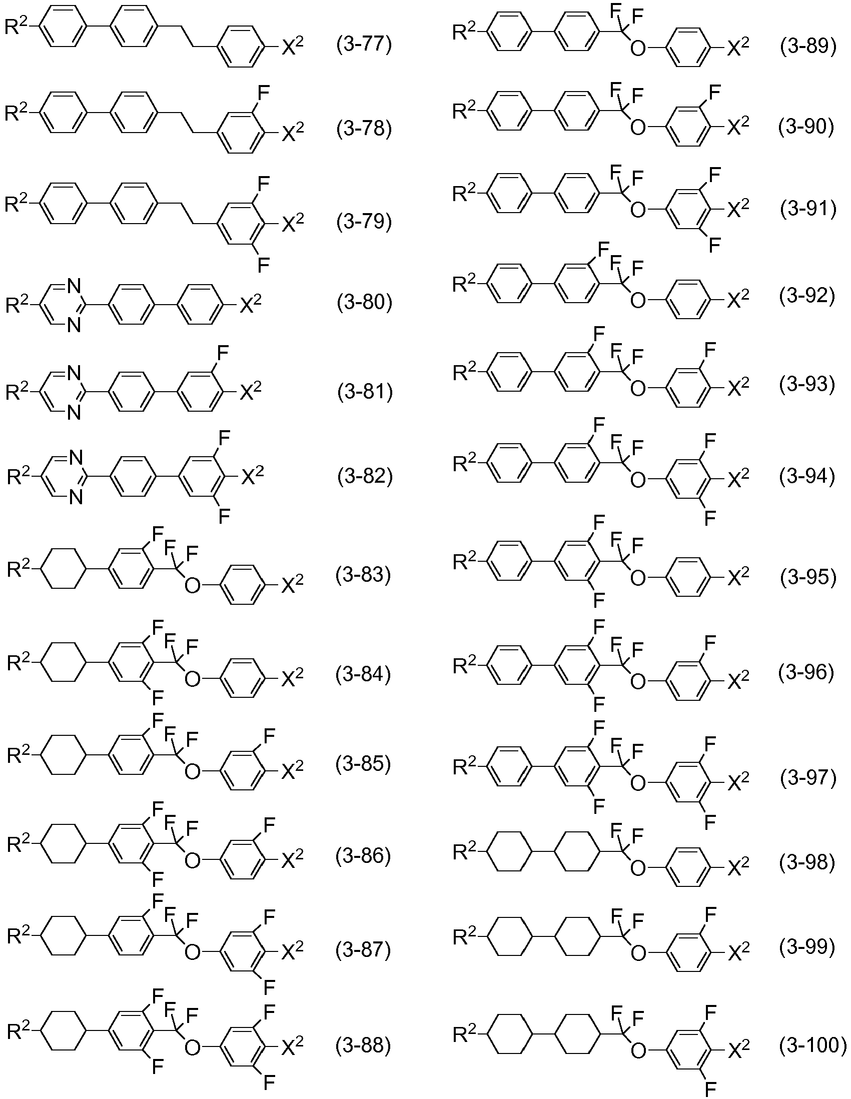

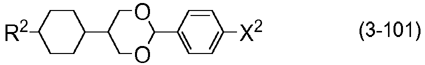

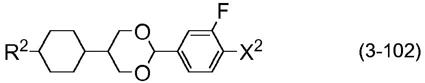

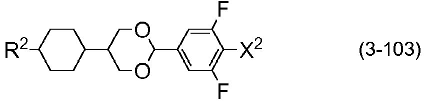

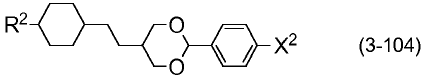

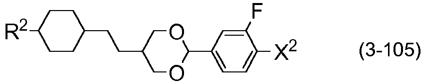

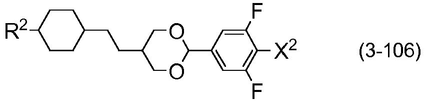

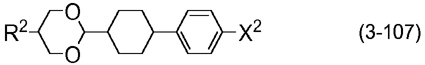

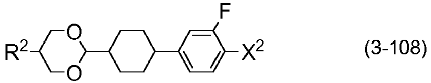

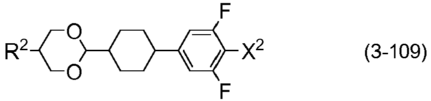

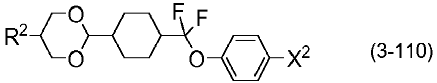

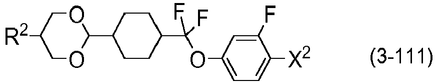

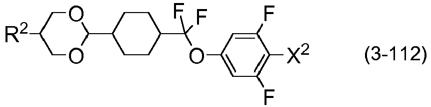

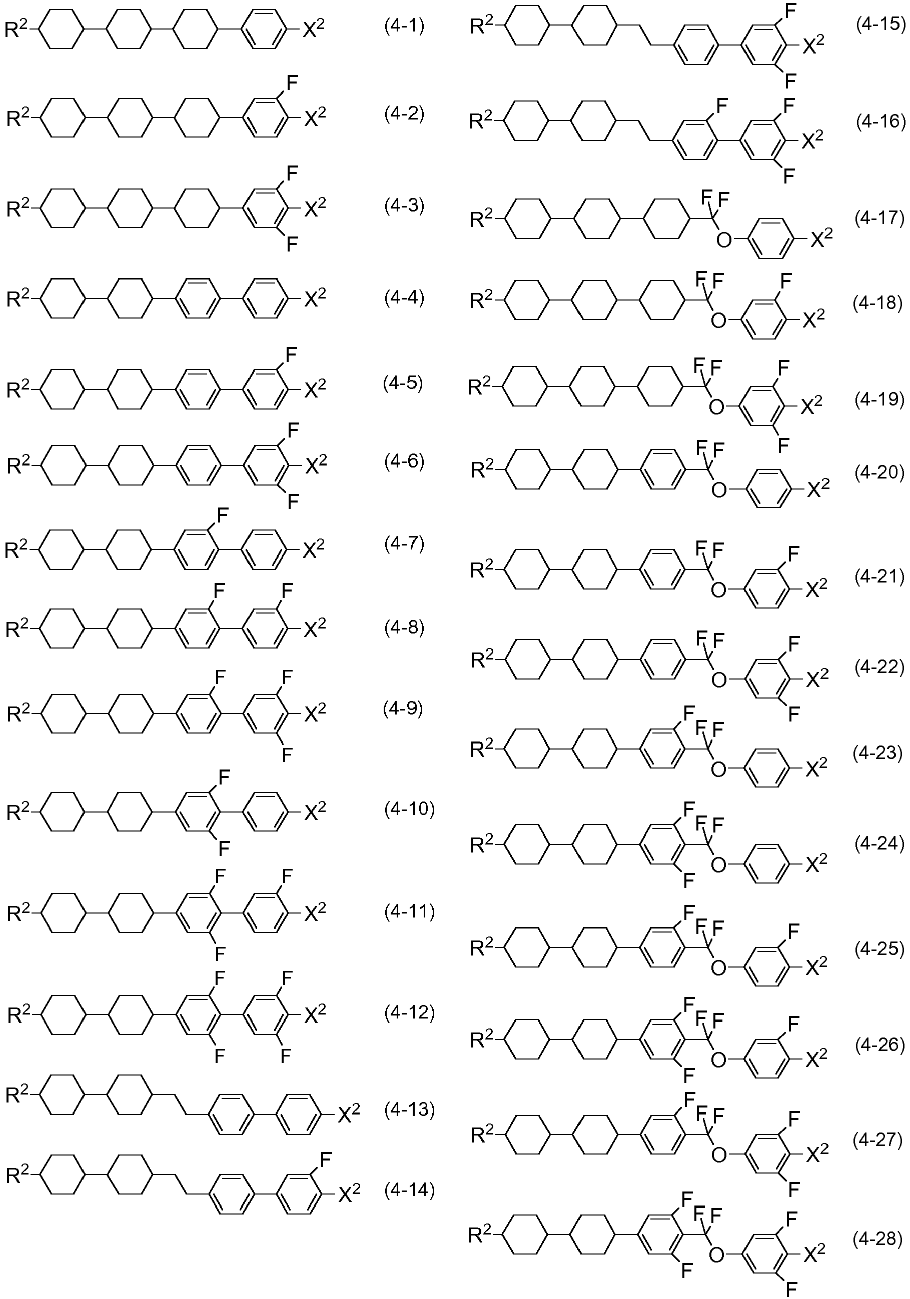

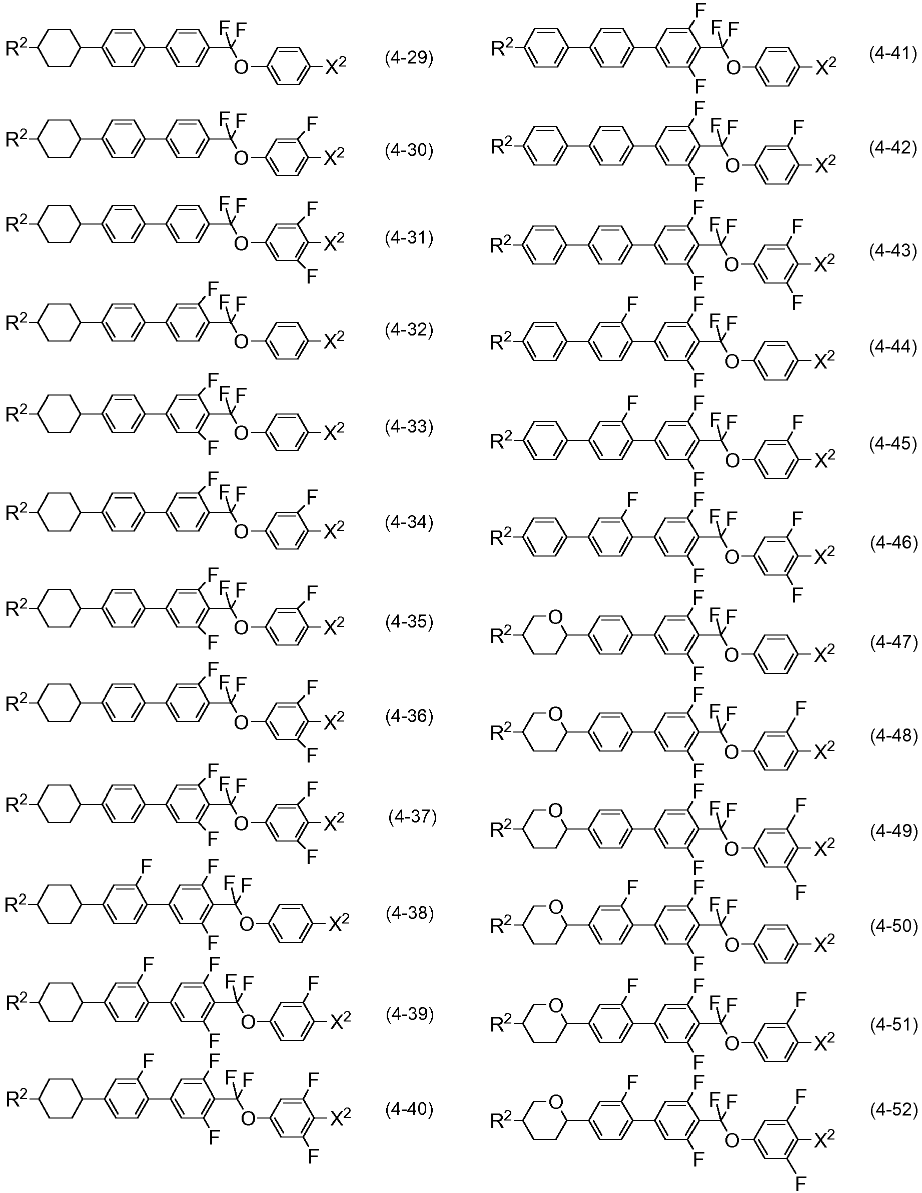

In the formulae, R² and X² are defined as above.

The compounds of formulae (2) to (4) (component B) have positive dielectric anisotropy and very high thermal or chemical stability, and are therefore useful for preparing liquid crystal compositions for TFT elements. Relative to the total weight of the liquid crystal composition, the content of the component B may be from 1 wt% to 99 wt%, preferably from 10 wt% to 97wt% and more preferably from 40 wt% to 95 wt%.

Suitable examples of the compounds of formula (5) (component C) are those of formulae (5-1) to (5-62).

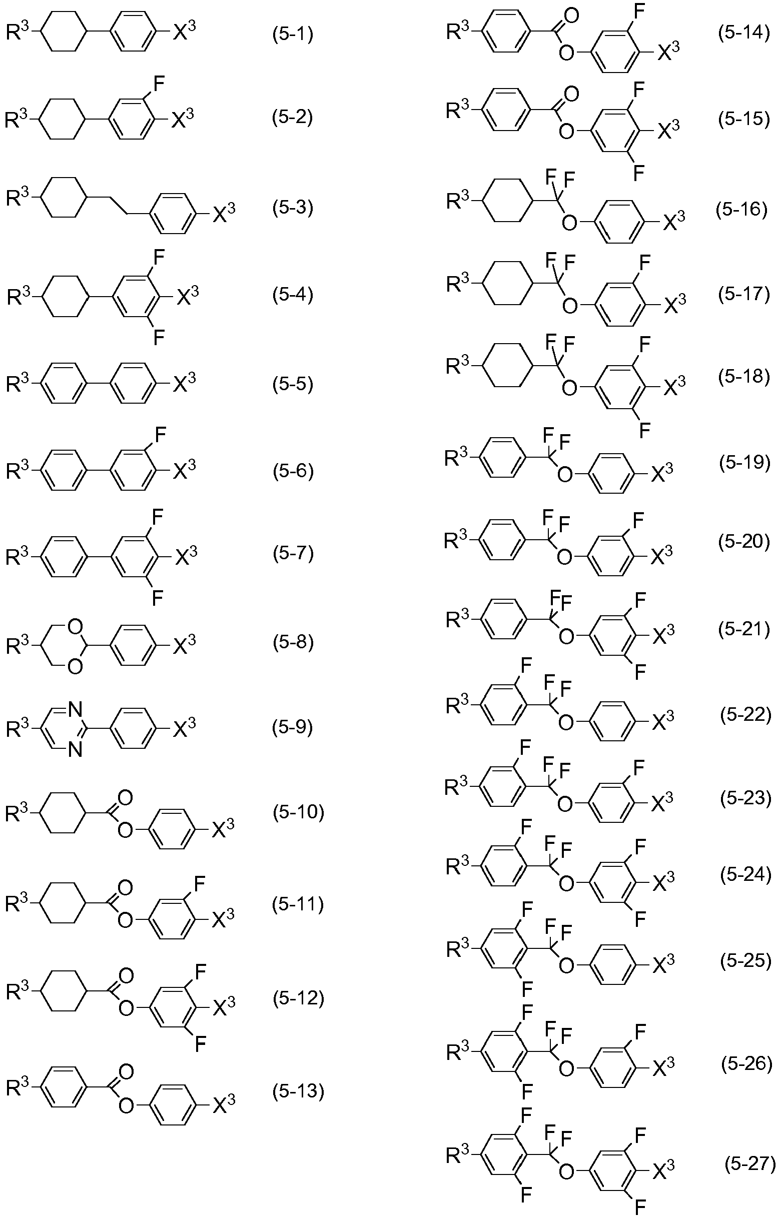

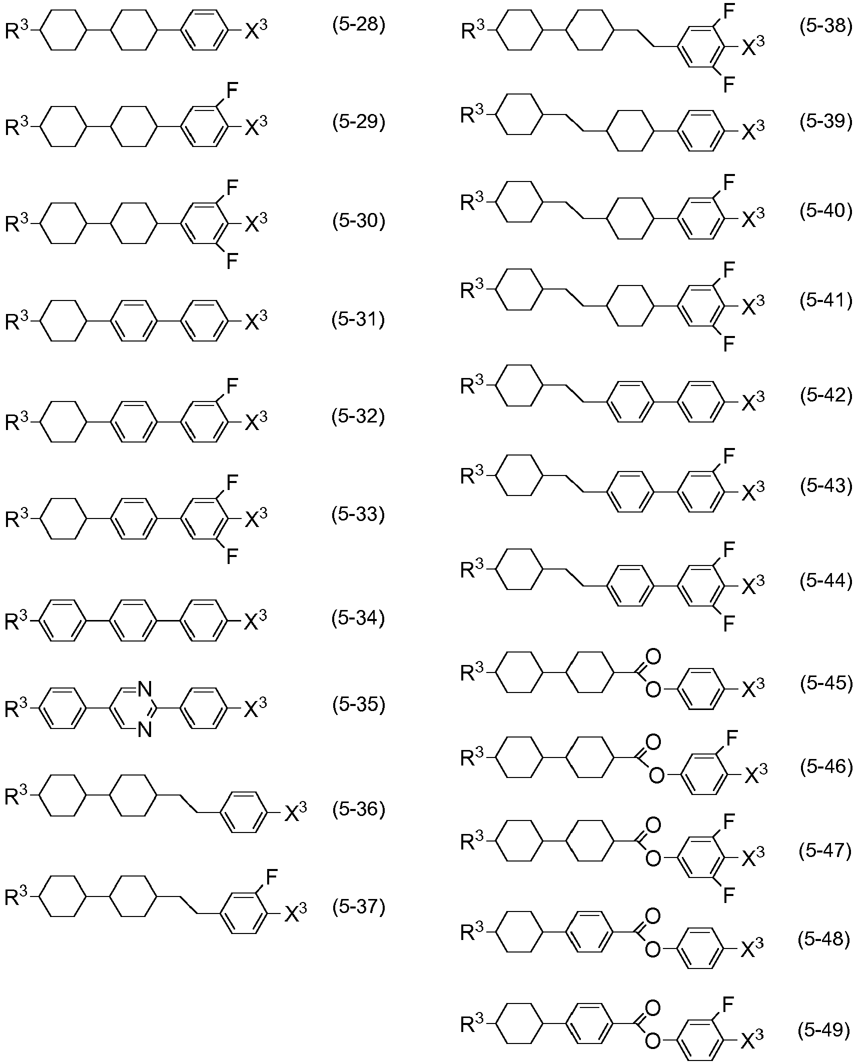

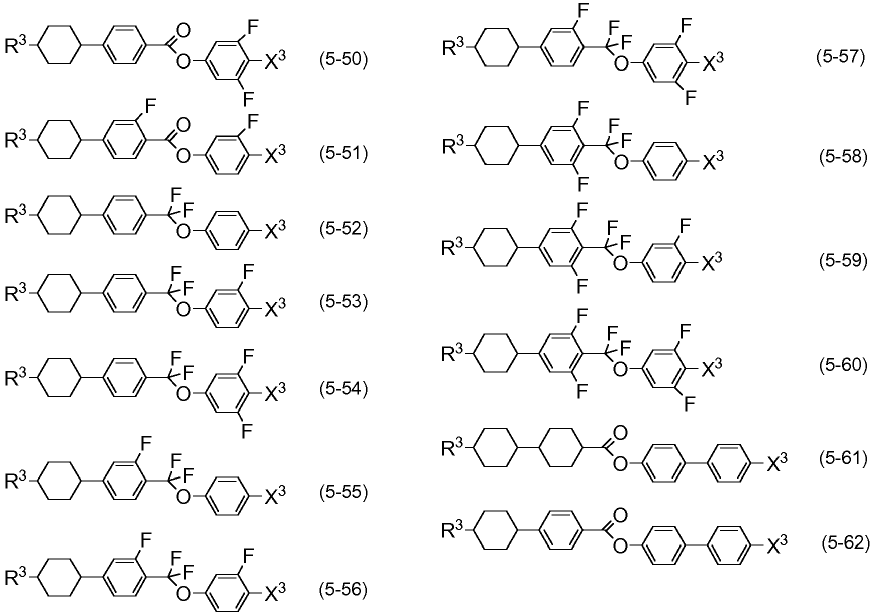

In these formulae, R³ and X³ are defined as above.

The compounds of formula (5) (component C) has large and positive dielectric anisotropy. When the liquid crystal composition contains the component C, the driving voltage thereof can be lowered, the viscosity and optical anisotropy can be adjusted, and the temperature range of the liquid crystal phase can be broadened.

Relative to the total weight of the composition, the content of the component C is preferably in the range of 0.1 wt% to 99.9 wt%, more preferably in the range of 10 wt% to 97 wt% and even more preferably in the range of 40 wt% to 95 wt%. Further, the threshold voltage, temperature range of the liquid crystal phase, optical anisotropy, dielectric anisotropy, viscosity and so on can be adjusted by mixing the components described below.

Suitable examples of the compounds of formula (6) (component D) are expressed by formulae (6-1) to (6-6). 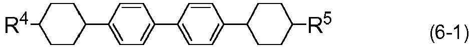 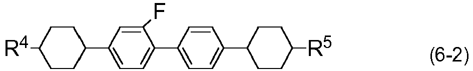 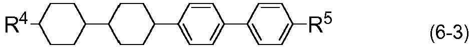 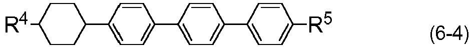 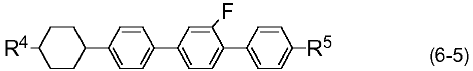 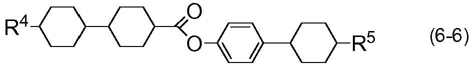 In these formulae, R⁴ and R⁵ are defined as above.

The compound of formula (6) (the component D) has a small absolute value of dielectric anisotropy, and is a nearly neutral compound. The compound of formula (6) has the effect of broadening the temperature range of optically isotropic liquid crystal phase possibly by raising the clearing point, or the effect of adjusting optical anisotropy.

Because the driving voltage of the liquid crystal composition is raised and the viscosity is lowered when the content of the compound of the component D is increased, the content is desirably higher if only the required value of the driving voltage of the liquid crystal composition can be achieved. When a liquid crystal composition for use in TFT is prepared, the content of the component D is preferably not more than 60 wt%, more preferably not more than 40 wt%, relative to the total weight of the composition.

The liquid crystal composition of this invention preferably contains 0.1 wt% to 99 wt% of at least one compound of formula (1) of the invention to exhibit good properties.

The liquid crystal composition of this invention generally can be prepared through a well-know process, for example, a process in which the required components are dissolved at a high temperature.

### 3. Compounds (7) to (11)

A third aspect of this invention is a liquid crystal composition obtained by adding a component selected from the following components E and F to the component A.

A preferred component added to the component A is a mixture obtained by mixing the component E or F, wherein the component E includes at least one compound selected from the group consisting of the compounds of the above formulae (7), (8), (9) and (10) above, and the component F includes at least one compound selected from the group consisting of the compounds of the above formula (11).

Moreover, for each component of the liquid crystal composition used in this invention, even an analogue containing isotopes of the element has little difference in physical properties.

In the scope of the component E, suitable examples of the compounds of formula (7) are expressed by formulae (7-1) to (7-8), suitable examples of the compounds of formula (8) are expressed by formulae (8-1) to (8-26), suitable examples of the compounds of formula (9) are expressed by formulae (9-1) to (9-22), and suitable examples of the compounds of formula (10) are expressed by formulae (10-1) to (10-5).

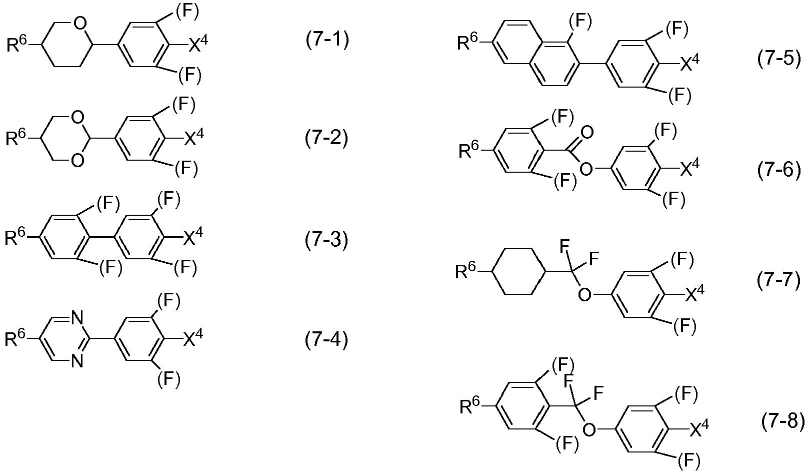

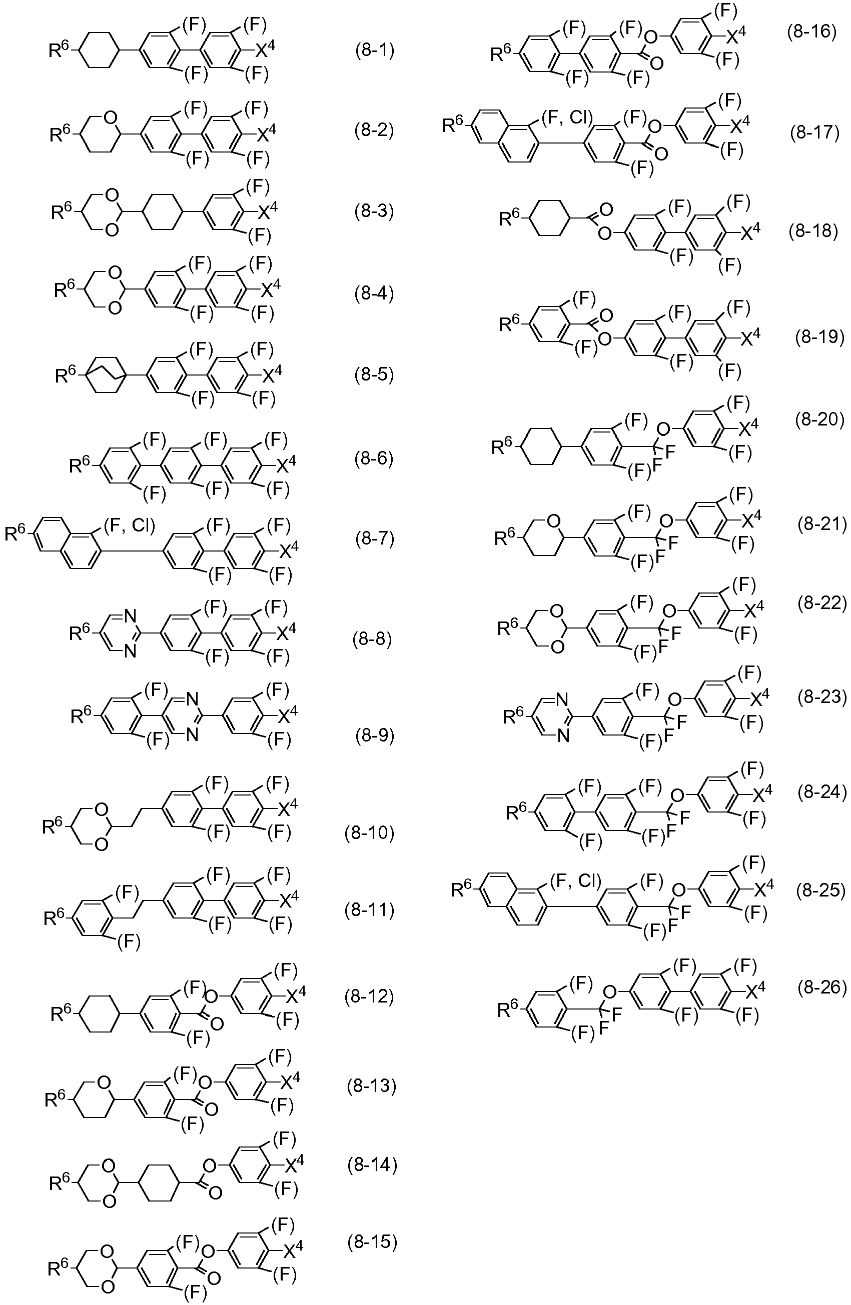

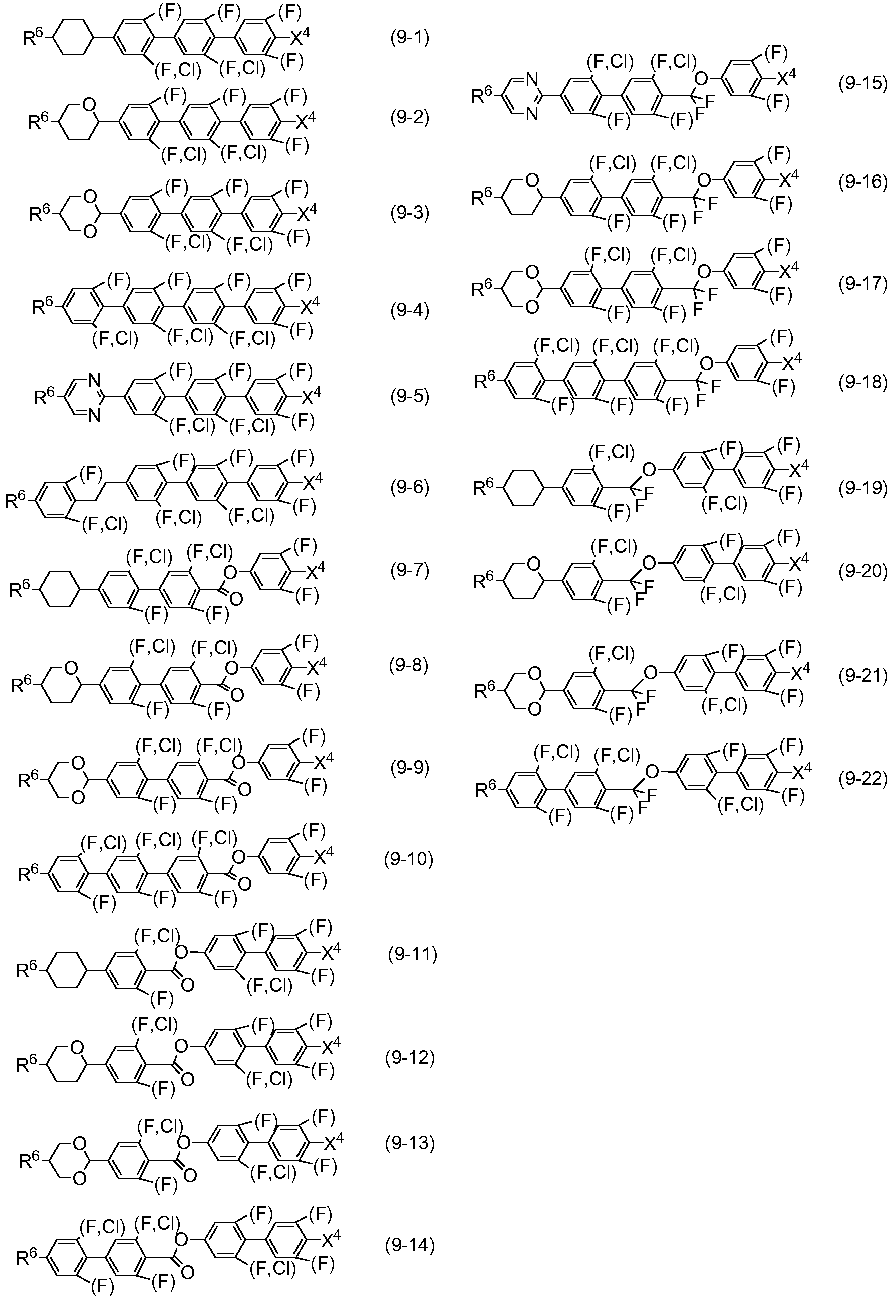

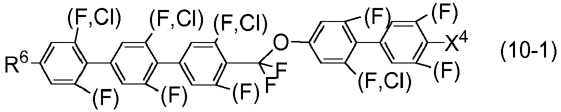

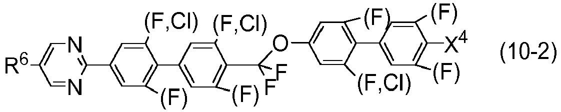

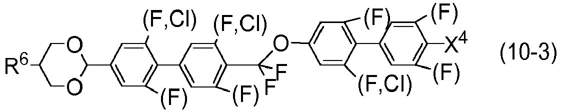

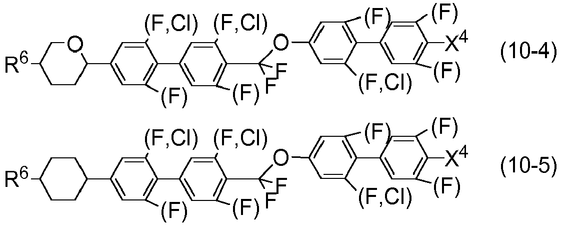

In the formulae, R⁶ and X⁴ are defined as above, (F) represents hydrogen or fluorine, and (F, Cl) represents hydrogen, fluorine or chlorine.

The compounds of formulae (7)-(10), namely the component E, have very large positive dielectric anisotropy and very high thermal and chemical stability, and are thus suitable for preparing a liquid crystal composition used in active driving mode like TFT driving. In the liquid crystal composition of this invention, the content of the component E is suitably in the range of 1 wt% to 99 wt%, preferably from 10 wt% to 97 wt% and more preferably from 40 wt% to 95 wt%, relative to the total weight of the liquid crystal composition. Further, if the liquid crystal composition further contains the compound of formula (6) (component D), the clearing point and the viscosity can be adjusted.

Suitable examples of the compounds of the above formula (11) (component F) are expressed by formulae (11-1) to (11-37).

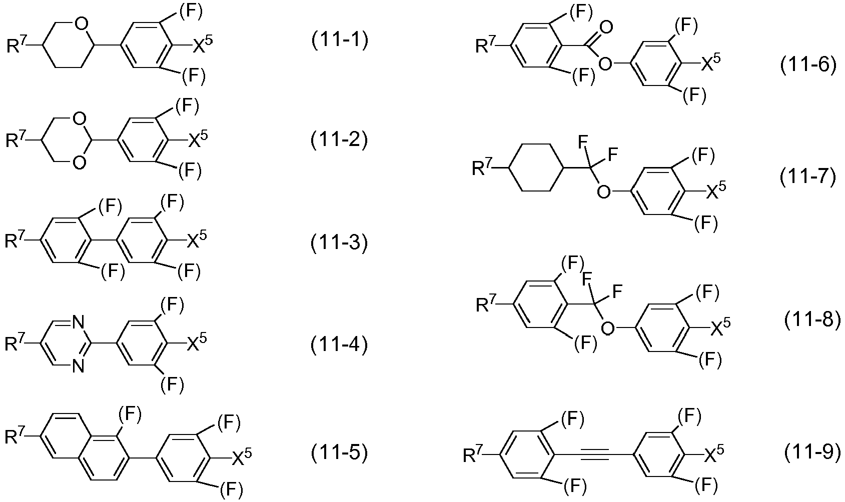

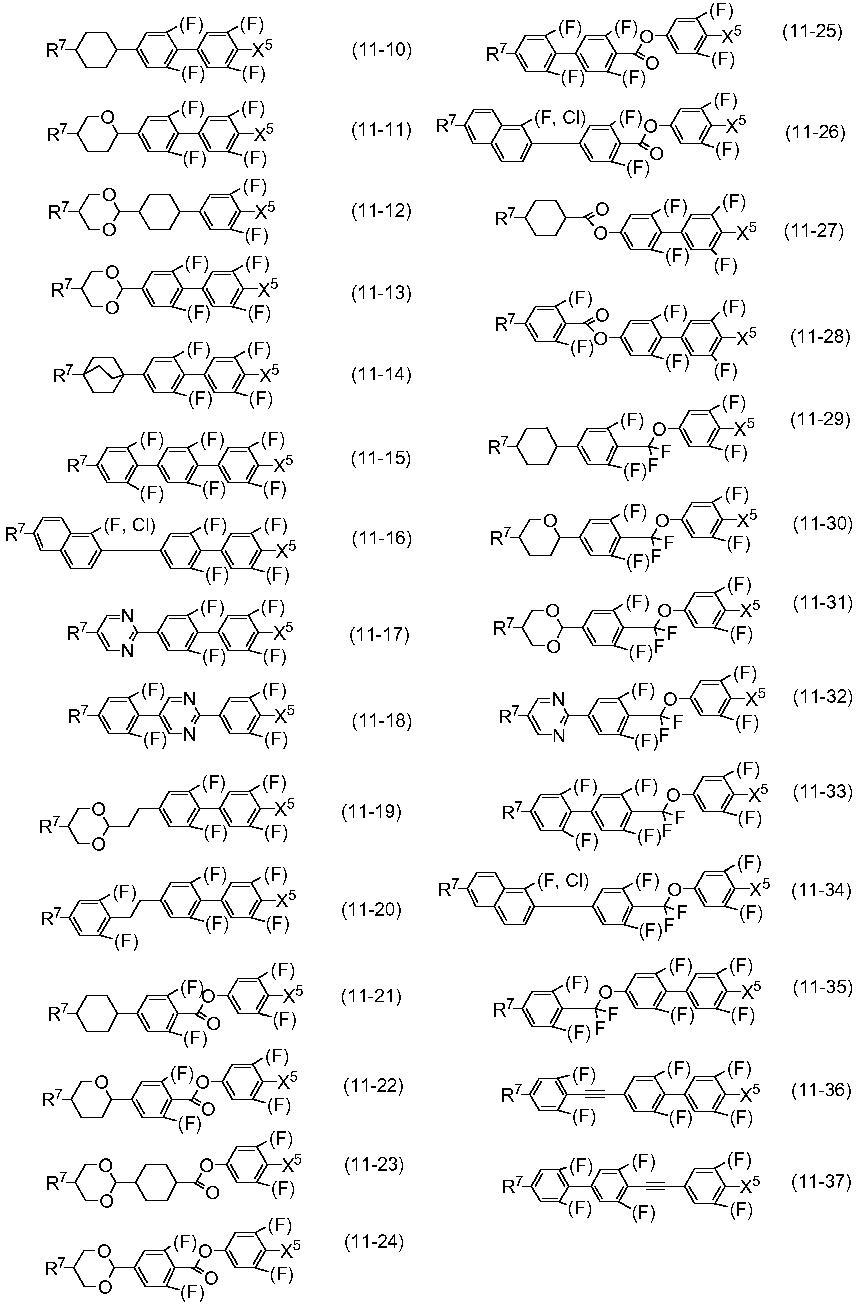

In these formulae, R⁷, X⁵, (F) and (F, Cl) are defined as above.

These compounds of formula (11), namely the component F, have very large positive dielectric anisotropy, and are thus mainly used to lower the driving voltage of the following elements: elements driven in an optically isotropic liquid crystal phase, polymer dispersed LCD (PDLCD), polymer network LCD (PNLCD), polymer stabilized cholesteric LCD (PSCLCD) and so on. If the liquid crystal composition contains the component F, the driving voltage of the composition can be lowered, the viscosity and the optical anisotropy can be adjusted, and the temperature range of the liquid crystal phase can be broadened. Moreover, the component F can also be used to improve the steepness of the voltage-transmittance curve.

The content of the component F is preferably in the range of 0.1 wt% to 99.9 wt%, more preferably in the range of 10 wt% to 97 wt%, and even more preferably in the range of 40 wt% to 95 wt%, relative to the total weight of the composition.

### 4. Composition Having Optically Isotropic Liquid Crystal Phase

### 4.1 Components of Composition Having Optically Isotropic Liquid Crystal Phase

The 4^{th} aspect of the invention is a liquid crystal composition that includes the compound of formula (1) and a chiral dopant and can be used in an optical element driven in an optically isotropic liquid crystal phase. The liquid crystal composition is a composition exhibiting an optically isotropic liquid crystal phase.

The compound of formula (1) has a low clearing point, a large dielectric anisotropy and a large optical anisotropy, and hence has an amount of suitably 5 wt% to 100 wt%, preferably 5 wt% to 80 wt% and more preferably 10 wt% to 70 wt%, relative to the total weight of the achiral liquid crystal composition without a chiral dopant added.

The amount of the chiral dopant relative to the total weight of the liquid crystal composition is preferably from 1 wt% to 40 wt%, more preferably from 3 wt% to 25 wt% and even more preferably from 5 wt% to 15 wt%. A liquid crystal composition containing the chiral dopant in these ranges easily exhibits an optically isotropic liquid crystal phase, and is therefore preferred.

The chiral dopant contained in the liquid crystal composition may include a single compound, or include two or more compounds.

### 4.2 Chiral Dopant

The chiral dopant contained in the optically isotropic liquid crystal composition is an optically active compound, and is preferably a compound with a large helical twisting power. With a compound having a large helical twisting power, the addition amount required for obtaining a desired pitch can be reduced, so the driving voltage is prevented from being raised, which is advantageous in practice. Specifically, the compounds of formulae (K1) to (K5) are preferred. 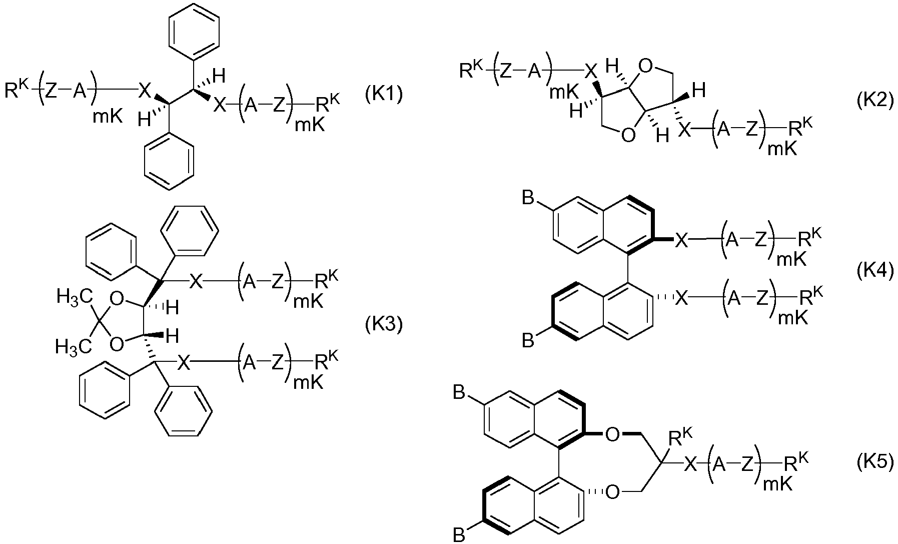 In formulae (K1)-(K5), each R^{K} is independently hydrogen, halogen, -C≡N, -N=C=O, -N-C=S, or C₁₋₂₀ alkyl in which arbitrary -CH₂- is optionally replaced by -O-, -S-, -COO- or -OCO-, arbitrary -CH₂-CH₂- is optionally replaced by -CH=CH-, -CF=CF- or -C≡C-, and arbitrary hydrogen is optionally replaced by halogen. Each A is independently an aromatic or non-aromatic, three- to eight-membered ring, or a fused ring of 9 or more carbons, wherein in these rings, arbitrary hydrogen is optionally replaced by halogen, C₁₋₃ alkyl or C₁₋₃ haloalkyl, -CH₂- optionally replaced by -O-, -S- or -NH-, and -CH= optionally replaced by -N=. Each B is independently hydrogen, halogen, C₁₋₃ alkyl, C₁₋₃ haloalkyl, an aromatic or non-aromatic, three- to eight-membered ring, or a fused ring of 9 or more carbons, wherein in these rings, arbitrary hydrogen is optionally replaced by halogen, C₁₋₃ alkyl or C₁₋₃ haloalkyl, -CH₂- optionally replaced by -O-, -S- or -NH-, and -CH= optionally replaced by -N=. Each Z is independently a single bond, or C₁₋₈ alkylene in which arbitrary -CH₂- is optionally replaced by -O-, -S-, -COO-, -OCO-, -CSO-, -OCS-, -N=N-, -CH=N- or -N=CH-, arbitrary -CH₂-CH₂- is optionally replaced by -CH=CH-, -CF=CF- or -C≡C-, and arbitrary hydrogen is optionally replaced by halogen. X is a single bond, -COO-, -OCO-, -CH₂O-, -OCH₂-, -CF₂O-, -OCF₂- or -CH₂CH₂-, and mK is an integer of 1 to 4.

Among the compounds of formulae (K1) to (K5), as a chiral dopant added in the liquid crystal composition, the compounds of formulae (K2-1) to (K2-8) in the scope of formula (K2), the compounds of formulae (K4-1) to (K4-6) in the scope of formula (K4) and the compounds of formulae (K5-1) to (K5-3) in the scope of formula (K5) are preferred. 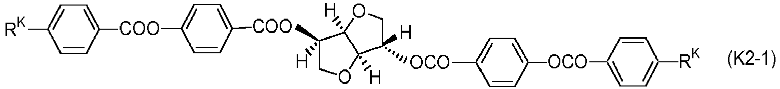 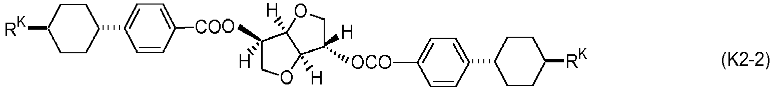 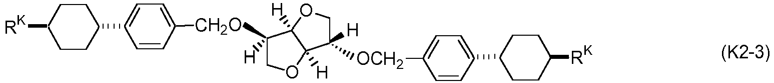 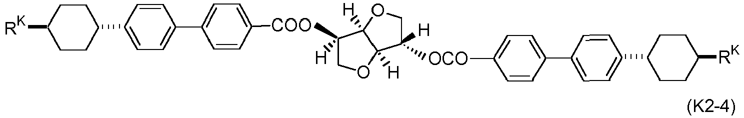 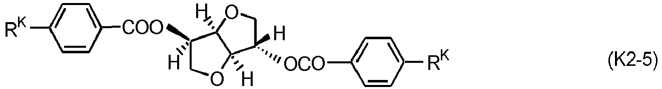 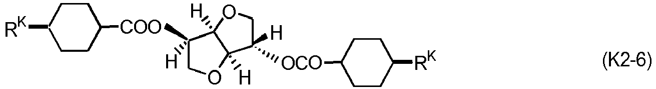 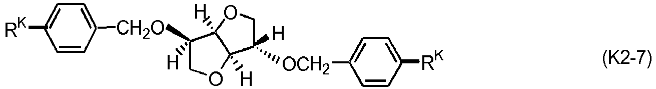 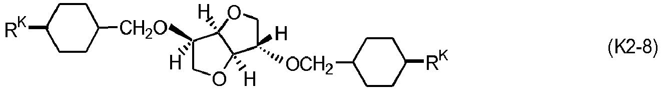 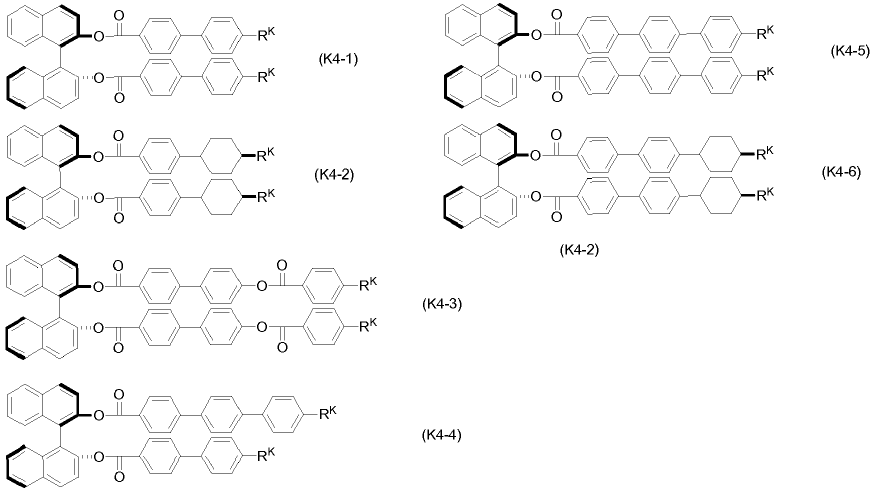 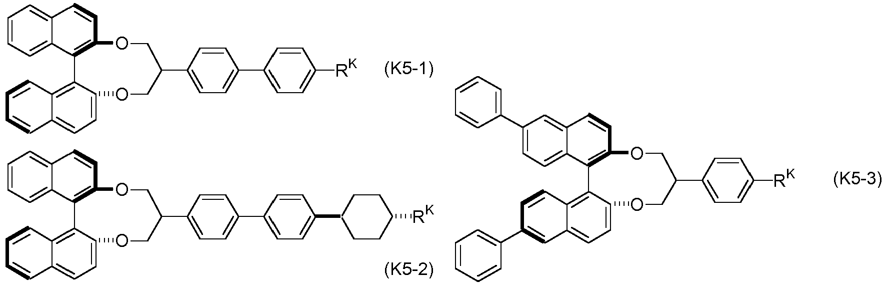 In these formulae, each R^{K} is independently C₃₋₁₀ alkyl in which the -CH₂- directly bonded to a ring is optionally replaced by -O- and arbitrary -CH₂-CH₂- is optionally replaced by -CH=CH-.

### 4.3 Optically Isotropic Liquid Crystal Phase

That a liquid crystal composition has optical isotropy means that the composition exhibits optical isotropy because of macroscopically isotropic arrangement of the liquid crystal molecules but has microscopic liquid crystal order. The pitch corresponding to the microscopic liquid crystal order of the liquid crystal composition (often referred to as "pitch", hereinafter) is preferably 700 nm or less, more preferably 500 nm or less, and even more preferably 350 nm or less.

Here, the so-called "non-liquid crystal isotropic phase" refers to a commonly defined isotropic phase, i.e., a disorder phase, or a phase that still exhibits isotropy due to fluctuation even when a region with a non-zero local order parameter is generated. For example, an isotropic phase formed at the high temperature side of a nematic phase is equivalent to the non-liquid crystal isotropic phase in this specification. The chiral liquid crystal in this specification also has a similar definition. Moreover, the term "optically isotropic liquid crystal phase" in this specification means a liquid crystal phase that exhibits optical isotropy without fluctuation, an example of which is a phase exhibiting a platelet structure, i.e., a blue phase in narrow sense.

The optically isotropic liquid crystal composition of the invention has an optically isotropic liquid crystal phase. However, the typical platelet structure in a blue phase is not observed under a polarizing microscope. Therefore, in this specification, a phase exhibiting the platelet structure is designated as a blue phase, and an optically isotropic liquid crystal phase including a blue phase is designated as an optically isotropic liquid crystal phase. That is, the blue phase is included in the optically isotropic liquid crystal phase.

Generally, the blue phases can be divided into three types, blue phase I, blue phase II and blue phase III, which are all optically active and isotropic. In a blue phase I or II, two or more colors of diffracted light produced by Bragg reflection from different lattice planes are observed. The blue phase is generally observed between the non-liquid crystal isotropic phase and the chiral nematic phase.

That the optically isotropic liquid crystal phase does not exhibit two or more colors of diffracted light means that a platelet structure observed in a blue phase I or II is not observed and the phase substantially exhibits a single color in the entire plane. For an optically isotropic liquid crystal phase not exhibiting two or more colors of diffracted light, brightness/darkness of the color is not necessarily even in plane.

An optically isotropic liquid crystal phase not exhibiting two or more colors of diffracted light has a merit of inhibiting the intensity of the reflected light caused by Bragg reflection, or shifting toward the short wavelength side.

Further, when a liquid crystal material reflecting visible light is used in a display element, sometimes a color variation problem may occur. However, for a liquid crystal not exhibiting two or more colors of diffracted light, the reflection of visible light may be eliminated due to the pitch larger than that in a blue phase in narrow sense (a phase exhibiting the platelet structure), as a result of reflection wavelength shift toward the short wavelength side.

The optically isotropic liquid crystal composition of the invention may be obtained by adding a chiral dopant to a composition having a nematic phase, wherein the chiral dopant is preferably added in a concentration such that the pitch is 700 nm or less. Moreover, the composition having a nematic phase contains a compound of formula (1) and other required components. Furthermore, the optically isotropic liquid crystal composition of the invention may alternatively be obtained by adding a chiral dopant to a composition having a chiral nematic phase but no optically isotropic liquid crystal phase. The composition having a chiral nematic phase but no optically isotropic liquid phase contains a compound of formula (1), an optically active compound, and other required components, wherein the optically active compound is preferably added in a concentration such that the pitch is 700 nm or more to exhibit an optically isotropic liquid crystal phase. The optically active compounds being added may be the above compounds with a large helical twisting power, that is, the compounds of formulae (K1) to (K5), formulae (K2-1) to (K2-8), formulae (K4-1) to (K4-6) and formulae (K5-1) to (K5-3). Moreover, the optically active compound being added may not have a large helical twisting power. Such an optically active compound is, for example, one added in a liquid crystal composition for use of an element driven in a nematic phase (in TN mode or STN mode, etc.).

Examples of the compound not having a so large helical twisting power are the following optically active compound (Op-1) to (Op-13).

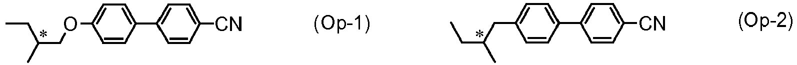

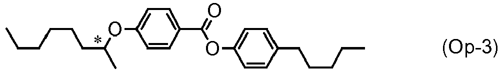

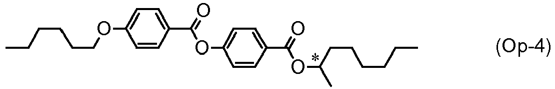

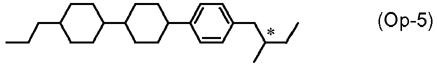

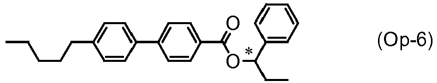

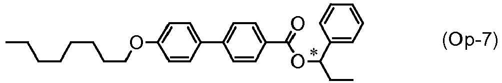

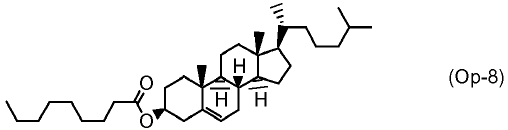

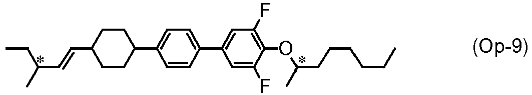

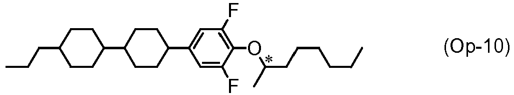

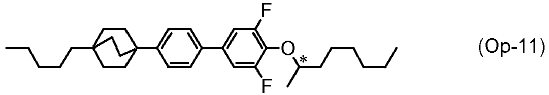

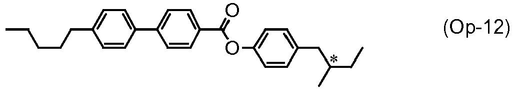

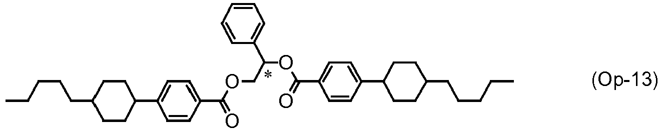

Moreover, the temperature range of the optically isotropic liquid crystal composition of the invention can be broadened by adding a chiral dopant to a liquid crystal composition having a broad temperature range for the co-existence of a nematic phase or a chiral nematic phase and an isotropic phase to exhibit an optically isotropic liquid crystal phase. For example, a composition exhibiting an optically isotropic liquid crystal phase in a broad temperature range can be prepared as follows. A liquid crystal compound having a high clearing point is mixed with a liquid crystal compound having a low clearing point to prepare a liquid crystal composition with a broad temperature range for the co-existence of a nematic phase and an isotropic phase. Then, a chiral dopant is added to the liquid crystal composition.

For a liquid crystal composition with a broad co-existence temperature range of a nematic or chiral nematic phase and a non-liquid crystal isotropic phase, the difference between the upper-limit temperature and the lower-limit temperature of the co-existence is preferably from 3°C to 150°C, and more preferably from 5°C to 150°C. Moreover, the liquid crystal composition preferably has a difference of 3°C to 150°C between the upper-limit temperature and the lower-limit temperature of the co-existence of the nematic phase and the non-liquid crystal isotropic phase.

When an electric field is applied to the liquid crystal medium of the invention in an optically isotropic liquid crystal phase, an electric-birefringence occurs but the Kerr effect does not necessarily occur.

Because the electric-birefringence of an optically isotropic liquid crystal phase increases with an increase in the pitch, the electric-birefringence can be increased by adjusting the species and content of the chiral dopant to increase the pitch, as long as other optical properties, such as the transmittance and the diffraction wavelength etc., could be satisfied.

### 4-4. Other Components

Other compounds, such as a polymer material, may be further added into the optically isotropic liquid crystal composition of the invention, so long as they do not affect the properties of the composition. In addition to the polymer material, the liquid crystal composition of the invention may also contain, for example, a dichroic dye or a photochromic compound. Examples of the dichroic dye include merocyanine dyes, styryl dyes, azo dyes, azomethine dyes, azoxy dyes, quinophthalone dyes, anthraquinone dyes, tetrazine dyes and so on.

### 5. Optically Isotropic Polymer/Liquid Crystal Composite Material

The 5^{th} aspect of the invention is a composite material of a polymer and a liquid crystal composition containing the compound of formula (1) and a chiral dopant, which exhibits optical isotropy. The polymer/liquid crystal composite material can be used in an optical element driven in an optically isotropic liquid crystal phase, which may include the liquid crystal composition (CLC) according to any one of claims 5 to 11, and a polymer.

The term "polymer/liquid crystal composite material" of the invention has no particular limitation, as long as it is a composite containing both a liquid crystal material and a polymeric compound, wherein the polymer may be partially or entirely not dissolved in the liquid crystal material so that the polymer is separated from the liquid crystal material. Further, in this specification, a nematic phase refers to one in narrow sense but does not include a chiral nematic phase, unless specifically indicated.

The optically isotropic polymer/liquid crystal composite material according to a preferred aspect of the invention can exhibit an optically isotropic liquid crystal phase in a broad temperature range. Moreover, the polymer/liquid crystal composite material according to a preferred aspect of the invention has very high response speed. Based on such effects, the polymer/liquid crystal composite material according to a preferred aspect of the invention is useful in an optical element such as a display element.

### 5-2. Polymer

Though the composite material of the invention can be produced by mixing an optically isotropic liquid crystal composition with a pre-polymerized polymer, it is preferably produced by mixing a low molecular weight monomer, macromonomer or oligomer, etc. (generally referred to as "monomer", hereinafter) as a raw material of the polymer with the liquid crystal composition CLC and then polymerizing the mixture. In this specification, the mixture containing a monomer and a liquid crystal composition is referred to as "polymerizable monomer/liquid crystal mixture", which may optionally contain a polymerization initiator, a curing agent, a catalyst, a stabilizer, a dichroic dye or a photochromic compound, etc., without compromising the effects of the invention. For example, if required, the polymerizable monomer/liquid crystal mixture of the invention may contain 0.1 to 20 parts by weight of a polymerization initiator, relative to 100 parts by weight of the polymerizable monomer.

The polymerization temperature is preferably such that the polymer/liquid crystal composite material exhibits high transparency and isotropy, and more preferably such that the mixture of the monomer and the liquid crystal material exhibits an isotropic phase or a blue phase, while the polymerization is carried out in the isotropic phase or optically isotropic liquid crystal phase. That is, the polymerization temperature is preferably set such that after the polymerization, the polymer/liquid crystal composite material substantially does not scatter light of wavelength greater than that of visible light and exhibit optical isotropy.

For example, a low molecular weight monomer, macromonomer or oligomer can be used as a raw material of the polymer constituting the composite material of the invention. In this specification, the raw-material monomer of the polymer covers low molecular weight monomers, macromonomers and oligomers, etc. Furthermore, the obtained polymer preferably has a three-dimensional cross-linked structure, and hence the raw-material monomer of the polymer preferably includes a multi-functional monomer having two or more polymerizable functional groups. The polymerizable functional groups have no particular limitation; examples thereof include acryloyl, methacryloyl, glycidyl, epoxy, oxetanyl, vinyl and so on. In view of the polymerization rate, acryloyl and methacryloyl are preferred. It is preferred that the raw material monomers of the polymer contain 10 wt% or more of a monomer having two or more polymerizable functional groups, since the obtained composite material of the invention can easily exhibit high transparency and isotropy.

Moreover, in order to obtain a suitable composite material, the polymer preferably has mesogen moieties, and a part or all of the raw material monomers of the polymer used can have a mesogen moiety.

### 5-2-1. Mono- and Di-functional Monomers Having A Mesogen Moiety

The mono- and di-functional monomers having a mesogen moiety has no particular limitation in structure, and can be, for example, the compounds of formula (M1) or (M2) below.

R^{a}-Y-(A^{M}-Z^{M})ₘ₁-A^{M}-Y-R^{b} (M1)

R^{b}-Y-(A^{M}-Z^{M})ₘ₁-A^{M}-Y-R^{b} (M2)

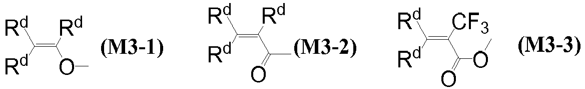

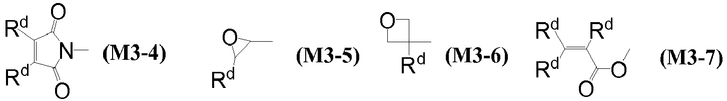

In formula (M1), each R^{a} is independently hydrogen, halogen, -C≡N, -N=C=O, -N=C=S, or C₁₋₂₀ alkyl in which arbitrary -CH₂- is optionally replaced by -O-, -S-, -CO-, -COO- or -OCO-, arbitrary -CH₂-CH₂- is optionally replaced by -CH=CH-, -CF=CF- or -C≡C-, and arbitrary hydrogen is optionally replaced by halogen or -C≡N. Each R^{b} is independently a polymerizable functional group of one of formulae (M3-1) to (M3-7).

R^{a} is preferably hydrogen, halogen, -C≡N, -CF₃, -CF₂H, -CFH₂, -OCF₃, -OCF₂H, C₁₋₂₀ alkyl, C₁₋₁₉ alkoxy, C₂₋₂₁ alkenyl or C₂₋₂₁ alkynyl, and is particularly preferably -C≡N, C₁₋₂₀ alkyl or C₁₋₁₉ alkoxy.

In formula (M2), each R^{b} is independently a polymerizable functional group of one of formulae (M3-1) to (M3-7).

In formulae (M3-1) to (M3-7), each R^{d} is independently hydrogen, halogen, or C₁₋₅ alkyl in which arbitrary hydrogen is optionally replaced by halogen. R^{d} is preferably hydrogen, halogen or methyl, and is particularly preferably hydrogen, fluorine or methyl.

Moreover, the compounds of formulae (M3-2), (M3-3), (M3-4) and (M3-7) are preferably polymerized with free radicals. The compounds of formulae (M3-1), (M3-5) and (M3-6) are preferably polymerized with cations. The above polymerizations are all active polymerization, and are initiated as a small amount of free radical or cationic active species is generated in the reaction system. To accelerate generation of the active species, a polymerization initiator can be used. The active species can be generated by light or heat.

In formulae (M1) and (M2), each A^{M} is independently an aromatic or non-aromatic five-membered ring, six-membered ring or fused ring of 9 or more carbons, in which -CH₂- is optionally replaced by -O-, -S-, -NH- or -NCH₃-, -CH= is optionally replaced by -N=, and hydrogen is optionally replaced by halogen, C₁₋₅ alkyl or C₁₋₅ haloalkyl. Specific examples of preferred A^{M} are 1,4-cyclohexylene, 1,4-cyclohexenylene, 1,4-phenylene, naphthalene-2,6-diyl, tetrahydronaphthalene-2,6-diyl, fluorene-2,7-diyl and bicyclo[2.2.2]octan-1,4-diyl. In these rings, arbitrary -CH₂-is optionally replaced by -O-, arbitrary -CH= is optionally replaced by -N=, and arbitrary hydrogen is optionally replaced by halogen, C₁₋₅ alkyl or C₁₋₅ haloalkyl.

In consideration of the stability of the compound, -CH₂-O-CH₂-O- with two oxygen atoms directly bonded to one another is preferred to -CH₂-O-O-CH₂- with two oxygen atoms directly bonded to one another. This also applies to the case of sulfur.

Among these rings, 1,4-cyclohexylene, 1,4-cyclohexenylene, 1,4-phenylene, 2-fluoro-1,4-phenylene, 2,3-difluoro-1,4-phenylene, 2,5-difluoro-1,4-phenylene, 2,6-difluoro-1,4-phenylene, 2-methyl-1,4-phenylene, 2-trifluoromethyl-1,4-phenylene, 2,3-bis(trifluoromethyl)-1,4-phenylene, naphthalene-2,6-diyl, tetrahydronaphthalene-2,6-diyl, fluorene-2,7-diyl, 9-methylfluorene-2,7-diyl, 1,3-dioxane-2,5-diyl, pyridine-2,5-diyl and pyrimidine-2,5-diyl are particularly preferred for A^{M}. Further, the stereo configuration of 1,4-cyclohexylene and 1,3-dioxane-2,5-diyl above is in the *trans*-form superior to in the *cis*-form.

Because 2-fluoro-1,4-phenylene and 3-fluoro-1,4-phenylene are identical in the structure, the latter is not exemplified. This also applies to the case of the relationship between 2,5-difluoro-1,4-phenylene and 3,6-difluoro-1,4-phenylene, etc.

In formulae (M1) and (M2), each Y is independently a single bond, or C₁₋₂₀ alkylene in which arbitrary -CH₂- is optionally replaced by -O- or -S- and arbitrary -CH₂-CH₂- is optionally replaced by -CH=CH-, -C≡C-, -COO- or -OCO-. Y is preferably a single bond, -(CH₂)ₘ₂-, -O(CH₂)ₘ₂- or -(CH₂)ₘ₂O-, wherein m2 is an integer of 1 to 20. Y is particularly preferably a single bond, -(CH₂)ₘ₂-, -O(CH₂)ₘ₂- or -(CH₂)ₘ₂O-, wherein m2 is an integer of 1 to 10. In consideration of the stability of the compound, -Y-R^{a} and -Y-R^{b} preferably include no -O-O-, -O-S-, -S-O- or -S-S-.

In formulae (M1) and (M2), each Z^{M} is independently a single bond, -(CH₂)ₘ₃-, -O(CH₂)ₘ₃-, -(CH₂)ₘ₃O-, -O(CH₂)ₘ₃O-, -CH=CH-, -C≡C-, -COO-, -OCO-, -(CF₂)₂-, -(CH₂)₂-COO-, -OCO-(CH₂)₂-, -CH=CH-COO-, -OCO-CH=CH-, -C≡C-COO-, -OCO-C≡C-, -CH=CH-(CH₂)₂-, -(CH₂)₂-CH=CH-, -CF=CF-, -C≡C-CH=CH-, -CH=CH-C≡C-, -OCF₂-(CH₂)₂-, -(CH₂)₂-CF₂O-, -OCF₂- or -CF₂O-, wherein m3 is an integer of 1-20.

Z^{M} is preferably a single bond, -(CH₂)ₘ₃-, -O(CH₂)ₘ₃-, -(CH₂)ₘ₃O-, -CH=CH-, -C≡C-, -COO-, -OCO-, -(CH₂)₂-COO-, -OCO-(CH₂)₂-, -CH=CH-COO-, -OCO-CH=CH-, -OCF₂- or -CF₂O-.

In formulae (M1) and (M2), m1 is an integer of 1-6, preferably an integer of 1-3. When m1 is 1, they are two-ring compounds with two rings such as six-membered rings. When m1 is 2 or 3, they are three-ring or four-ring compounds. For example, when m1 is 1, the two A^{M}'s can be identical or different. Moreover, when m1 is 2, the three A^{M}'s (or two Z^{M}'s) can be identical or different. When m1 is from 3 to 6, it is also the case. This also applies to respective cases of R^{a}, R^{b}, R^{d}, Z^{M}, A^{M} and Y.

Even when the compound (M1) of formula (M1) or the compound (M2) of formula (M2) contains an isotope, such as ²H (deuterium) and ¹³C, in an amount higher than the natural abundance, it is useful due to the identical properties thereof.

More preferred examples of the compounds (M1) and (M2) are compounds (M1-1) to (M1-41) and (M2-1) to (M2-27) of formulae (M1-1) to (M1-41) and (M2-1) to (M2-27). In these compounds, R^{a}, R^{b}, R^{d}, Z^{M}, A^{M}, Y and p are defined as in the cases of formulae (M1) and (M2) in the above aspects of the invention.

The following partial structures of the compounds (M1-1) to (M1-41) and (M2-1) to (M2-27) are described below. The partial structure (a1) represents 1,4-phenylene in which arbitrary hydrogen is replaced by fluorine. The partial structure (a2) represents 1,4-phenylene in which arbitrary hydrogen is optionally replaced by fluorine. The partial structure (a3) represents 1,4-phenylene in which arbitrary hydrogen is optionally replaced by fluorine or methyl. The partial structure (a4) represents fluorenylene in which the hydrogen at position 9 is optionally replaced by methyl.

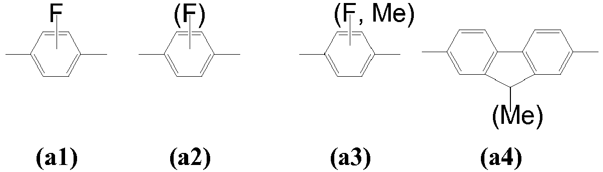

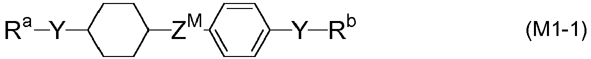

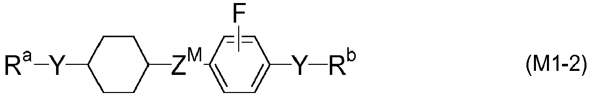

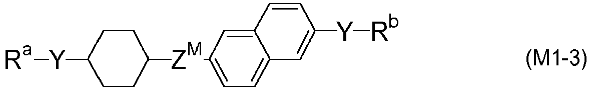

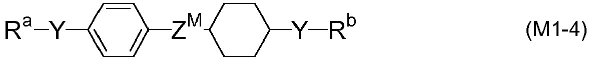

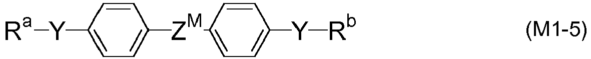

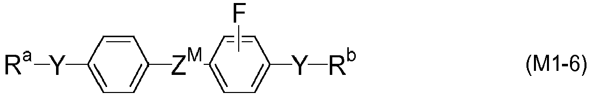

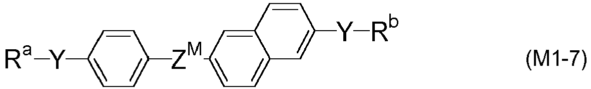

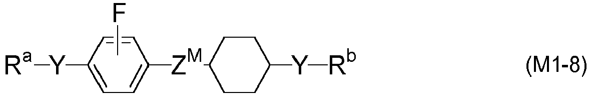

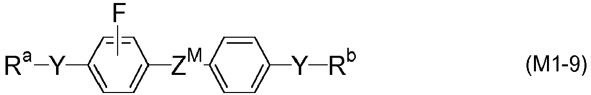

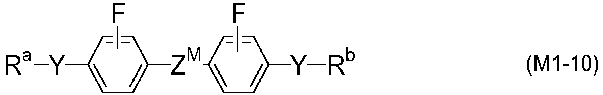

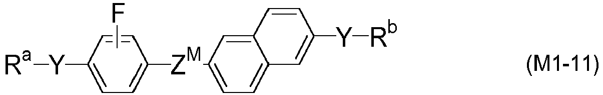

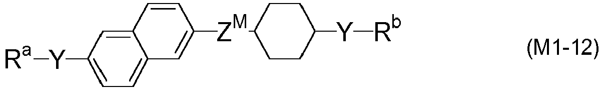

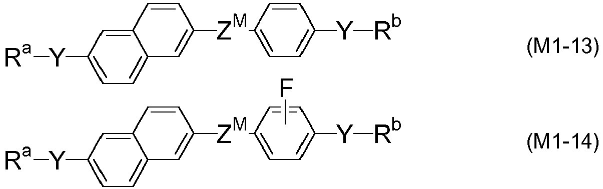

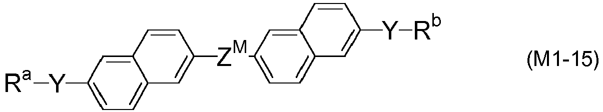

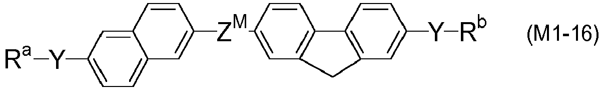

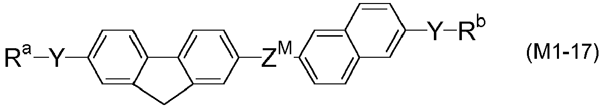

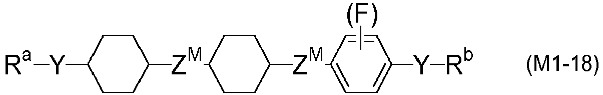

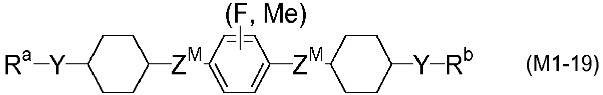

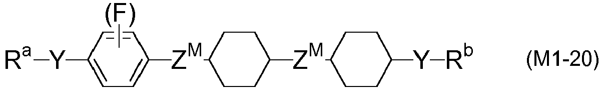

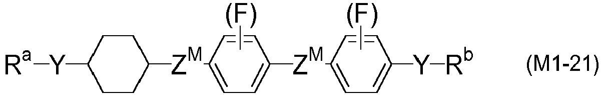

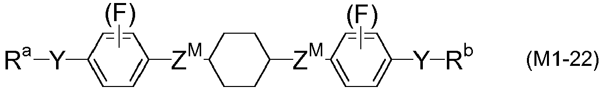

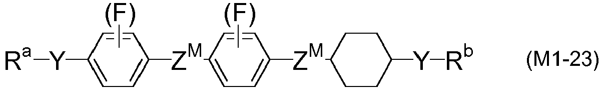

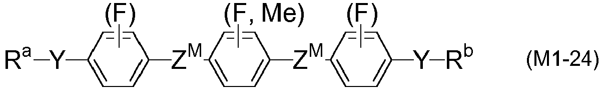

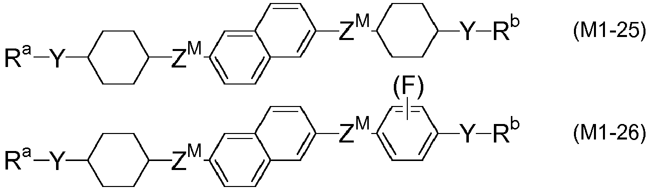

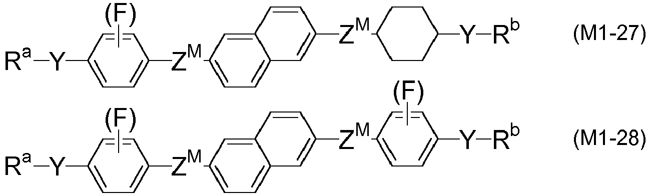

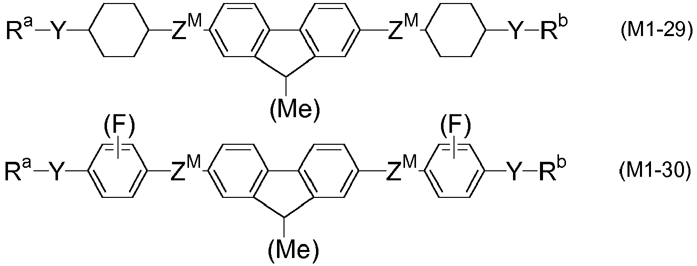

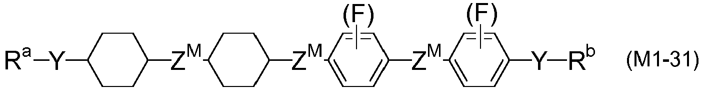

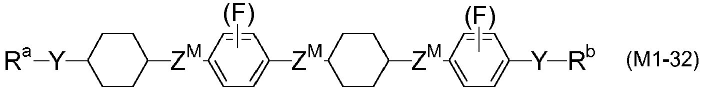

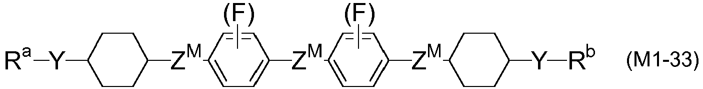

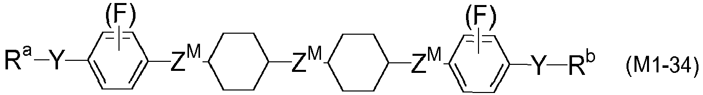

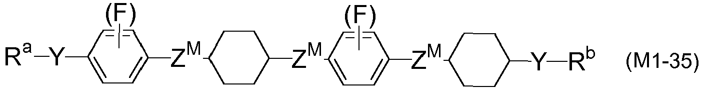

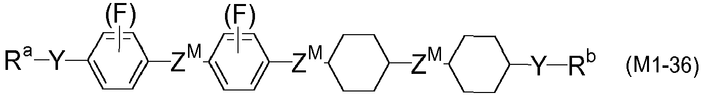

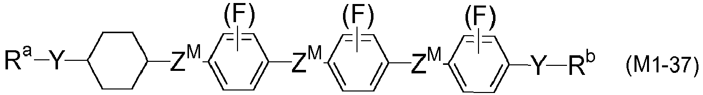

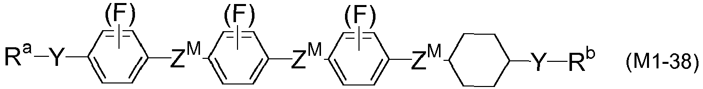

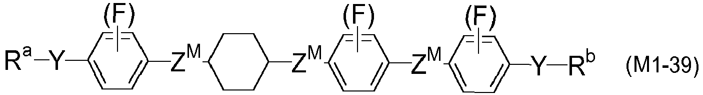

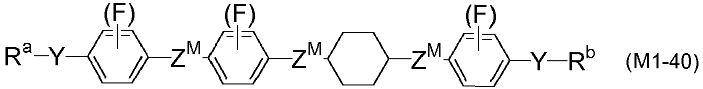

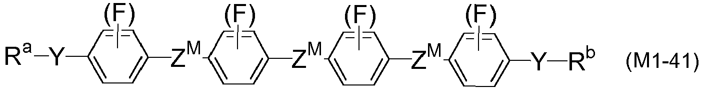

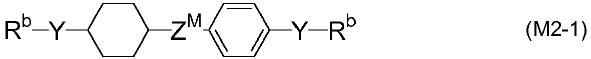

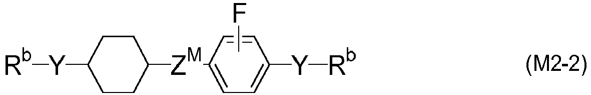

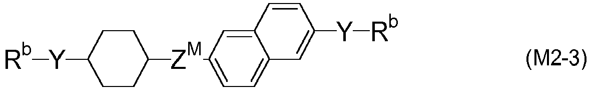

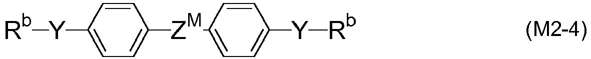

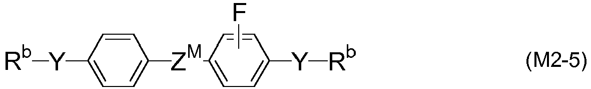

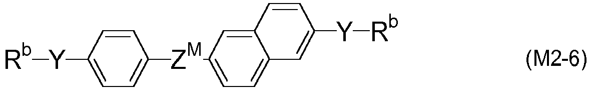

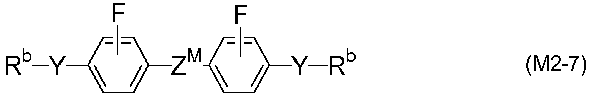

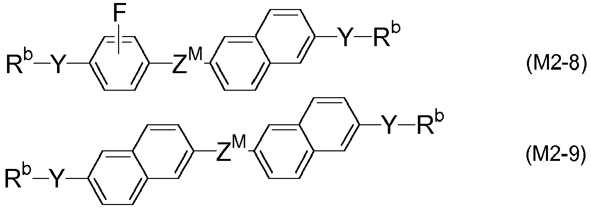

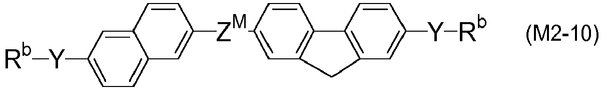

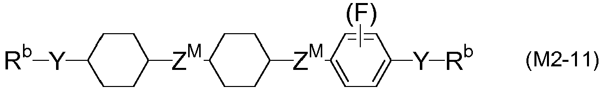

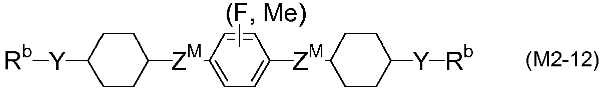

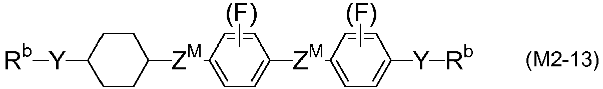

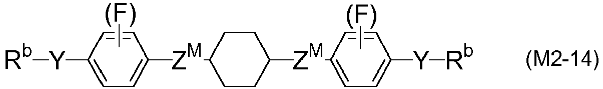

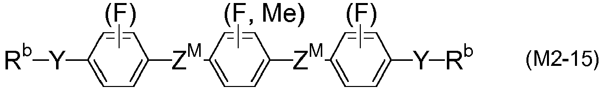

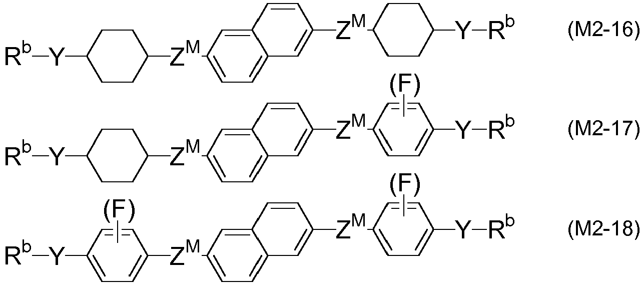

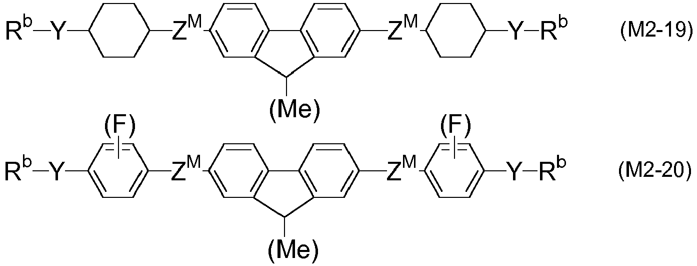

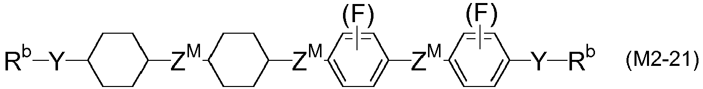

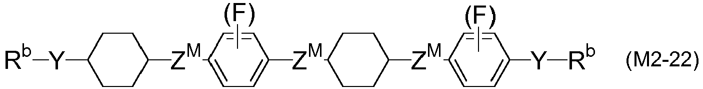

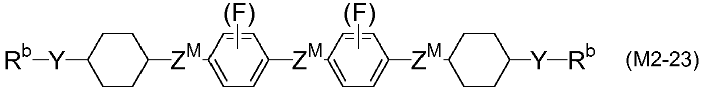

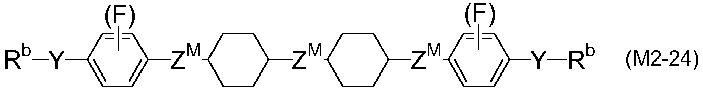

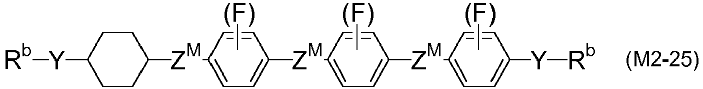

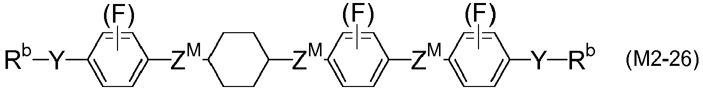

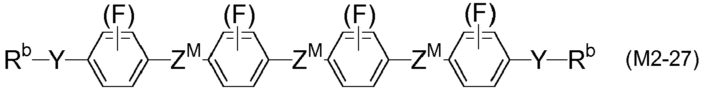

A monomer having no aforementioned mesogen moiety, and a polymerizable compound having a mesogen moiety other than the monomers (M1) and (M2) can be used, if required.

In order to optimize the optical isotropy of the polymer/liquid crystal composite material of the invention, a monomer having a mesogen moiety and three or more polymerizable functional groups can be used. Such a monomer may be a well-known compound, for example, one of formulae (M4-1) to (M4-3), and more specifically a compound described in Japanese Patent Publication Nos. 2000-327632, 2004-182949 and 2004-59772. In formulae (M4-1)-(M4-3), R^{b}, Za, Y and (F) are defined as above.

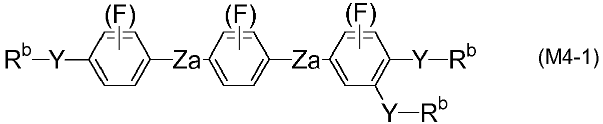

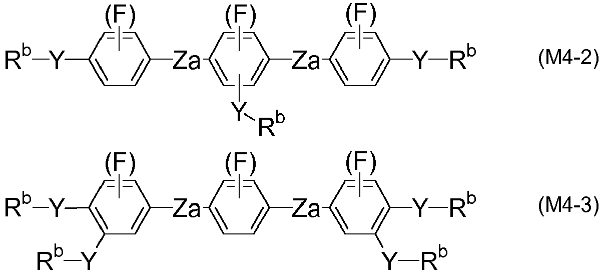

### 5-2-2. Monomer Having No Mesogen Moiety and Having Polymerizable Functional Groups

Examples of the monomer having no mesogen moiety and having polymerizable groups are straight and branched acrylates of C₁₋₃₀, straight and branched diacrylates of C₁₋₃₀, and monomers having three or more polymerizable groups. Examples of the monomers having three or more polymerizable groups are, but not limited to, glycerol·propoxylate (IPO/OH) triacrylate, pentaerythritol·propoxylate·triacrylate, pentaerythritol·triacrylate, trimethylolpropane·ethoxylate·triacrylate, trimethylolpropane·propoxylate·triacrylate, trimethylolpropane·triacrylate, di(trimethylolpropane)tetraacrylate, pentaerythritol·tetraacrylate, di(pentaerythritol)pentaacrylate, di(pentaerythritol)hexaacrylate and trimethylolpropane·triacrylate.

### 5-2-3. Polymerization Initiator

The polymerization reaction for producing the polymer constituting the composite material of the invention is not particularly limited, and may be, e.g., photo-radical polymerization, thermo-radical polymerization or photo-cationic polymerization, etc.

The polymerization initiators useful for photo-radical polymerization are, for example, DAROCUR™ 1173 and 4265 (both are trade names, from BASF Japan Ltd.) and IRGACURE™ 184, 369, 500, 651, 784, 819, 907, 1300, 1700, 1800, 1850 and 2959 (all are trade names, from BASF Japan Ltd.).

Preferred examples of the initiators causing radical polymerization with heat and being useful in thermo-radical polymerization are: benzoyl peroxide, diisopropyl peroxydicarbonate, *t*-butyl peroxy2-ethylhexanoate, *t*-butyl peroxypivalate, *t*-butyl peroxydiisobutyrate, lauroyl peroxide, dimethyl 2,2'-azobisisobutyrate (MAIB), di-*t*-butyl peroxide (DTBPO), azobisisobutyronitrile (AIBN) and azobiscyclohexanecarbonitrile (ACN), etc.

Examples of polymerization initiators useful in photo-cationic polymerization are diaryliodonium salt (referred to as "DAS", hereinafter) and triarylsulfonium salt (referred to as "TAS", hereinafter), etc.

Examples of DAS are diphenyliodonium tetrafluoroborate, diphenyliodonium hexafluorophosphonate, diphenyliodonium hexafluoroarsenate, diphenyliodonium trifluoromethanesulfonate, diphenyliodonium trifluoroacetate, diphenyliodonium *p*-toluenesulfonate, diphenyliodonium tetrakis(pentafluorophenyl)borate, 4-methoxyphenylphenyliodonium tetrafluoroborate, 4-methoxyphenylphenyliodonium hexafluorophosphonate, 4-methoxyphenylphenyliodonium hexafluoroarsenate, 4-methoxyphenylphenyliodonium trifluoromethanesulfonate, 4-methoxyphenylphenyliodonium trifluoroacetate, and 4-methoxyphenylphenyliodonium *p*-toluenesulfonate.

DAS can be sensitized by adding a photosensitizer, such as thioxanthone, phenothiazine, chlorothioxanthone, xanthone, anthracene, diphenyl anthracene or rubrene, etc.

Examples of TAS are triphenylsulfonium tetrafluoroborate, triphenylsulfonium hexafluorophosphonate, triphenylsulfonium hexafluoroarsenate, triphenylsulfonium trifluoromethanesulfonate, triphenylsulfonium trifluoroacetate, triphenylsulfonium *p*-toluenesulfonate, triphenylsulfonium tetrakis(pentafluorophenyl)borate, 4-methoxyphenyldiphenylsulfonium tetrafluoroborate, 4-methoxyphenyldiphenylsulfonium hexafluorophosphonate, 4-methoxyphenyldiphenylsulfonium hexafluoroarsenate, 4-methoxyphenyldiphenylsulfonium trifluoromethanesulfonate, 4-methoxyphenyldiphenylsulfonium trifluoroacetate, and 4-methoxyphenyldiphenylsulfonium-*p*-toluenesulfonate, etc.

Specific examples of the photo-cationic polymerization initiator are Cyracure™ UVI-6990, UVI-6974 and UVI-6992 (all are trade names, from UCC Corporation), ADEKA OPTOMER™ SP-150, SP-152, SP-170 and SP-172 (all are trade names, from ADEKA Corporation), Rhodorsil Photoinitiator™ 2074 (trade name, from Rhodia Japan Corporation), IRGACURE™ 250 (trade name, from BASF Japan Ltd.) and UV-9380C (trade name, from GE/Toshiba Silicone Co. Ltd.), etc.

### 5-2-4. Curing Agents and Others

In preparing the polymer constituting the composite material of the invention, in addition to the monomers and polymerization initiator mentioned above, other suitable component(s), for example, curing agent, catalyst and/or stabilizer may also be added.

The well-known latent curing agents commonly used for epoxy resins can be used. Examples of the latent curing agents for epoxy resins are amine curing agents, Novolac curing agents, imidazole curing agents and anhydride curing agents, etc. Examples of amine curing agents are aliphatic polyamines such as diethylenetriamine, triethylenetetraamine, tetraethylenepentaamine, m-xylenediamine, trimethyl hexanediamine, 2-methyl-pentanediamine and diethylaminopropylamine; alicyclic polyamines such as isophorone diamine, 1,3-diaminomethylcyclohexane, bis(4-aminocyclohexyl)methane, norbornenediamine, 1,2-diaminocyclohexane and Laromin; and aromatic polyamines such as diaminodiphenylmethane, diaminodiphenylethane and m-phenylenediamine, etc.

Examples of the Novolac curing agents are phenol/Novolac resin, bisphenol/ Novolac resin, etc. Examples of the imidazole curing agents are 2-methylimidazole, 2-ethylhexylimidazole, 2-phenylimidazole and 1-cyanoethyl-2-phenylimidazolium trimellitate, etc.

Examples of the anhydride curing agents are tetrahydrophthalic anhydride, hexahydrophthalic anhydride, methyltetrahydrophthalic anhydride, methylhexahydrophthalic anhydride, methylcyclohexene tetracarboxylic dianhydride, phthalic anhydride, trimellitic anhydride, pyromellitic anhydride, and benzophenonetetracarboxylic dianhydride, etc.

Furthermore, a curing promoter may also be used to facilitate the curing reaction of a polymerizable compound with glycidyl, epoxy or oxetanyl and the curing agent. Examples of the curing promoter are tertiary amines such as benzyldimethylamine, tris(dimethylaminomethyl)phenol and dimethylcyclohexylamine; imidazoles such as 1-cyanoethyl-2-ethyl-4-methylimidazole and 2-ethyl-4-methylimidazole; organo-phosphorus compounds such as triphenylphosphine; quaternary phosphosium salts such as tetraphenylphosphosium bromide; diazobicyclo alkenes such as 1,8-diazobicyclo-[5.4.0]undecene-7 or an organic acid salt thereof; quaternary ammonium salts such as tetraethylammonium bromide, and tetrabutylammonium bromide; boron compounds such as boron trifluoride and triphenyl borate, etc. These curing promoters can be used alone, or in a combination of two or more.

Moreover, a stabilizer is preferably added to prevent unwanted polymerization, for example, during storage. The stabilizer can be any compound well known to a person of ordinary skill in the art; representative examples thereof are 4-ethoxyphenol, hydroquinone and butylated hydroxytoluene (BHT), etc.

### 5-3. Content of Liquid Crystal Composition, etc.

The content of the liquid crystal composition in the polymer/liquid crystal composite material of the invention is preferably as high as possible, so long as it is within a range in which the composite material can exhibit an optically isotropic liquid crystal phase. This is because the electric-birefringence of the composite material of the invention is greater when the content of the liquid crystal composition is higher.

In the polymer/liquid crystal composite material of the invention, the content of the liquid crystal composition is preferably from 60 wt% to 99 wt%, more preferably from 60 wt% to 95 wt% and particularly preferably from 65 wt% to 95 wt%, relative to the composite material. The content of the polymer is preferably from 1 wt% to 40 wt%, more preferably from 5 wt% to 40 wt% and particularly preferably from 5 wt% to 35 wt%, relative to the composite material.

### 5-4. Other Components

The polymer/liquid crystal composite material of the invention may also contain, for example, a dichroic dye and a photochromic compound, without compromising the effects of the invention.

The invention is further described with reference to the examples, but is not limited thereto. Furthermore, "%" denotes "wt%", unless specifically indicated.

### 6. Optical element

The 6^{th} aspect of the invention is an optical element, which contains the liquid crystal composition or the polymer/liquid crystal composite material (both referred to as "liquid crystal medium" hereinafter) of this invention and is driven in an optically isotropic liquid crystal phase.

The liquid crystal medium is optically isotropic in absence of an electric field but exhibits an optical anisotropy in presence of an electric field, so that optical modulation can be achieved with an electric field.

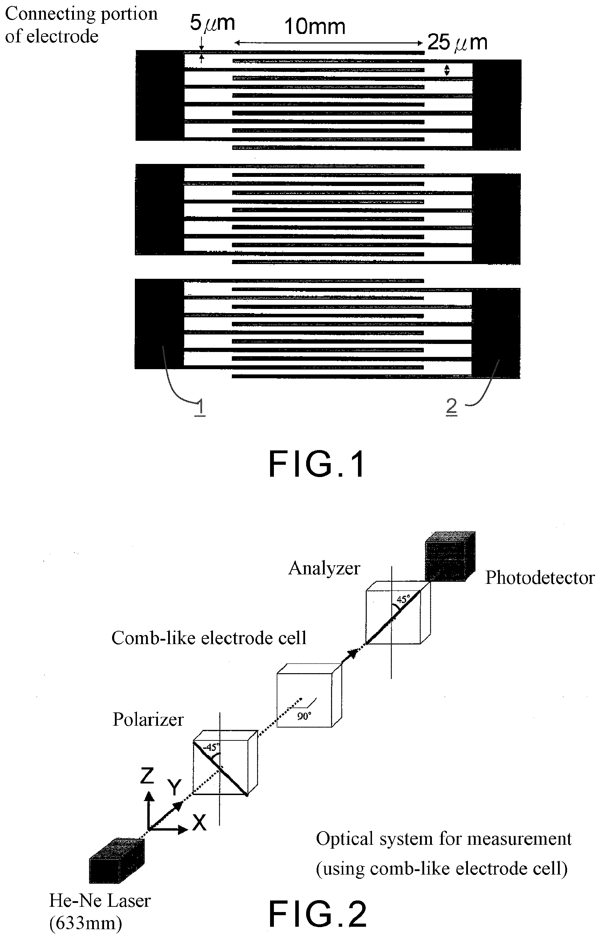

The structure of the liquid crystal display element is, for example, shown in FIG. 1, in which the electrodes on the comb-like electrode substrate are arranged such that parts of the electrode 1 extending from the left side and parts of the electrode 2 extending from the right side are alternatively arranged. When there is a potential difference between the electrodes 1 and 2, the comb-like electrode substrate is provided with an electric field in two directions (upward and downward), as shown in FIG. 1.

### [Examples]

An obtained compound was characterized with the proton nuclear magnetic resonance (¹H-NMR) spectrum and the gas chromatogram obtained from the gas chromatography (GC) analysis. The analysis methods are firstly described below.

¹H-NMR analysis: ¹H-NMR analysis was carried out using DRX-500 (made by Bruker BioSpin). In the measurement, a sample prepared in an example was dissolved in a deuterated solvent capable of dissolving the sample, such as CDCl₃, and was then measured with the NMR apparatus of 500 MHz at room temperature in 24 times of accumulation. In the resulting NMR spectrum, "s" denotes singlet, "d" denotes doublet, "t" denotes triplet, "q" denotes quartet and "m" denotes multiplet. Tetramethylsilane (TMS) was used as the standard of zero chemical shift (*δ*).

GC analysis: GC analysis was carried out using a GC apparatus Model GC-14B (made by Shimadzu Corporation). The column was the capillary column CBP1-M25-025 (length=25 m, inner diameter=0.22 mm, film thickness=0.25 µm) made by Shimadzu Corporation, and the stationary liquid phase was dimethylpolysiloxane (without polarity). The carrier gas was helium, in a flow rate adjusted to 1 ml/min. The sample evaporation chamber was set at 300°C, and the detector (flame ionization detector, FID) was set at 300°C.

A sample was dissolved in toluene to give a solution of 1 wt%, and then 1 *µ*l of the solution was injected into the sample evaporation chamber.

The recorder used was Chromatopac Model C-R6A made by Shimadzu Corporation, or an equivalent thereof. The resulting gas chromatogram showed peak retention times and peak areas corresponding to the component compounds.

The solvent for diluting the sample was, for example, chloroform or hexane, etc. The column used was, for example, capillary column DB-1 (length=30m, inner diameter =0.32 mm, film thickness=0.25 *µ*m) made by Agilent Technologies Inc., HP-1 (length= 30 m, inner diameter=0.32 mm, film thickness=0.25 *µ*m) made by Agilent Technologies Inc., Rtx-1 (length=30 m, inner diameter=0.32 mm, film thickness=0.25 *µ*m) made by Restek Corporation, or BP-1 (length=30 m, inner diameter=0.32 mm, film thickness= 0.25 *µ*m) made by SGE International Pty. Ltd.

The area ratios of the peaks in the gas chromatogram correspond to the ratios of the component compounds. Generally, the weight percentages of the component compounds in the analyzed sample are not completely identical to the area percentages of the peaks. In the invention, however, when the above columns are used, the correction coefficient is substantially equal to one, and therefore the weight percentages of the component compounds in the analyzed sample are substantially equivalent to the area percentages of the peaks. This is because there is no significant difference among the correction coefficients of the component compounds. In order to more accurately calculate the ratios of the liquid crystal compounds in the liquid crystal composition with GC, the internal standard method for GC can be used, wherein GC measurements were simultaneously performed on an accurately weighed specified amount of a liquid crystal compound component (detected component) and a liquid crystal compound as standard (standard), and a relative intensity was calculated in advance as a peak area ratio of the detected component to the standard. If a correction was done using the relative intensity expressed as peak area ratio of each component to the standard, the ratios of the liquid crystal compounds in the liquid crystal composition can be more accurately calculated with GC analysis.

### Samples for Determining Characteristic Values of Liquid Crystal Compounds

Upon measuring the characteristic values, there are two methods, i.e., taking a pure compound as a sample, and mixing a compound in a mother liquid crystal to form a sample.

When a sample prepared by mixing a compound with a mother liquid crystal is measured, the following method is used for the measurement. Firstly, 15 wt% of the obtained liquid crystal compound was mixed with 85 wt% of the mother liquid crystal to prepare a sample, and then the characteristic value of the compound is calculated from the measured value with the extrapolation method according to the equation below.$\left[Extrapolated Value\right] = \left(100\times \left[measured value of the sample\right]-\left[wt% of the mother liquid crystal\right]\times \left[measured value of the mother liquid crystal\right]\right) / \left[wt% of the liquid crystal compound\right]$

While a smectic phase or crystal might be separated at the above ratio of the liquid crystal compound to the mother liquid crystal at 25°C, the ratio of the liquid crystal compound and the mother liquid crystal was changed to 10 wt%:90 wt%, 5 wt%:95 wt% and 1 wt%:99 wt% in order. The composition without separation of a smectic phase or crystal at 25°C was measured for a characteristic value, and the characteristic value of the liquid crystal compound is calculated by extrapolation based on the above equation.

There are numerous mother liquid crystals that can be used for the measurement. For example, the composition of the mother liquid crystal A is as follows (wt%). Mother Liquid Crystal A:

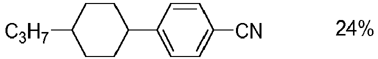

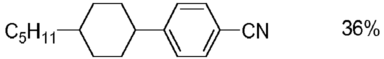

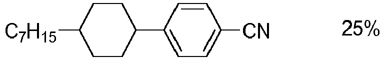

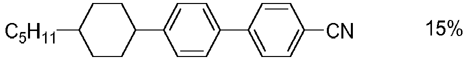

### Method for Measuring Characteristic Values of Liquid Crystal Compounds

The measurement of the characteristic values was carried out with the methods described below. These methods are mostly those described in EIAJ·ED-2521A of the Standard of Electric Industries Association of Japan, or modified versions of the same. Moreover, the TN element used in the measurement was not equipped with TFT.

For the determined values, in a case that the liquid crystal compound itself is used as a sample, the obtained values are recorded as experiment data. In a case that a mixture of the liquid crystal compound and a mother liquid crystal is a sample, the extrapolated values obtained through extrapolation are recorded as experiment data.

The phase structure and the phase transition temperature (°C) were measured using the methods (1) and (2) below.
(1) A compound was placed on a hot plate (Hot Stage FP-52 by Mettler, Corp.) in a melting point measuring apparatus equipped with a polarizing microscope, and the phase behaviour and its change were observed by the polarizing microscope while the sample is heated at a rate of 3°C/min, to determine the type of the liquid crystal phase.
(2) A scanning calorimetry DSC-7 system or Diamond DSC system (made by Perkin Elmer Corp.) is used, in a heating or cooling rate of 3°C/min, and the on-set temperature of the endothermic or exothermic peak accompanying with the phase change of the sample was calculated with extrapolation to determine the phase transition temperature.

Hereinafter, a crystal is represented by "K". In a case where two crystals are distinguished from each other, they are represented by "K₁" and "K₂". A smectic phase is represented by "Sm", a nematic phase is represented by "N", and a liquid (isotropic phase) is represented by "I". In a case that a smectic B phase and a smectic A phase are distinguished from each other in the smectic phase, they are expressed as "SmB" and "SmA". "BP" represents a blue phase or an optically isotropic liquid crystal phase. A biphase co-existence is sometimes represented by (N*+I) or (N*+BP). Specifically, (N*+I) represents a phase in which a non-liquid crystal isotropic phase and a chiral nematic phase coexist, and (N*+BP) represents a phase in which a BP phase or an optically isotropic liquid crystal phase and a chiral nematic phase coexist. "Un" represents a non-optically isotropic unidentified phase. For the expression of the phase transition temperature, for example, "K 50.0 N 100.0 I" means that the phase transition temperature (T_{KN}) from the crystal to the nematic phase is 50.0°C and that (T_{NI}) from the nematic phase to the liquid is 100.0°C. This also applies to the cases of other expressions.

The upper-limit temperature of a nematic phase (T_{NI}, °C): a sample as a mixture of a liquid crystal compound and a mother liquid crystal was placed on a hot plate (Hot Stage FP-52 by Mettler Corp.) in a melting point measuring apparatus equipped with a polarizing microscope, and was observed by the polarizing microscope while heated at a rate of 1°C/min. The temperature at which a part of the sample began to change from a nematic phase to an isotropic liquid was recorded as the upper-limit temperature of the nematic phase, which is sometimes abbreviated to "upper-limit temperature" hereinafter.

Low-temperature compatibility: samples were prepared by mixing a mother liquid crystal with a liquid crystal compound such that the content of the latter was 20 wt%, 15 wt%, 10 wt%, 5 wt%, 3 wt% or 1 wt%, and then placed into glass bottles. The glass bottles were kept in a freezer at -10°C or -20°C for a certain period, and the presence or absence of crystal or a smectic phase was observed.

Viscosity (*η,* determined at 20°C, mPa·s): the viscosity of a mixture of a liquid crystal compound and a mother liquid crystal was measured with an E-type viscometer.

Optical anisotropy (Δ*n*): the measurement was done at 25°C utilizing light of 589 nm, with an Abbe refractometer having a polarizing plate mounted on the ocular lens. After the surface of the main prism is rubbed in a direction, a sample as a mixture of a liquid crystal compound and a mother liquid crystal was dropped onto the main prism. The refractive index *n*_{//} was determined when the polarizing direction was parallel to the rubbing direction, and the refractive index *n*_{⊥} was determined when the polarizing direction was perpendicular to the rubbing direction. The optical anisotropy (Δ*n*) was calculated according to the equation of "Δ*n* = *n*_{//} - *n*_{⊥}".

Dielectric anisotropy (Δ*ε*: determined at 25°C): a sample as a mixture of a liquid crystal compound and a mother liquid crystal was fed into a liquid crystal cell with a distance (cell gap) of 9 *µ*m between two glass substrates and a twist angle of 80°. The liquid crystal cell was applied with a voltage of 20 V, and the dielectric constant (*ε*_{//}) in the major-axis direction of the liquid crystal molecule was determined. Then, a voltage of 0.5 V was applied, and the dielectric constant (*ε*_{⊥}) in the minor axis direction of the liquid crystal molecule was determined. The dielectric anisotropy (Δ*ε*) was calculated according to the equation of "Δ*ε* = *ε_{∥}* - *ε*_{⊥}".

### Pitch (p, determined at 25°C, nm)

The pitch length was measured with selective reflection (Handbook of Liquid Crystal, p. 196, 2000, from Maruzen). For the selective reflection wavelength *λ*, the relationship <*n*>*p*/*λ* = 1 exists, wherein *<n>* denotes the average refractive index and can be calculated from the equation of *"*<*n*> = {(*n_{∥}*² + *n*_{⊥}²*)*/2}^{1/2}". The selective reflection wavelength can be determined by a microspectrophotometer MSV-350 made by Japan Electronics Co., Ltd. The pitch was calculated by dividing the obtained reflection wavelength with the average refractive index <*n*>. When the concentration of the optically active compound is low, the pitch of a cholesteric liquid crystal having a reflection wavelength at the long wavelength side of visible light is proportional to the reciprocal of the concentration. Therefore, multiple points were measured in the pitch length of the liquid crystal having a selective reflection wavelength in the visible light region, and then the pitch was calculated using linear extrapolation method. Herein, the "optically active compound" is equivalent to the chiral dopant in the invention.

### [Example 1]

### Synthesis of Compound (S1)

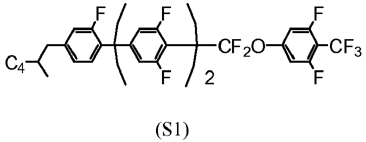

The compound corresponds to formula (1-1-2i) with R^{1a} being -C₄H₉, R^{1b} being hydrogen, L¹ being hydrogen, L², L³ and L⁴ being fluorine and Y¹ being -CF₃.

At first, the synthesis schemes of the compound (S1-03) and the compound (S1-07) as intermediate materials are shown below.

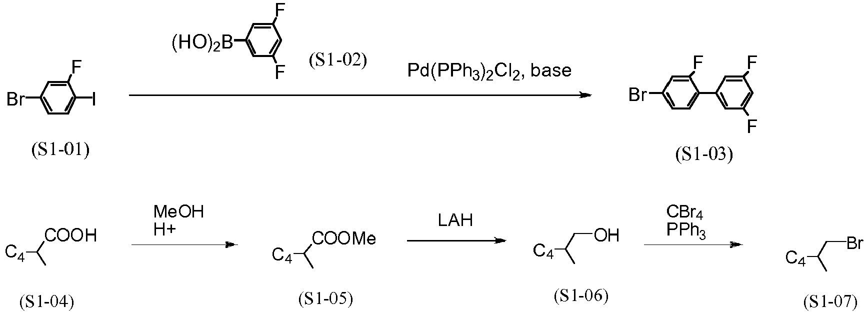

### (Stage 1-1) Synthesis of Compound (S1-03)

Under a nitrogen flow, a mixed solution of 21.5 g (71.6 mmol) of 1-bromo-3-fluoro-4-iodobenzene (S1-01), 11.3 g (71.6 mmol) of 3,5-difluorophenylboric acid (S1-02), 0.503 g (0.716 mmol) of (bistriphenylphosphine)palladium dichloride, 0.375 g (1.43 mmol) of triphenylphosphine, 14.8g (107 mmol) of potassium carbonate, 5.71g (17.9 mmol) of tetrabutylammonium bromide, 100 mL of ethanol and 100 mL of toluene was heated and stirred at 80°C for 6 hours. The reaction solution was poured in water and then extracted twice with 300 mL of toluene. The organic phase was washed three times with water and then concentrated under a reduced pressure. Then, the residue was purified through silica-gel column chromatography using heptane as a solvent to obtain 15.8 g (55.1 mmol, yield: 77%) of the compound (S1-03).

### (Stage 1-2) Synthesis of Compound (S1-06)

Under a nitrogen flow, 1.0 mL of 98% concentrated sulfuric acid was added in a solution of 54.1 g (413 mmol) of 2-methyl-hexanolic acid (S1-04) in 150 mL of methanol, and the mixture was heated and stirred at 70°C for 2 hours. The reaction solution was pured in water and then extracted twice with 300mL of diethyl ether. The organic phase was washed once with sodium bicarbonate water and three times with water, and was then concentrated under an ordinary pressure to obtain 42.6 g (296 mmol, yield: 72%) of the compound (S1-05).

Under a nitrogen flow, a solution of 42.6 g (296 mmol) of the above obtained compound (S1-05) in 150 mL of THF was slowly dripped, at 0°C to 10°C, in a mixed solution of 7.88 g (207 mmol) of LAH (lithium aluminium hydride) and 50 mL of THF, and the mixture was stirred directly at the temperature for 2 hours. After a IN aqueous solution of sulfuric acid was added dropwise in the solution at 0°C, the mixture was poured in water and then extracted by 500 mL of ethyl acetate. The organic phase was washed three times by water and then concentrated under an ordinary pressure to obtain 33.6 g (290 mmol, yield: 98%) of the compound (S1-06).

### (Stage 1-3) Synthesis of Compound (S1-07)

Under a nitrogen flow, a dichloromethane solution of 129 g (388 mmol) of carbon tetrabromide was slowly dripped, at 0°C, into a mixed solution of 30.0 g (259 mmol) of the compound (S1-06) obtained in the precedent stage and 84.8 g (323 mmol) of triphenylphosphine in 200 mL of dichloromethane, and the mixture was stirred at an ordinary temperature for 30 min. The reaction solution was poured in water and then added with 500 mL of dichloromethane. The organic phase was washed three times with water and then concentrated under an ordinary pressure. The residue was purified through silica-gel column chromatography using n-pentane as a solvent to obtain 33.4 g (186 mmol, yield: 72%) of the compound (S1-07).

Next, the obtained compounds (S1-03) and (S1-07) were used to synthesize the compound (S1) through the following scheme.

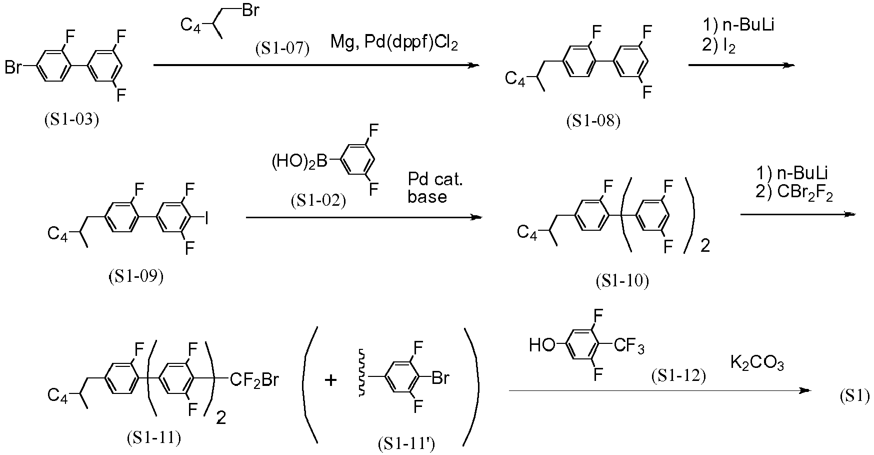

### (Stage 1-4) Synthesis of Compound (S1-08)

Under a nitrogen flow, a solution of 25.0 g (140 mmol) of the compound (S1-07) obtained in the precedent stage in 100 mL of THF was slowly dripped into a mixed solution of 3.39 g (140 mmol) of magnesium and 10 mL of THF to prepare a Grignard reagent, while the system was maintained at 20°C to 30°C. In another container, a mixed solution of 26.7 g (93.1 mmol) of the compound (S1-03) obtained in Stage 1-1, 0.760 g (0.931 mmol) of dichloro{bis(diphenylphosphino)ferrocene} palladium and 200 mL of THF was prepared, the previously obtained Grignard reagent was slowly dripped in, and the mixture was heated and stirred at 50°C for 6 hours. The reaction solution was poured in a IN aqueous solution of HCl and extracted twice with 400 mL of toluene. The organic phase was washed three times with water and once with sodium bicarbonate water, and was then concentrated under a reduced pressure. The residue was purified through silica-gel column chromatography using n-heptane as a solvent to obtain 24.5 g (80.0 mmol, yield: 86%) of the compound (S1-08).

### (Stage 1-5) Synthesis of Compound (S1-09)

Under a nitrogen flow, a 1.67mol/L solution of 88.1 mmol of n-butyl lithium in 52.7 mL of hexane was slowly dripped, at -60°C, into a solution of 24.5 g (80.0 mmol) of the compound (S1-08) obtained in the precedent stage in 150 mL of THF, and the mixture was stirred directly at the temperature for one hour. Then, a solution of 22.3 g (88.1 mmol) of iodine in 100 mL of THF was slowly dripped in the system, and the mixture was stirred directly at the resulting temperature for 2 hours. The reaction solution was poured in water and extracted twice with 300 mL of toluene. The organic phase was washed three times with water and once with an aqueous solution of sodium thiosulfate, and was then concentrated under a reduced pressure. The residue was purified through silica-gel column chromatography using n-heptane as a solvent to obtain 27.6 g (63.9 mmol, yield: 80%) of the compound (S1-09).

### (Stage 1-6) Synthesis of Compound (S1-10)

Under a nitrogen flow, a mixed solution of 27.6 g (63.9 mmol) of the compound (S1-09) obtained in the precedent stage, 10.6 g (67.1 mmol) of 3,5-difluorophenylboric acid, 35.3 g (256 mmol) of potassium carbonate and 6.17 g (19.2 mmol) of tertabutylammonium bromide in 100 mL of toluene, 100 mL of ethanol and 10 mL of water was heated and stirred at 70°C for 3 hours. The reaction solution was poured in water and added with 300 mL of toluene. The organic phase was washed three times with water and once with sodium bicarbonate water, and was then concentrated under a reduced pressure. The residue was purified through silica-gel column chromatography using n-heptane as a solvent to obtain 23.5 g (56.2 mmol, yield: 88%) of the compound (S1-10).

### (Stage 1-7) Synthesis of Compound (S1-11)

Under a nitrogen flow, a 1.67 mol/L solution of 58.3 mmol of n-butyl lithium in 34.9 mL of hexane was slowly dripped, at -40°C, into a solution of 23.2 g (55.5 mmol) of the compound (S1-10) obtained in the precedent stage in 150 mL of THF, and the mixture was stirred directly at the temperature for one hour. Next, a solution of 12.8 g (61.1 mmol) of dibromodifluoromethane in 50 mL of THF was slowly dripped in the system directly at the temperature, and the mixture was stirred for one hour while the temperature slowly returned to the ordinary temperature. The reaction solution was poured in water and extracted with 300 mL of toluene. The organic phase was washed three times with water and then concentrated under a reduced pressure. The residue was purified through silica-gel column chromatography using toluene/n-heptane with a volume ratio of 1:5 as a solvent to obtain 27.6 g (75%, 37.8 mmol, yield: 68%) of a mixture of the compoud (S1-11) and the compoud (S1-11'). In next reaction, the mixture was used directly.

### (Stage 1-8) Synthesis of Compound (S1)

Under a nitrogen flow, a mixed solution of 5.00 g (75%, 6.40 mmol) of the mixture of the compoud (S1-11) and the compoud (S1-11') obtained in the precedent stage, 1.85 g (13.4mmol) of potassium carbonate and 0.206 g (0.640 mmol) of tetrabutylammonium bromide in 50 mL of DMF was heated and stirred at 40°C for 30 min. Then, 1.33 g (6.72 mmol) of 3,5-difluoro-4-trifluoromethylphenol (S1-12) was slowly added in the system, and the mixture was heated and stirred at 80°C for 5 hours. The reaction solution was poured in water and extracted with 100 mL of toluene. The organic phase was washed three times with water and twice with sodium bicarbonate water, and was then concentrated under a reduced pressure. The residue was purified with silica-gel column chromatography using toluene/n-heptane with a volume ratio of 1:5 as a solvent, recrystallization using ethanol/ethyl acetate with a volume ratio of 1:1 as a solvent, and fitration, so that 1.50 g (2.26 mmol, yield: 35%) of the compound (S1) was obtained as the final target material. The phase transition temperatures (°C) of the compound were expressed by "C 72.8 (SmA 56.9 N 69.1) I".

¹H-NMR (CDCl₃): *δ* (ppm) 0.893 (d, 3H), 0.905 (t, 3H), 1.02-1.37 (m, 6H), 1.75 (m, 1H), 2.40 (dd, 1H), 2.69 (dd, 1H), 6.98-7.05 (m, 4H), 7.22 (d, 2H), 7.26 (d, 2H), 7.36 (dd, 1H).
¹⁹F-NMR (CDCl₃): *δ* (ppm) -56.8 (t, 3F), -62.5 (t, 2F), -108.8 (m, 2F), -111.3 (dt, 2F), -114.7 (d, 2F), -118.7 (dd, IF)

### Characteristic valueties of Liquid Crystal Compound (S1)

The four compounds that are described above as the mother liquid crystal A were mixed to prepare the mother liquid crystal A having a nematic phase. The characteristic values of the mother liquid crystal A were as follows:
Upper-limit temperature (T_{NI})=71.7°C; Δ*ε*=11.0; and Δ*n*=0.137.

A liquid crystal composition AS1 was preparded, which included 85 wt% of the mother liquid crystal A and 15 wt% of the compound (S1) obtained in Example 1. The characteristic values of the obtained liquid crystal composition AS1 were measured, and the extrapolated values of the characteristic values of the liquid crystal compound (S1) were calculated from the measured values through extrapolation. The extrapolated values are as follows:
Upper-limit temperature (T_{NI})=35.0°C; Δ*ε*=57.4; and Δ*n*=0.144.

It is clear that the liquid crystal compound (S1) obtained with the above steps is a compound exhibiting a low melting point and also having good compatibility with other liquid crystal compounds and large dielectric anisotropy and optical anisotropy.

### [Example 2]

### Synthesis of Compound (S2)

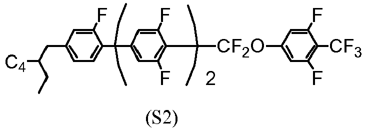

The compound corresponds to formula (1-1-2i) with R^{1a} being -C₄H₉, R^{1b} being -CH₃, L¹ being hydrogen, L², L³ and L⁴ being fluorine, and Y¹ being -CF₃.

The synthesis scheme of the compound (S2) is shown below, wherein 1-bromo-3-ethylheptane (S2-07) was obtained commercially.

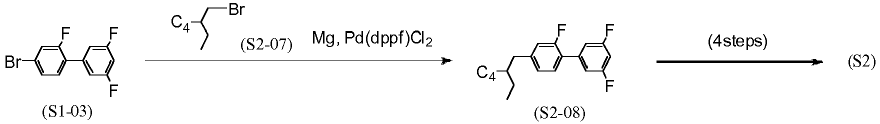

### (Stage 2-1) Synthesis of Compound (S2-08)

With the same operation in Stage 1-4 of Example 1, 5.04 g (26.1 mmol) of the compound (S2-07) was used to obtain 4.64 g (14.5 mmol, yield: 55%) of the compound (S2-08) in the same way.

### (Stage 2-2) Synthesis of Compound (S2)

With the same operations in Stages 1-5 to 1-8 of Example 1, 1.20 g (1.77 mmol, overall yield: 12%) of the compound (S2) was obtained from 4.64 g (14.5 mmol) of the compound (S2-08) obtaind in the precedent stage. The phase transition temperature (°C) of the compound was expressed by "C 64.5 I".
¹H-NMR (CDCl₃): *δ* (ppm) 0.897 (d, 3H), 0.902 (t, 3H), 1.25-1.36 (m, 8H), 1.61 (m, 1H), 2.58 (m, 2H), 6.98-7.06 (m, 4H), 7.22 (d, 2H), 7.26 (d, 2H), 7.35 (dd, 1H).
¹⁹F-NMR (CDCl₃): *δ* (ppm) -56.8 (t, 3F), -62.5 (t, 2F), -108.8 (m, 2F), -111.2 (dt, 2F), -114.8 (d, 2F), -118.7 (dd, IF).

### [Example 3]

### Synthesis of Compound (S3)

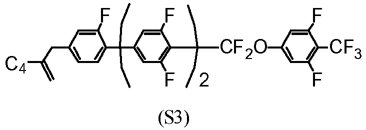

The compound corresponds to formula (1-1-1i) with R^{1a} being -C₄H₉, R^{1b} being hydrogen, L¹ being hydrogen, L², L³ and L⁴ being fluorine, and Y¹ being -CF₃.

The compound (S3) was synthesized according to the following scheme.

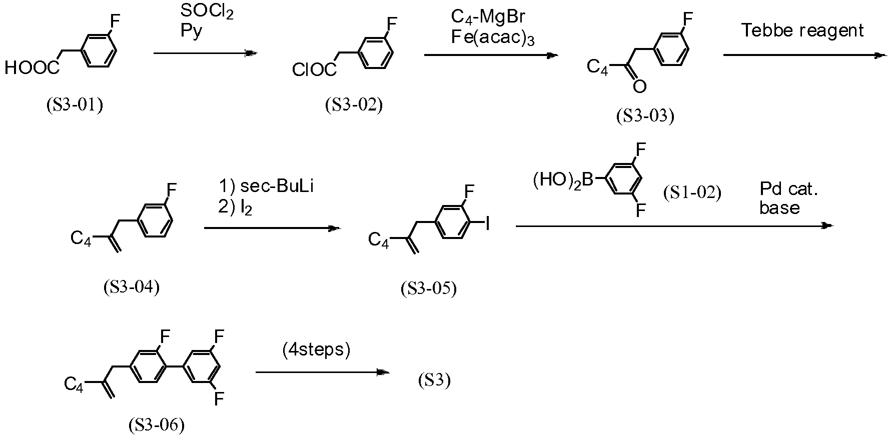

### (Stage 3-1) Synthesis of Compound (S3-03)

Under a nitrogen flow, 0.3 mL of pyridine was added in a solution of 30.0 g (195 mmol) of the carboxylic derivative (S3-01) in 100 mL of toluene, 25.5 g (214 mmol) of thionyl chloride was added while the system was maintained at 40°C to 50°C, and the mixture was heated and stirred directly at the temperature for 30 min. The reaction solution was then directly concentrated under a reduced pressure to obtain 32.0 g (185 mmol) of the compound (S3-02).

Under a nitrogen flow, a 0.91 mol/L solution of 204 mmol of n-butyl magnesium bromide in 224 mL of THF was slowly dripped, at -30°C, into a solution of 32.0 g (185 mmol) of the compound (S3-02) obtained in the precedent stage and 0.960 g of iron (III) acetylacetonate in 250 mL of THF, and the mixture was stirred directly at the temperature for 3 hours. The reaction solution was poured in IN aqueous solution of HCl and extracted with 600 mL of toluene. The organic phase was washed three times with water and once with sodium bicarbonate water, and was then concentrated under a reduced pressure. The residue was purified through silica-gel column chromatography using toluene/n-heptane with a volume ratio of 1:1 as a solvent to obtain 25.0 g (128 mmol, yield: 66%) of the compound (S3-03).

### (Stage 3-2)

Under a nitrogen flow, 250 mL (125 mmol) of Tebbe reagent (0.5 mol/L) was slowly dripped, at -10°C to 0°C, into a solution of 20.2 g (104 mmol) of the compound (S3-03) obtained in the precedent stage in 100 mL of toluene, and the mixture was stirred at 0°C for 20 hours. The reaction solution was poured in water and added with 100 mL of diethyl ether. The organic phase was washed three times with water and then concentrated under a reduced pressure. The residue was purified through silica-gel column chromatography using n-heptane as a solvent to obtain 9.00 g (47 mmol, yield: 45%) of the compound (S3-04).

### (Stage 3-3)

Under a nitrogen flow, a 1.07 mol/L solution of 14.9 mmol of sec-butyl lithium in 14.0 mL of cyclohexane was slowly dripped, at -60°C, into a solution of 1.40 g (7.29 mmol) of the compound (S3-04) obtained in the precedent stage in 50 mL of THF, and the mixture was stirred directly at the temperature for one hour. Next, a solution of 2.04 g (8.02 mmol) of iodine in 10 mL of THF was slowly added dropwise, and the mixture was stirred for one hour while the temperature slowly returned to the ordinary temperature. The reaction solution was poured in water and extracted with 100 mL of toluene. The organic phase was washed three times with water and twice with an aqueous solution of sodium thiosulfate, and was then concentrated under a reduced pressure. The residue was purified with silica-gel column chromatography using n-heptane as a solvent to obtain 1.80 g (5.92 mmol, yield: 81%) of the compound (S3-05).

### (Stage 3-4)

Under a nitrogen flow, a solution of 1.80 g (5.92 mmol) of the compound (S3-05) obtained in the precedent stage, 0.982 g (6.22 mmol) of 3,5-difluorophenylboric acid (S1-02), 3.27 g (23.7 mmol) of potassium carbonate, 0.191 g (0.592 mmol) of tetrabutylammonium bromide and 0.10 g of NX-type Pd/C in 10 mL of ethanol and 10 mL of toluene was heated and stirred at 70°C for 3 hours. The reaction solution was poured in water and added with 100 mL of toluene. The organic phase was washed three times with water and once with sodium bicarbonate water, and was then concentrated under a reduced pressure. The residue was purified through silica-gel column chromatography using n-heptane as a solvent to obtain 1.40 g (4.60 mmol, yield: 78%) of the compound (S3-06).

### (Stage 3-5)

With entirely the same operations in Stages 1-5 to 1-8 of Example 1, 0.40 g (0.604 mmol, overall yield: 13%) of the compound (S3) was obtained from 1.40 g (4.60 mmol) of the compound (S3-06) obtaind in the precedent stage. The phase transition temperature (°C) of the compound was expressed by "C 59.2 I".
¹H-NMR (CDCl₃): *δ* (ppm) 0.905 (t, 3H), 1.31 (m, 2H), 1.44 (m, 2H), 2.00 (t, 2H), 3.38 (s, 2H), 4.79 (s, 1H), 4.90 (d, 1H), 7.00 (d, 2H), 7.05 (dd, 1H), 7.09 (dd, 1H), 7.22 (d, 2H), 7.27 (d, 2H), 7.38 (dd, 1H).
¹⁹F-NMR (CDCl₃): *δ* (ppm) -56.8 (t, 3F), -62.5 (t, 2F), -108.8 (m, 2F), -111.2 (dt, 2F), -114.7 (d, 2F), -118.4 (dd, IF).

### Physical Properties of Liquid Crystal Compound (S3)

A liquid crystal composition AS3 was preparded, which included 85 wt% of the mother liquid crystal A and 15 wt% of the compound (S3) obtained in Example 3. The characteristic values of the obtained liquid crystal composition AS3 were measured, and the extrapolated values of the characteristic values of the liquid crystal compound (S3) were calculated from the measured values through extrapolation. The extrapolated values are as follows:
Upper-limit temperature (T_{NI})=-9.60°C; Δ*ε*=49.7; and Δ*n*=0.124.

It is clear that the liquid crystal compound (S3) obtained with the above steps is a compound exhibiting a low melting point and also having good compatibility with other liquid crystal compounds and large dielectric anisotropy and optical anisotropy.

### [Example 4]

### Synthesis of Compound (S4)

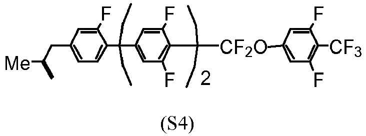

The compound corresponds to formula (1-1-1i) with R^{1a} being -CH₃, R^{1b} being hydrogen, L¹ being hydrogen, L², L³ and L⁴ being fluorine, and Y¹ being -CF₃.

The compound (S4) was synthesized according to the following scheme.

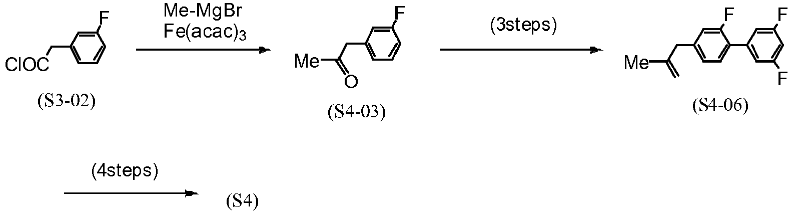

### (Stage 4-1)

In this stage, 2.08 g (13.7mmol, yield: 70%) of the compound (S4-03) was obtained with the same operations in Example 3, except that 3.38 g (19.6 mmol) of the compound (S3-02) obtained in Stage 3-1 was used as a starting material and methyl magnesium bromide was used instead of the butyl magnesium bromide in Stage 3-2.

### (Stage 4-2)

With entirely the same operations in Stages 3-3 to 3-5 of Example 3, 1.83 g (7.00 mmol, overall yield: 51%) of the compound (S4-06) was obtained from 2.08 g (13.7 mmol) of the compound (S4-03) obtaind in the precedent stage.

### (Stage 4-3)

With entirely the same operations in Stages 1-5 to 1-8 of Example 1, 0.890 g (1.47 mmol, overall yield: 21%) of the compound (S4) was obtained from 1.83 g (7.00 mmol) of the compound (S4-06) obtaind in the precedent stage. The phase transition temperatures (°C) of the compound were expressed by "C 89.4 C·93.0 I".
¹H-NMR (CDCl₃): *δ* (ppm) 1.72 (s, 3H), 3.37 (s, 2H), 4.80 (d, 1H), 4.90 (s, 1H), 7.00 (d, 2H), 7.06 (dd, 1H), 7.09 (dd, 1H), 7.22 (d, 2H), 7.27 (d, 2H), 7.38 (dd, 1H).
¹⁹F-NMR (CDCl₃): *δ* (ppm) -56.7 (t, 3F), -62.4 (t, 2F), -108.7 (m, 2F), -111.2 (dt, 2F), -114.5 (d, 2F), -118.3 (dd, IF).

### [Example 5]

### Synthesis of Compound (S5)

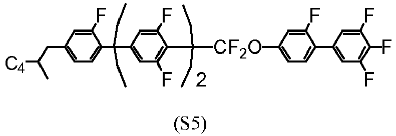

The compound corresponds to formula (1-2-2f) with R^{1a} being -C₄H₉, R^{1b} being hydrogen, L¹ being hydrogen, L², L³, L⁴ and L⁵ being fluorine, and Y¹ being fluorine.

The compound (S5) was synthesized according to the following scheme.

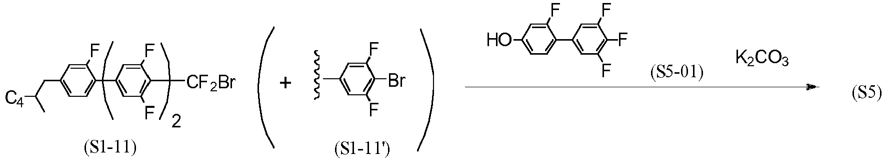

In this example, 1.20 g (1.69 mmol, overall yield: 29%) of the compound (S5) was obtained with the same operations in Example 1, except that 4.00 g (75%, 5.48 mmol) the mixture of the compounds (S1-11) and (S1-11') obtained in Stage 1-7 was used as a starting material and 1.39 g (5.76 mmol) of the compound (S5-01) was used instead of the compound (S1-12) in Stage 1-8. The phase transition temperatures (°C) of the compound were expressed by "C 77.4 N 165.0 I".
¹H-NMR (CDCl₃): *δ* (ppm) 0.894 (d, 3H), 0.905 (t, 3H), 1.19-1.40 (m, 6H), 1.76 (m, 1H), 2.41 (dd, 1H), 2.69 (dd, 1H), 6.99 (d, 1H), 7.04 (d, 1H), 7.15-7.29 (m, 8H), 7.37 (m, 2H).
¹⁹F-NMR (CDCl₃): *δ* (ppm) -61.8 (t, 2F), -111.3 (dt, 2F), -111.4 (d, 2F), -114.9 (dd, 1F), -118.7 (dd, 1F), -134.7 (dd, 2H), -161.7 (tt, 1H).

### Physical Properties of Liquid Crystal Compound (S5)

The four compounds that are described above as the mother liquid crystal A were mixed to prepare the mother liquid crystal A having a nematic phase. The characteristic values of the mother liquid crystal A were as follows:
Upper-limit temperature (T_{NI})=71.7°C; Δ*ε*=11.0; and Δ*n*=0.137.

A liquid crystal composition AS5 was preparded, which included 85 wt% of the mother liquid crystal A and 15 wt% of the compound (S5) obtained in Example 5. The characteristic values of the obtained liquid crystal composition AS5 were measured, and the extrapolated values of the characteristic values of the liquid crystal compound (S5) were calculated from the measured values through extrapolation. The extrapolated values are as follows:
Upper-limit temperature (T_{NI})=91.0°C; Δ*ε*=52.9; and Δ*n*=0.190.

It is clear that the liquid crystal compound (S5) obtained with the above steps is a compound exhibiting a low melting point and also having good compatibility with other liquid crystal compounds and large dielectric anisotropy and optical anisotropy.

### [Example 6]

### Synthesis of Compound (S6)

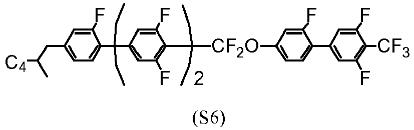

The compound corresponds to formula (1-2-2f) with R^{1a} being -C₄H₉, R^{1b} being hydrogen, L¹ being hydrogen, L², L³, L⁴ and L⁵ being fluorine, and Y¹ being -CF₃.

The compound (S6) was synthesized according to the following scheme.

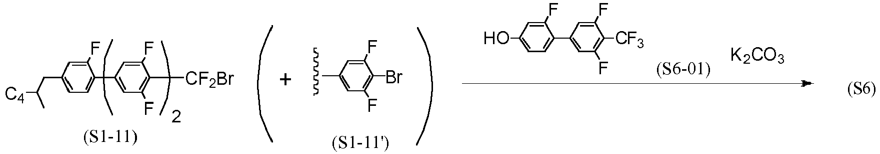

In this example, 1.05 g (1.38 mmol, overall yield: 26%) of the compound (S6) was obtained with the same operations in Example 1, except that 4.00 g (75%, 5.48 mmol) the mixture of the compounds (S1-11) and (S1-11') obtained in Stage 1-7 was used as a starting material and 1.57 g (5.37 mmol) of the compound (S6-01) was used instead of the compound (S1-12) in Stage 1-8. The phase transition temperatures (°C) of the compound were expressed by "C 116.4 SmA 135.2 N 169.1 I".
¹H-NMR (CDCl₃): *δ* (ppm) 0.891 (d, 3H), 0.907 (t, 3H), 1.16-1.40 (m, 6H), 1.76 (m, 1H), 2.41 (dd, 1H), 2.69 (dd, 1H), 6.99 (dd, 1H), 7.04 (dd, 1H), 7.21-7.28 (m, 8H), 7.37 (t, 1H), 7.45 (t, 1H).
¹⁹F-NMR (CDCl₃): *δ* (ppm) -56.8 (t, 3F), -61.8 (t, 2F), -111.1 (m, 2F), -111.3 (dt, 2F), -114.1 (dd, 1F), -114.8 (d, 2H), -118.7 (dd, 1H).

### [Example 7]

### Synthesis of Compound (S7)

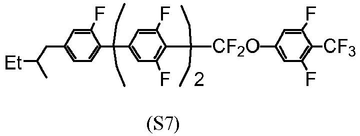

The compound corresponds to formula (1-1-2i) with R^{1a} being ethyl, R^{1b} being hydrogen, L¹ being hydrogen, L², L³ and L⁴ being fluorine, and Y¹ being -CF₃.

The compound (S7) was synthesized according to the following scheme.

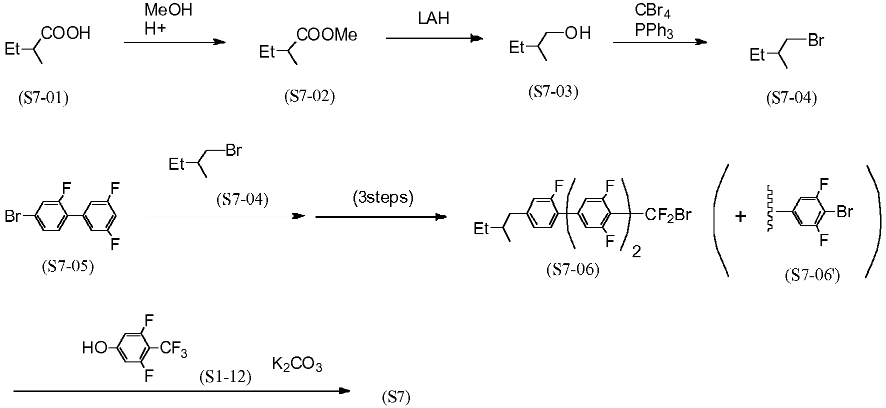

With the same operations in Example 1, 2.50 g (3.93 mmol, yield: 11%) of the compound (S7) was obtained from the compound (S7-01). The phase transition temperatures (°C) of the compound were expressed by "C 84.5 (N 69.9) I".
¹H-NMR (CDCl₃): *δ* (ppm) 0.893 (d, 3H), 0.939 (t, 3H), 1.02-1.25 (m, 1H), 1.38-1.46 (m, 1H), 1.67-1.72 (m, 1H), 2.42 (dd, 1H), 2.69 (dd, 1H), 6.98-7.06 (m, 4H), 7.22 (d, 2H), 7.26 (d, 2H), 7.36 (dd, 1H).
¹⁹F-NMR (CDCl₃): *δ* (ppm) -56.8 (t, 3F), -62.5 (t, 2F), -108.8 (m, 2F), -111.2 (dt, 2F), -114.7 (d, 2F), -118.7 (dd, IF).

### [Reference Example 8]

### Synthesis of Compound (S8)

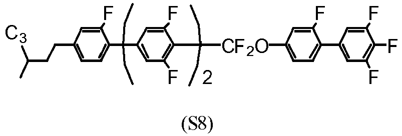

The compound corresponds to formula (1-2-3f) with R^{1a} being -C₃H₇, R^{1b} being hydrogen, L¹ being hydrogen, L², L³, L⁴ and L⁵ being fluorine, and Y¹ being fluorine.

The compound (S8) was synthesized according to the following scheme.

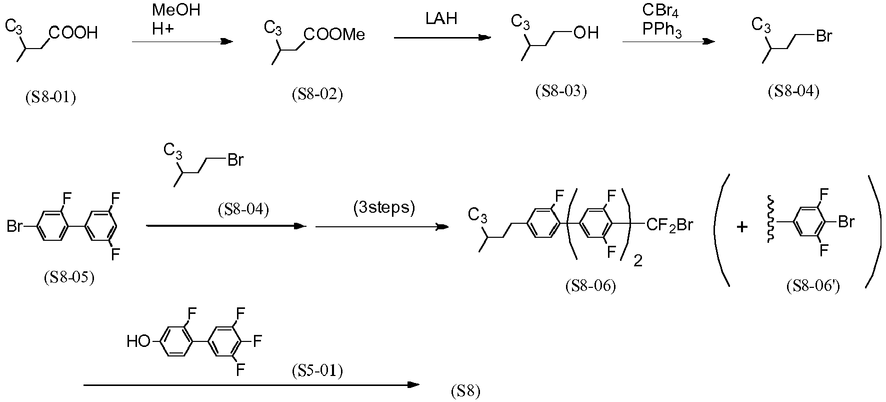

With the same operations in Stages 1-1 to 1-7 of Example 1, 7.0 g (12.8 mmol) of the compound (S8-06) was obtained from the compound (S8-01).

Next, with the same operations in Example 5, 3.17 g (4.48 mmol, yield: 35%) of the compound (S8) was obtained from the compound (S8-06). The phase transition temperature (°C) of the compound was expressed by "C 95.5 SA 102.2 N 153.1 I".
¹H-NMR (CDCl₃): *δ* (ppm) 0.902 (t, 3H), 0.950 (d, 3H), 1.16-1.51 (m, 6H), 1.63-1.67 (m, 1H), 2.60-2.73 (m, 2H), 7.03 (dd, 1H), 7.08 (dd, 1H), 7.16-7.26 (m, 8H), 7.36 (dd, 1H), 7.39 (dd, 1H)
¹⁹F-NMR (CDCl₃): *δ* (ppm) -61.7 (t, 2F), -111.2 (dt, 2F), -114.7 (d, 2F), -114.9 (dd, 1F), -118.5 (dd, 1F), -134.7 (dd, 2F), -161.8 (tt, IF)

### [Reference Example 9]

### Synthesis of Compound (S9)

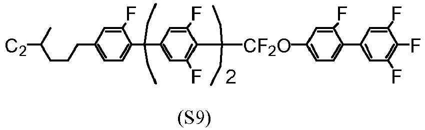

The compound corresponds to formula (1-2-5f) with R^{1a} being ethyl, R^{1b} being hydrogen, L¹ being hydrogen, L², L³, L⁴ and L⁵ being fluorine, and Y¹ being fluorine.

The compound (S9) was synthesized according to the following scheme.

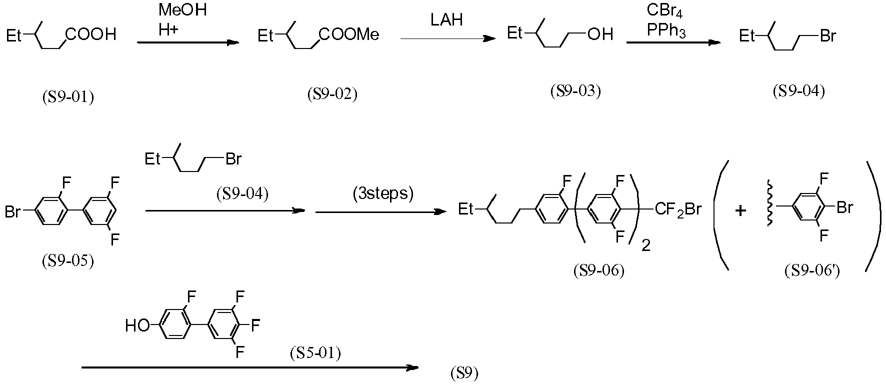

With the same operations in Stages 1-1 to 1-7 of Example 1, 7.5 g (13.7 mmol) of the compound (S9-06) was obtained from the compound (S9-01).

Next, with the same operations in Example 5, 3.55 g (5.01 mmol, yield: 36%) of the compound (S9) was obtained from the compound (S9-06). The phase transition temperatures (°C) of the compound were expressed by "C 93.6 (SA 64.2) N 163.0 I".
¹H-NMR (CDCl₃): *δ* (ppm) 0.870 (t, 3H), 0.871 (d, 3H), 1.14-1.22 (m, 2H), 1.32-1.40 (m, 3H), 1.59-1.71 (m, 2H), 2.61-2.68 (m, 2H), 7.03 (dd, 1H), 7.09 (dd, 1H), 7.16-7.26 (m, 8H), 7.36 (dd, 1H), 7.39 (dd, 1H).
¹⁹F-NMR (CDCl₃): *δ* (ppm) -61.8 (t, 2F), -111.2 (dt, 2F), -114.7 (d, 2F), -114.9 (dd, 1F), -118.5 (dd, 1F), -134.7 (dd, 2F), -161.7 (tt, IF).

### [Reference Example 10]

### Synthesis of Compound (S10)

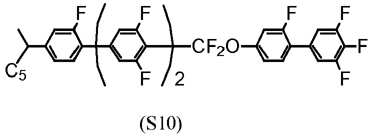

The compound corresponds to formula (1-2-7f) with R^{1a} being methyl, R^{1b} being -C₅H₁₁, L¹ being hydrogen, L², L³, L⁴ and L⁵ being fluorine, and Y¹ being fluorine.

The compound (S10) was synthesized according to the following scheme.

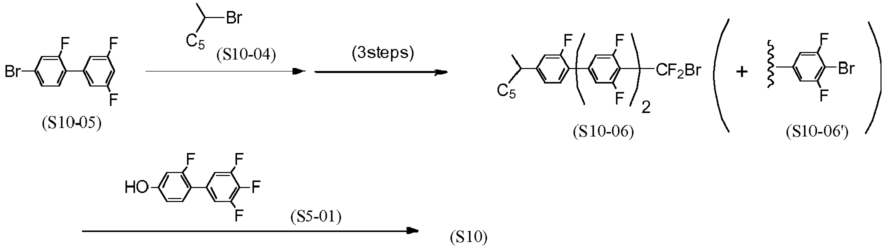

The compound (S10-04) was obtained commercially. With the same operations in Example 5, 2.00 g (2.82 mmol, yield: 33%) of the compound (S10) was obtained from the compound (S10-04). The phase transition temperatures (°C) of the compound were expressed by "C 115.4 N 176.6 I".
¹H-NMR (CDCl₃): *δ* (ppm) 0.871 (t, 3H), 1.18-1.26 (m, 6H), 1.27 (d, 3H), 1.57-1.60 (m, 2H), 2.74 (m, 1H), 7.02 (dd, 1H), 7.08 (dd, 1H), 7.16-7.27 (m, 8H), 7.37 (dd, 1H), 7.39 (dd, 1H).
¹⁹F-NMR (CDCl₃): *δ* (ppm) -61.8 (t, 2F), -111.3 (dt, 2F), -114.8 (d, 2F), -114.9 (dd, 1F), -118.3 (dd, 1F), -134.7 (dd, 2F), -161.7 (tt, IF).

### [Comparative Example]

The phase transition temperatures of the compounds (ref. 01) to (ref. 03) included in the Patent Documents are as follows.

| | | |
|---|---|---|
| 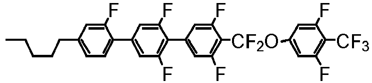 | (ref. 01) | C 92.7 N 98.7 I |
| 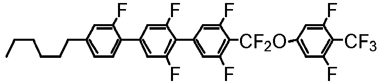 | (ref. 02) | C 93.9 N 94.8 I |
| 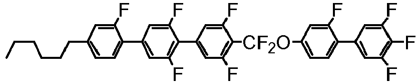 | (ref. 03) | C 91.0 (SA 81.3) N 189.2 I |

It is clear that the melting point of the compound (S1) of this invention is lower by about 20°C than those of the similar compounds (ref. 01) and (ref. 02).

It is clear that the melting point of the compound (S3) of this invention is lower by about 32°C than those of the similar compounds (ref. 01) and (ref. 02).

It is clear that the melting point of the compound (S5) of this invention is lower by about 17°C than that of the similar compound (ref. 03).

### (Composition of the Invention)

In the invention, the characteristic values of a liquid crystal composition were measured by the methods described below, which are mostly the methods described in EIAJ·ED-2521A of the Standard of Electric Industries Association of Japan, or modified versions of the same. The TN element used in the measurement was not equipped with TFT.

Upper-limit temperature of a nematic phase (NI, °C): a sample was placed on a hot plate in a melting point measuring apparatus equipped with a polarizing microscope and heated at a rate of 1°C/min. The temperature at which a part of the sample began to change from a nematic phase to an isotropic liquid was recorded as the upper-limit temperature of the nematic phase, which is sometimes abbreviated to "upper-limit temperature" below.

Lower-limit temperature of a nematic phase (T_{C}, °C): a sample having a nematic phase was kept in a freezer at 0°C, -10°C, -20°C, -30°C or -40°C for 10 days, and observed for the liquid crystal phase. For example, in a case where the sample exhibits a nematic phase at -20°C but changes to crystal or a smectic phase at -30°C, the T_{C} is recorded as "≤-20°C". Hereinafter, the lower-limit temperature of a nematic phase is often abbreviated to "lower-limit temperature".

Transition temperature of an optically isotropic liquid crystal phase: a sample was placed on a hot plate in a melting point measuring apparatus equipped with a polarizing microscope with crossed Nicols, which was initially heated to a temperature allowing formation of a non-liquid crystal isotropic phase, and then cooled in a rate of 1°C/min until a chiral nematic phase or an optically isotropic liquid crystal phase was completely formed. The phase transition temperature during this cooling process was measured. Then, the temperature was raised in a rate of 1°C/min, and the phase transition temperature during this heating process was measured. In the invention, unless specifically indicated, the phase transition temperature in the heating process was recorded as the phase transition temperature. When it was difficult to determine the phase transition temperature of the optically isotropic liquid crystal phase in the dark field under crossed Nicols, the phase transition temperature could be determined after the polarizing plate is deviated from the crossed Nicol state by 1° to 10°.

Viscosity (*η,* determined at 20°C, mPa·s): The viscosity was measured with an E-type viscometer.

Rotation viscosity (*γ*1, determined at 25°C, mPa·s):
1) For a sample with a positive dielectric anisotropy: the measurement was done following the method described in M. Imai et al., Molecular Crystals and Liquid Crystals, Vol. 259, 37 (1995). The sample was placed into a TN element with a twist angle of 0° and a distance (cell gap) of 5 *µ*m between two glass substrates. The TN element was applied with a voltage in a range of 16 to 19.5 V, stepwise by 0.5 V. After a period of 0.2 second with no application of voltage, a voltage application was repeated with a rectangular wave (rectangular pulse of 0.2 second) followed by a period of 2 seconds of no voltage. The peak current and the peak time of the transient current resulting from the application of the voltage were measured. Then, the value of rotation viscosity was calculated based on the measurements and Equation (8) described in page 40 of the paper of M. Imai et al. The dielectric anisotropy required for this calculation was obtained by using the element used in the measurement of the rotation viscosity, following the later-described method for determining dielectric anisotropy.
2) For a sample with a negative dielectric anisotropy: the measurement was done following the method described in M. Imai et al., Molecular Crystals and Liquid Crystals, Vol. 259, 37 (1995). The sample was placed into a vertical alignment element with a distance (cell gap) of 20 *µ*m between two glass substrates. The element was applied with a voltage in a range of 30 to 50 V, stepwise by 1 V. After a period of 0.2 second without voltage application, a voltage application was repeated with a rectangular wave (rectangular pulse of 0.2 second) followed by a period of 2 seconds of no voltage. The peak current and the peak time of the transient current resulting from the voltage application were measured. Then, the value of rotation viscosity was calculated based on the measured values and Equation (8) described in page 40 of the paper of M. Imai et al. The dielectric anisotropy value required for this calculation was obtained by using the method described below.

Optical anisotropy (Δ*n,* determined at 25°C): the measurement was done using light of 589 nm, with an Abbe refractometer having a polarizing plate mounted on the ocular lens. After the surface of the main prism was rubbed in a direction, the sample was dropped onto the main prism. The refractive index *n*_{//} was determined when the polarizing direction was parallel to the rubbing direction, and the refractive index *n*_{⊥} was determined when the polarizing direction was perpendicular to the rubbing direction. The optical anisotropy was calculated according the equation "Δ*n* = *n*_{//} - *n*_{⊥}". When the sample was a composition, the above process could be used to determine the optical anisotropy.

Dielectric anisotropy (Δ*ε*, determined at 25°C): when the sample is a compound, the compound was mixed in a suitable composition and measured for the dielectric anisotropy. The dielectric anisotropy value of the compound was obtained as an extrapolated value.
1) For a composition with a positive dielectric anisotropy: a sample was placed into a liquid crystal cell with a distance (cell gap) of 9 *µ*m between two glass substrates and a twist angle of 80°. The liquid crystal cell was applied with a voltage of 20 V to determine the dielectric constant (*ε*_{//}) in the major-axis direction of the liquid crystal molecule. Then, a voltage of 0.5 V was applied to determine the dielectric constant (*ε*_{⊥}) in the minor axis direction of the liquid crystal molecule. The dielectric anisotropy was calculated according to the equation of "Δ*ε* = *ε*_{//} - *ε*_{⊥}".
2) For a composition with a negative dielectric anisotropy: a sample was placed into a liquid crystal cell processed into homeotropic alignment, and applied with a voltage of 0.5 V to determine the dielectric constant *ε*_{//}. Then, the sample was placed into a liquid crystal cell processed into homogeneous alignment, and applied with a voltage of 0.5 V to determine dielectric constant *ε*_{⊥}. The dielectric anisotropy was calculated according to the equation of "Δ*ε* = *ε*_{//} - *ε*_{⊥}".

Threshold voltage (Vth, determined at 25°C, V): when the sample was a compound, the compound was mixed in a suitable composition and measured for the threshold voltage. The threshold voltage of the compound was obtained as an extrapolated value.
1) For a composition with a positive dielectric anisotropy: a sample was placed into a liquid crystal display element of a normally white mode with a distance of (0.5/Δ*n*) *µ*m between two glass substrates and a twist angle of 80°, in which Δ*n* was the optical anisotropy determined using the above method. A rectangular wave with a frequency of 32 Hz was applied to the element. Then, the magnitude of rectangular wave was increased, and the voltage value at which the transmittance of light through the element reached 90% was determined.
2) For a composition with a negative dielectric anisotropy: a sample was placed into a liquid crystal display element of a normally black mode, which has a distance (cell gap) of 9 *µ*m between two glass substrates and was processed into homeotropic alignment. A rectangular wave with a frequency of 32 Hz was applied to the element. Then, the magnitude of the rectangular wave was increased, and the voltage value at which the transmittance of light through the element reached 10% was determined.

Voltage holding ratio (VHR, determined at 25°C, %): the TN element used for the determination had a polyimide alignment film, and had a distance (cell gap) of 6 *µ*m between two glass substrates. The sample was placed into the element, which was then sealed with a UV-polymerizable adhesive. Then, the TN element was charged by applying a pulse voltage (5V, 60 ms). The voltage attenuation was determined using a high-speed voltmeter at an interval of 16.7 ms, and the area A between the voltage curve and the horizontal axis per unit cycle was calculated. The voltage holding ratio was the percentage of the area A relative to the non-attenuated area B.

Helical pitch (determined at 20°C, *µ*m): The helical pitch was measured by a conoscope. A sample was injected into a conoscope, and then the distance (*a*, *µ*m) between the disclination lines observed from the wedge-type liquid crystal cell was measured. The helical pitch (*p*) could be calculated according to the formula *p* = 2·*a*·tan*θ,* wherein *θ* is the angle between the two glass plates in the wedge-type cell.

Alternatively, the pitch length can be determined with selective reflection (Handbook of Liquid Crystal, p. 196, 2000, by Maruzen). For the selective reflection wavelength *λ*, the relationship <*n*>*p*/*λ* = 1 exists, wherein *<n>* denotes the average refractive index and can be calculated following the equation "<*n*> = {(*n*_{//}*²* + *n*_{⊥}²)/2}^{1/2}. The selective reflection wavelength was determined by a microspectrophotometer MSV-350 manufactured by Japan Electronics Co., Ltd. The pitch was obtained by dividing the measured reflection wavelength with the average refractive index.

When the concentration of the chiral reagent is low, the pitch of a cholesteric liquid crystal having a reflection wavelength at the long wavelength side of visible light is proportional to the reciprocal of the concentration. Therefore, multiple points were measured in the pitch length of the liquid crystal having selective reflection wavelength in the visible light region, and the pitch was calculated with linear extrapolation.

Hereinafter, the proportion (percentage) of a component or a liquid crystal compound is weight percentage (wt%) relative to the total weight of the liquid crystal compounds. The composition can be prepared by mixing the components including liquid crystal compounds after they are weighted. Thus, the wt% of each component can be easily calculated.

### [Example 11]

A liquid crystal composition A was prepared by mixing the following liquid crystal compounds in the following weight percentages. The corresponding general formulae are recited at the right side of the structural formulae, with the compound numbering of the compounds used in the invention.

**Liquid Crystal Composition A:**

| | | |
|---|---|---|
| 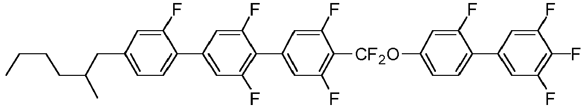 | (S-5) | 52.9wt% |
| 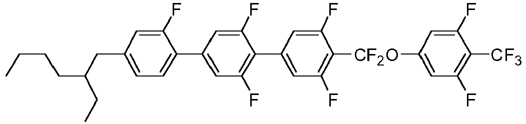 | (S-2) | 47.1wt% |

The phase transition temperature (°C) of the liquid crystal composition A is expressed by "I 94.0-91.4 N".

Next, a liquid crystal composition B, which included 94.0 wt% of the liquid crystal composition A and 6.0 wt% of the chiral dopant BN-H5 expressed by the following formula, was obtained.

The phase transition temperature (°C) of the liquid crystal composition B is expressed by "N* 77.1 BP >78 I". Moreover, the BP structure is uniform at least right above the N* phase without co-existence of the isotropic phase. Furthermore, the BP-I transition could not be clearly identified with the polarizing microscope.

Moreover, BN-H5 was obtained from (R)-(+)-1,1'-bi(2-naphthol) and a corresponding carboxylic acid, with an esterification using dicyclohexylcarbodiimide (DCC).

### BN-H5:

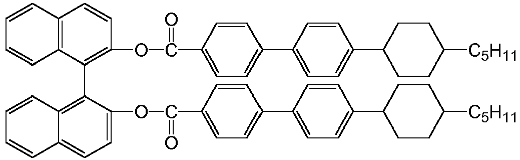

### [Example 12] Preparation of Mixture of Monomer and Liquid Crystal Composition

The liquid crystal composition B of 88.3 wt%, n-dodecyl acrylate of 6.0 wt%, 1,4-di(4-(6-(acryloyloxy)hexyloxy)benzoyloxy)-2-methylbenzene (LCA-6) of 4.7 wt%, and 2,2'-dimethoxyphenylacetophenone as a photo-polymerization initiator of 1.0 wt% were mixed to prepare a mixture B-1M as a mixture of a liquid crystal composition and a monomer.

### LCA-6:

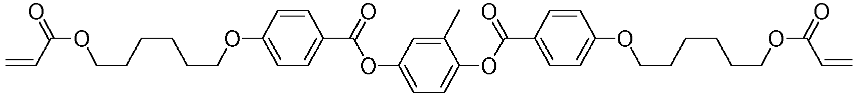

### Preparation of Polymer/Liquid Crystal Composite Material

The liquid crystal mixture B-1M was held between a non-aligned comb-like electrode substrate and the opposite glass substrate (without electrode) with a cell gap of 9 *µ*m, and then the resulting liquid crystal cell was heated until the liquid crystal became a blue phase at 46.0°C. In this state, the cell was irradiated with UV light of 365 nm in an intensity of 23 mW·cm⁻² for 1 min for polymerization.

The polymer/liquid crystal composite material B-1P thus prepared maintained an optically isotropic liquid crystal phase even after being cooled to room temperature.

Moreover, as shown in FIG. 1, the electrodes on the comb-like electrode substrate were arranged such that parts of the electrode 1 extending from the left side and parts of the electrode 2 from the right side were alternatively arranged. Therefore, when a potential difference is present between the electrodes 1 and 2, the comb-like electrode substrate is provided with an electric field in two (upward and downward) directions of the figure in the view of one electrode, as shown in FIG. 1.

### [Example 13]

The polymer/liquid crystal composite material B-1P obtained in Example 12 was held between a liquid crystal cell and arranged in the optical system of FIG. 2 to measure its electrooptical properties. The light source was the white light source of a polarizing microscope Eclipse LV100POL manufactured by Nikon. The above liquid crystal cell was arranged in the optical system in a manner such that the incident light on the cell was perpendicular to the surface of the cell, and the line direction of the comb-like electrode was at 45° with respect to the polarizer and the analyzer, respectively. The correlation of the voltage and the transmittance was investigated at room temperature. When a rectangular wave of 55 V was applied, the transmittance was 85% or more and the transmitted light intensity was saturated.

### [Example 14]

A liquid crystal composition C was prepared by mixing the following liquid crystal compounds in the following weight percentages. The corresponding general formulae are recited at the right side of the structural formulae, with the compound numbering of the compounds used in the invention.

**Liquid Crystal Composition C:**

| | | |
|---|---|---|
| 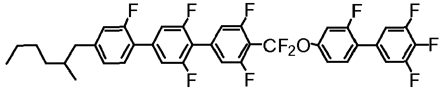 | (S-5) | 9.9% |
| 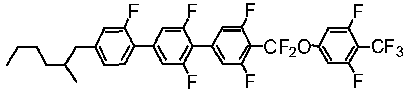 | (S-1) | 15.5% |
| 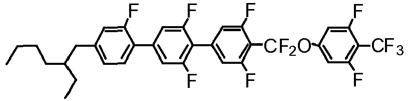 | (S-2) | 7.8% |
| 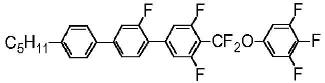 | (9-18) | 1.9% |
| 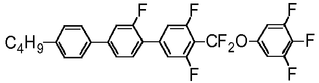 | (9-18) | 1.9% |
| 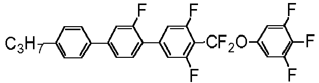 | (9-18) | 1.9% |
| 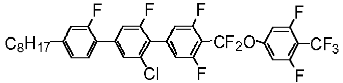 | (9-18) | 6.8% |
| 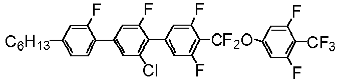 | (9-18) | 10.0% |
| 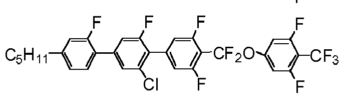 | (9-18) | 2.0% |
| 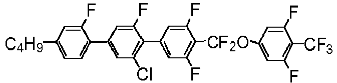 | (9-18) | 6.8% |
| 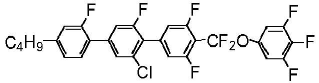 | (9-18) | 4.7% |
| 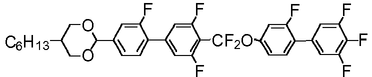 | (10-3) | 3.3% |
| 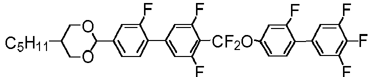 | (10-3) | 5.3% |
| 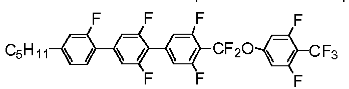 | (9-18) | 1.9% |
| 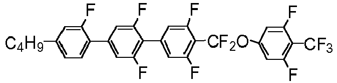 | (9-18) | 1.4% |
| 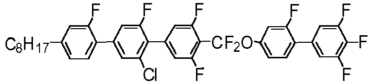 | (10-1) | 6.9% |
| 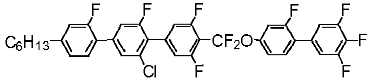 | (10-1) | 6.8% |
| 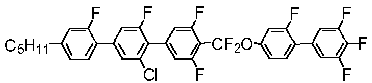 | (10-1) | 2.0% |
| 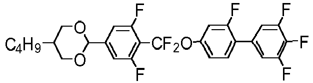 | (9-21) | 3.2% |

The phase transition temperature (°C) of the liquid crystal composition C is expressed by "I 86-88 N".

Next, a liquid crystal composition D, which included 93.7 wt% of the liquid crystal composition C, 3.0 wt% of the chiral dopant BN-H4 expressed by the following formula and 3.3 wt% of the above chiral dopant BN-H5, was obtained.

The phase transition temperature (°C) of the liquid crystal composition D is expressed by "N* 71.4-71.8 BP 74.3-77.4 I". Moreover, in the range of 71.8 to 74.3°C, the BP structure is uniform without co-existance of the N* phase or the isotropic phase.

### BN-H4:

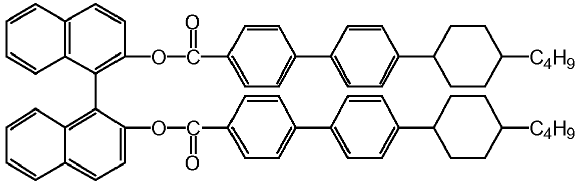

### [Example 15] Preparation of Mixture of Monomer and Liquid Crystal Composition

The liquid crystal composition D of 88.3 wt%, n-dodecyl acrylate of 6.0 wt%, 1,4-di(4-(6-(acryloyloxy)hexyloxy)benzoyloxy)-2-methylbenzene (LCA-6) of 4.7 wt%, and 2,2'-dimethoxyphenylacetophenone as a photo-polymerization initiator of 1.0 wt% were mixed to prepare a mixture D-1M as a mixture of a liquid crystal composition and a monomer.

### LCA-6:

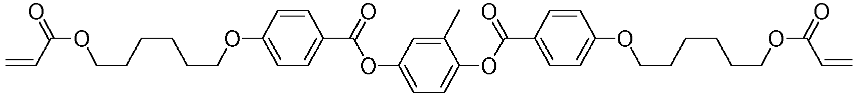

### Preparation of Polymer/Liquid Crystal Composite Material

The liquid crystal mixture D-1M was held between a non-aligned comb-like electrode substrate and the opposite glass substrate (without electrode) with a cell gap of 9 *µ*m, and then the resulting liquid crystal cell was cooled from 40°C in a rate of 2°C/min until the liquid crystal became a blue phase (a supercooled blue phase) at 33.0°C. In this state, the cell was irradiated with UV light of 365 nm in an intensity of 23 mW·cm⁻² for 1 min for polymerization.

The polymer/liquid crystal composite material D-1P thus prepared maintained an optically isotropic liquid crystal phase even after being cooled to room temperature.

### [Example 16]

The polymer/liquid crystal composite material D-1P obtained in Example 15 was held between a liquid crystal cell and arranged in the optical system of FIG. 2 to measure its electrooptical properties. The light source was the white light source of a polarizing microscope Eclipse LV100POL manufactured by Nikon. The above liquid crystal cell was arranged in the optical system in a manner such that the incident light on the cell was perpendicular to the surface of the cell, and the line direction of the comb-like electrode was at 45° with respect to the polarizer and the analyzer, respectively. The correlation of the voltage and the transmittance was investigated at room temperature. When a rectangular wave of 62.5 V was applied, the transmitted light intensity was saturated.

### [Example 17]

A liquid crystal composition E was prepared by mixing the following liquid crystal compounds in the following weight percentages. The corresponding general formulae are recited at the right side of the structural formulae, with the compound numbering of the compounds used in the invention.

**Liquid Crystal Composition E:**

| | | |
|---|---|---|
| 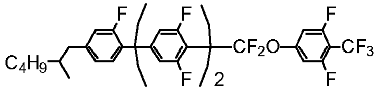 | (S6) | 10.0% |
| 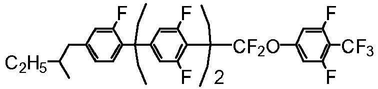 | (S7) | 10.0% |
| 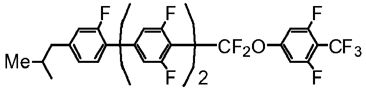 | (S8) | 10.0% |
| 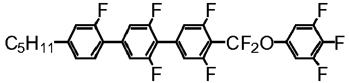 | (9-18) | 3.0% |
| 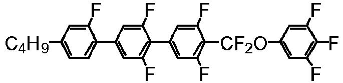 | (9-18) | 3.0% |
| 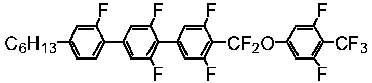 | (9-18) | 5.5% |
| 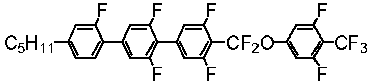 | (9-18) | 5.5% |
| 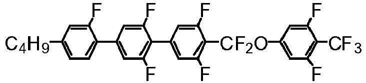 | (9-18) | 5.5% |
| 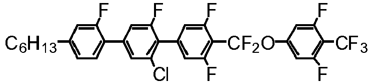 | (9-18) | 4.7% |
| 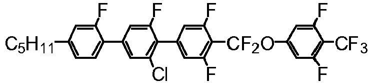 | (9-18) | 4.7% |
| 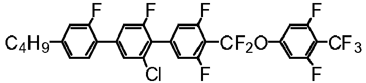 | (9-18) | 4.7% |
| 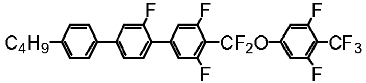 | (9-18) | 4.5% |
| 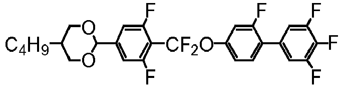 | (9-21) | 10.0% |
| 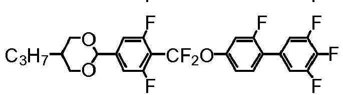 | (9-21) | 10.0% |
| 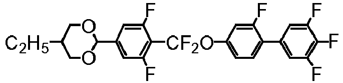 | (9-21) | 10.0% |
| 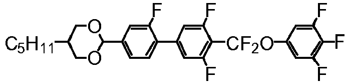 | (10-3) | 4.5% |
| 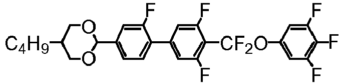 | (10-3) | 4.4% |
| 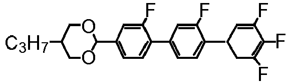 | (9-3) | 10.0% |

The phase transition temperature (°C) of the liquid crystal composition E is expressed by "N 81.5-82.2 I".

Next, a liquid crystal composition F, which included 94.7 wt% of the liquid crystal composition E and 5.3 wt% of the above chiral dopant BN-H5, was obtained.

The phase transition temperature (°C) of the liquid crystal composition F is expressed by "N* 72.5-72.6 BP 73.6-74.6 I". Moreover, in the range of 72.5 to 73.6°C, the BP structure is uniform without co-existance of the N* phase or the isotropic phase.

### BN-H5:

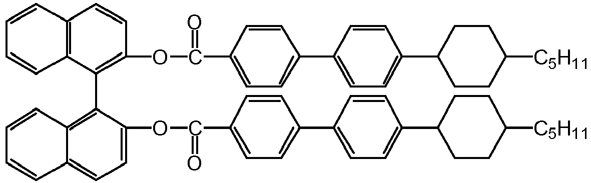

### [Example 18] Preparation of Mixture of Monomer and Liquid Crystal Composition

The liquid crystal composition F of 88.3 wt%, n-dodecyl acrylate of 6.0 wt%, 1,4-di(4-(6-(acryloyloxy)hexyloxy)benzoyloxy)-2-methylbenzene (LCA-6) of 4.7 wt%, and 2,2'-dimethoxyphenylacetophenone as a photo-polymerization initiator of 1.0 wt% were mixed to prepare a mixture F-1M as a mixture of a liquid crystal composition and a monomer.

### LCA-6:

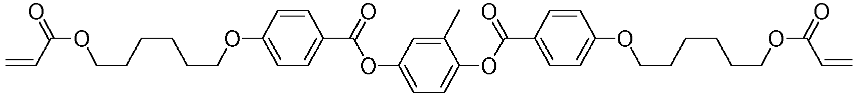

### Preparation of Polymer/Liquid Crystal Composite Material

The liquid crystal mixture F-1M was held between a non-aligned comb-like electrode substrate and the opposite glass substrate (without electrode) with a cell gap of 9 *µ*m, and then the resulting liquid crystal cell was heated from 38.0°C in a rate of 2°C/min until the liquid crystal became a blue phase at 43.0°C. In this state, the cell was irradiated with UV light of 365 nm in an intensity of 23 mW·cm⁻² for 1 min for polymerization.

The polymer/liquid crystal composite material F-1P thus prepared maintained an optically isotropic liquid crystal phase even after being cooled to room temperature.

### [Example 19]

The polymer/liquid crystal composite material F-1P obtained in Example 18 was held between a liquid crystal cell and arranged in the optical system of FIG. 2 to measure its electrooptical properties. The light source was the white light source of a polarizing microscope Eclipse LV100POL manufactured by Nikon. The above liquid crystal cell was arranged in the optical system in a manner such that the incident light on the cell was perpendicular to the surface of the cell, and the line direction of the comb-like electrode was at 45° with respect to the polarizer and the analyzer, respectively. The correlation of the voltage and the transmittance was investigated at room temperature. When a rectangular wave of 40.0 V was applied, the transmitted light intensity was saturated.

### [Industrial Utility]

The applications of the invention include, for example, optical elements, such as display elements using polymer/liquid crystal composites, and so on.

## Claims

1. A compound represented by formula (1), 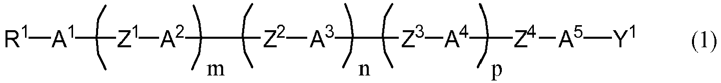 wherein in formula (1), R¹ is C₄₋₂₀ alkyl or alkenyl branching at a carbon of 2-position thereof; rings A¹, A², A³, A⁴ and A⁵ are independently 1,4-phenylene, 1,3-dioxane-2,5-diyl, tetrahydropyran-2,5-diyl, tetrahydropyran-3,6-diyl, pyrimidine-2,5-diyl, pyridine-2,5-diyl or naphthalene-2,6-diyl, and in these rings arbitrary hydrogen is optionally replaced by fluorine or chlorine, Z¹, Z², Z³ and Z⁴ are independently a single bond, or C₁₋₄ alkylene in which arbitrary -CH₂- is optionally replaced by -O-, -COO-, -OCO- or -CF₂O-, arbitrary -CH₂-CH₂-is optionally replaced by -CH=CH-, -CF=CF- or -C≡C-, and arbitrary hydrogen is optionally replaced by halogen; Y¹ is fluorine, chlorine, -SF₅, -C≡N, -N=C=S, or C₁₋₃ alkyl in which arbitrary hydrogen is replaced by halogen, and in the alkyl arbitrary -CH₂- is optionally replaced by -O-, and arbitrary -CH₂-CH₂- is optionally replaced by -CH=CH- or -C≡C-; m is 1, n is 1, and p is 0 or 1.

2. The compound of claim 1, wherein in formula (1), arbitrary one of the rings A¹, A², A³ and A⁴ is 1,4-phenylene in which one or two hydrogens are replaced by fluorine.

3. The compound of claim 1, which is represented by arbitrary one of formulae (1-1) to (1-2), 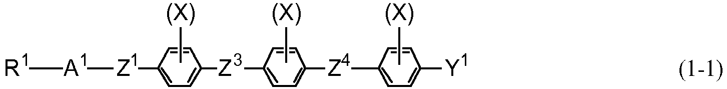 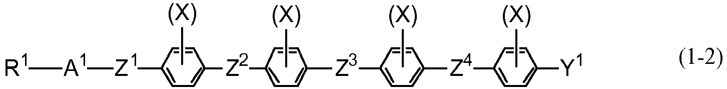 wherein in formulae (1-1) to (1-2), R¹ is C₄₋₂₀ alkyl or alkenyl branching at a carbon of 2-position thereof; the ring A¹ is 1,4-phenylene, 1,3-dioxane-2,5-diyl, tetrahydropyran-2,5-diyl, tetrahydropyran-3,6-diyl, pyrimidine-2,5-diyl or pyridine-2,5-diyl, and in these rings arbitrary hydrogen is optionally replaced by fluorine; Z¹, Z², Z³ and Z⁴ are independently a single bond, -CH₂-CH₂-, -COO- or -CF₂O-, with a proviso that arbitrary one of Z¹, Z², Z³ and Z⁴ is -COO- or -CF₂O-; Y¹ is fluorine, chlorine, -C≡N, or C₁₋₃ alkyl in which arbitrary hydrogen is replaced by fluorine, and in the alkyl arbitrary -CH₂- is optionally replaced by -O- and arbitrary -CH₂-CH₂- is optionally replaced by -CH=CH-; X is fluorine or chlorine, wherein a symbol of 1,4-phenylene and (X) connected by a straight line, 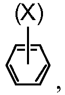 represents 1,4-phenylene in which one or two hydrogens are optionally replaced by X.

4. The compound of claim 3, which is represented by arbitrary one of formulae (1-1-1) and (1-1-2) and formulae (1-2-1), (1-2-1), (1-2-9) and (1-2-10), 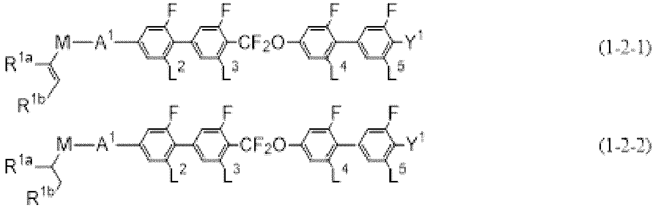 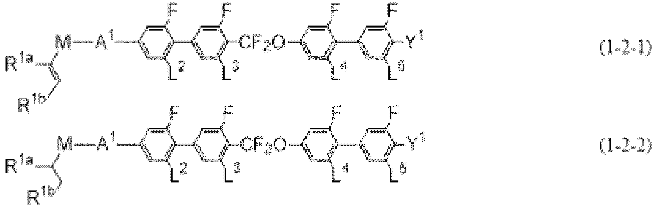 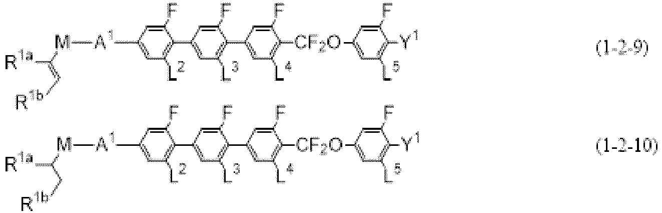 wherein in formulae (1-1-1) and (1-1-2) and (1-2-1), (1-2-2), (1-2-9) and (1-2-10), R^{1a} is C₁₋₁₀ alkyl in which arbitrary -CH₂-CH₂- is optionally replaced by -CH=CH-; R^{1b} is hydrogen, or C₁₋₁₀ alkyl in which arbitrary - CH₂-CH₂- is optionally replaced by -CH=CH-; M is -CH₂-; L², L³, L⁴ and L⁵ are independently hydrogen, fluorine or chlorine; the ring A¹ is 1,4-phenylene, 1,3-dioxane-2,5-diyl, tetrahydropyran-2,5-diyl, tetrahydropyran-3,6-diyl, pyrimidine-2,5-diyl or pyridine-2,5-diyl, and in these rings arbitrary hydrogen is optionally replaced by fluorine; Y¹ is fluorine, chlorine, -C≡N, C₁₋₃ alkyl in which arbitrary hydrogen is replaced by fluorine, alkenyl in which arbitrary hydrogen is replaced by fluorine, or alkoxy in which arbitrary hydrogen is replaced by fluorine.

5. A liquid crystal composition, comprising the compound of any one of claims 1 to 4 and a chiral dopant, and exhibiting an optically isotropic liquid crystal phase.

6. The liquid crystal composition of claim 5, further comprising at least one compound selected from the group consisting of compounds represented by formulae (2), (3) and (4), 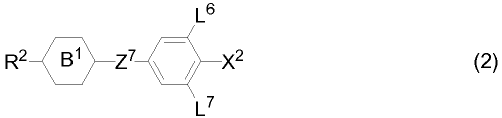 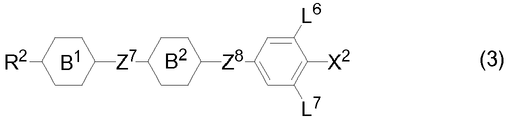 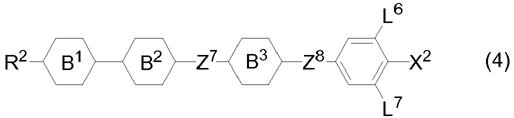 wherein R² is straight alkyl of C₁₋₁₀ or straight alkenyl of C₂₋₁₀, and in the alkyl and the alkenyl, arbitrary hydrogen is optionally replaced by fluorine and arbitrary -CH₂- is optionally replaced by -O-; X² is fluorine, chlorine, -OCF₃, -OCHF₂, -CF₃, -CHF₂, -CH₂F, -OCF₂CHF₂ or - OCF₂CHFCF₃; ring B¹, ring B² and ring B³ are independently 1,4-cyclohexylene, 1,3-dioxane-2,5-diyl, pyrimidine-2,5-diyl, tetrahydropyran-2,5-diyl, 1,4-phenylene, naphthalene-2,6-diyl, 1,4-phenylene in which arbitrary hydrogen is replaced by fluorine, or naphthalene-2,6-diyl in which arbitrary hydrogen is replaced by fluorine or chlorine; Z⁷ and Z⁸ are independently - (CH₂)₂-, -(CH₂)₄-, -COO-, -CF₂O-, -OCF₂-, -CH=CH-, -C≡C-, -CH₂O- or a single bond; L⁶ and L⁷ are independently hydrogen or fluorine.

7. The liquid crystal composition of claims 5 or 6, further comprising at least one compound selected from the group consisting of compounds represented by formula (5), or at least one compound selected from the group consisting of compounds represented by formula (6), 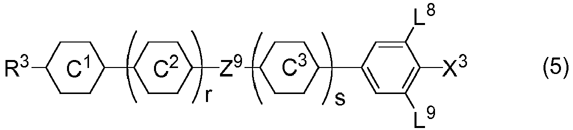 wherein R³ is straight alkyl of C₁₋₁₀ or straight alkenyl of C₂₋₁₀, and in the alkyl and the alkenyl, arbitrary hydrogen is optionally replaced by fluorine and arbitrary -CH₂- is optionally replaced by -O-; X³ is -C≡N or -C≡C-C≡N; ring C¹, ring C² and ring C³ are independently 1,4-cyclohexylene, 1,4-phenylene, 1,4-phenylene in which arbitrary hydrogen is replaced by fluorine, naphthalene-2,6-diyl, naphthalene-2,6-diyl in which arbitrary hydrogen is replaced by fluorine or chlorine, 1,3-dioxane-2,5-diyl, tetrahydropyran-2,5- diyl or pyrimidine-2,5-diyl; Z⁹ is -(CH₂)₂-, -COO-, -CF₂O-, -OCF₂-, -C≡C-, -CH₂O- or a single bond; L⁸ and L⁹ are independently hydrogen or fluorine; and r is 1 or 2, s is 0 or 1, and r+s is 0, 1 or 2, 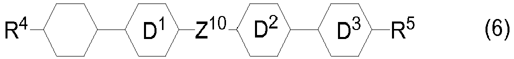 wherein R⁴ and R⁵ are independently straight alkyl of C₁₋₁₀ or straight alkenyl of C₂₋₁₀, and in the alkyl and the alkenyl, arbitrary hydrogen is optionally replaced by fluorine and arbitrary - CH₂- is optionally replaced by -O-; ring D¹, ring D² and ring D³ are independently 1,4-cyclohexylene, pyrimidine-2,5-diyl, 1,4-phenylene, 2-fluoro-1,4-phenylene, 3-fluoro-1,4-phenylene or 2,5-difluoro-1,4-phenylene; and Z¹⁰ is -C≡C-, -COO-, -(CH₂)₂-, -CH=CH- or a single bond.

8. The liquid crystal composition of claim 5, further comprising at least one compound selected from the group consisting of compounds represented by formula (5) of claim 7, and at least one compound selected from the group consisting of compounds represented by formula (6) of claim 7.

9. The liquid crystal composition of any one of claims 5 to 8, further comprising at least one compound selected from the group consisting of compounds represented by formulae (7), (8), (9) and (10), at least one compound selected from the group consisting of compounds represented by formula (11), or at least one compound selected from the group consisting of compounds represented by formulae (7), (8), (9), and (10) and at least one compound selected from the group consisting of compounds represented by formula (11), 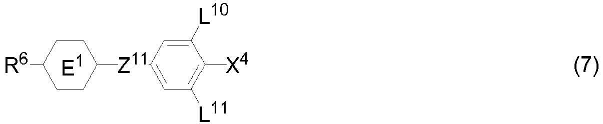 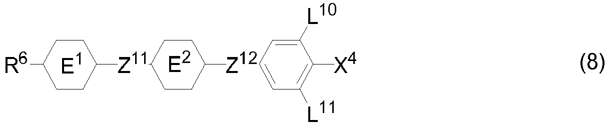 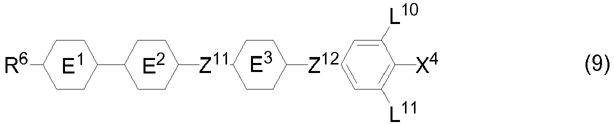 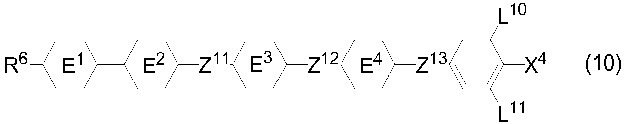 wherein R⁶ is straight alkyl of C₁₋₁₀, straight alkenyl of C₂₋₁₀ or straight alkynyl of C₂₋₁₀, and in the alkyl, the alkenyl and the alkynyl, arbitrary hydrogen is optionally replaced by fluorine and arbitrary -CH₂- is optionally replaced by -O-; X⁴ is fluorine, chlorine, -SF₅, -OCF₃, -OCHF₂, - CF₃, -CHF₂, -CH₂F, -OCF₂CHF₂ or -OCF₂CHFCF₃; ring E¹, ring E², ring E³ and ring E⁴ are independently 1,4-cyclohexylene, 1,3-dioxane-2,5-diyl, pyrimidine-2,5-diyl, tetrahydropyran-2,5-diyl, 1,4-phenylene, naphthalene-2,6-diyl, 1,4-phenylene in which arbitrary hydrogen is replaced by fluorine or chlorine, or naphthalene-2,6-diyl in which arbitrary hydrogen is replaced by fluorine or chlorine; Z¹¹, Z¹² and Z¹³ are independently -(CH₂)₂-, -(CH₂)₄-, -COO-, -CF₂O-, -OCF₂-, -CH=CH-, -C≡C-, -CH₂O- or a single bond; and L¹⁰ and L¹¹ are independently hydrogen or fluorine; 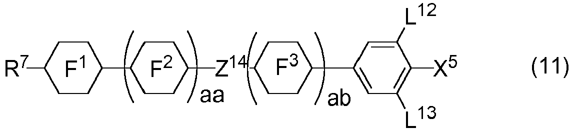 wherein R⁷ is straight alkyl of C₁₋₁₀, straight alkenyl of C₂₋₁₀ or straight alkynyl of C₂₋₁₀, and in the alkyl, the alkenyl and the alkynyl, arbitrary hydrogen is optionally replaced by fluorine and arbitrary -CH₂- is optionally replaced by -O-; X⁵ is -C≡N, -N=C=S or -C≡C-C≡N; ring F¹, ring F² and ring F³ are independently 1,4-cyclohexylene, 1,4-phenylene, 1,4-phenylene in which arbitrary hydrogen is replaced by fluorine or chlorine, naphthalene-2,6-diyl, naphthalene-2,6-diyl in which arbitrary hydrogen is replaced by fluorine or chlorine, 1,3-dioxane-2,5-diyl, tetrahydropyran-2,5-diyl, or pyrimidine-2,5- diyl; Z¹⁴ is -(CH₂)₂-, -COO-, -CF₂O-, -OCF₂-,-C≡C-, -CH₂O- or a single bond; L¹² and L¹³ are independently hydrogen or fluorine; and aa is 0, 1 or 2, ab is 0 or 1, and aa+ab is 0, 1 or 2.

10. The liquid crystal composition of claims 5 or 9, which exhibits a chiral nematic phase at any temperature in a range of 70°C to -20°C and has a helical pitch of 700 nm or less at a temperature in at least a part of the range of 70°C to -20°C.

11. The liquid crystal composition of any one of claims 5 to 10, wherein the chiral dopant includes at least one compound selected from the group consisting of compounds represented by formulae (K1)-(K5), 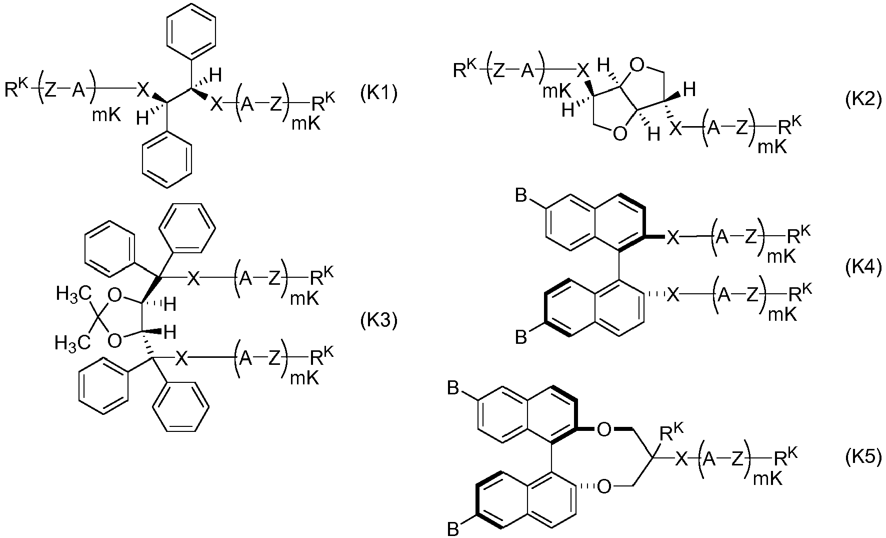
wherein each R^{K} is independently hydrogen, halogen, -C≡N, -N=C=O, -N-C=S, or C₁₋₂₀ alkyl in which arbitrary -CH₂- is optionally replaced by -O-, -S-, -COO- or -OCO-, arbitrary -CH₂-CH₂- is optionally replaced by -CH=CH-, -CF=CF- or -C≡C-, and arbitrary hydrogen is optionally replaced by halogen; each A is independently an aromatic or non-aromatic, three- to eight-membered ring, or a fused ring of 9 or more carbons, and in these rings arbitrary hydrogen is optionally replaced by halogen, C₁₋₃ alkyl or C₁₋₃ haloalkyl, -CH₂- is optionally replaced by -O-, -S- or -NH-, and -CH= is optionally replaced by -N=; each B is independently hydrogen, halogen, C₁₋₃ alkyl, C₁₋₃ haloalkyl, an aromatic or non-aromatic, three- to eight-membered ring, or a fused ring of 9 or more carbons, and in these rings arbitrary hydrogen is optionally replaced by halogen, C₁₋₃ alkyl or C₁₋₃ haloalkyl, -CH₂- is optionally replaced by -O-, -S- or -NH-, and -CH= is optionally replaced by -N=; each Z is independently a single bond, or C₁₋₈ alkylene in which arbitrary -CH₂- is optionally replaced by -O-, -S-, -COO-, -OCO-, -CSO-, -OCS-, - N=N-, -CH=N- or -N=CH-, arbitrary -CH₂-CH₂- is optionally replaced by -CH=CH-, -CF=CF-or -C≡C-, and arbitrary hydrogen is optionally replaced by halogen;
X is a single bond, -COO-, -OCO-, -CH₂O-, -OCH₂-, -CF₂O-, -OCF₂- or -CH₂CH₂-; and
mK is an integer of 1-4.

12. A mixture, comprising the liquid crystal composition of any one of claims 5 to 11, and a polymerizable monomer.

13. A polymer/liquid crystal composite material, being obtained by polymerizing the mixture of claim 12 in a non-liquid crystal isotropic phase or in an optically isotropic liquid crystal phase, and being used in an element driven in an optically isotropic liquid crystal phase.

14. The polymer/liquid crystal composite material of claim 13, wherein a content of the liquid crystal composition is from 70 wt% to 99 wt% and a content of the polymer is from 1 wt% to 30 wt%.

15. An optical element, comprising: a liquid crystal medium disposed between two substrates with electrodes being disposed on a surface of one or both of the substrates, and an electric-field applying means applying an electric field to the liquid crystal medium via the electrodes, wherein the liquid crystal medium is the liquid crystal composition of any one of claims 5 to 11, or the polymer/liquid crystal composite material of any one of claim 13 or 14.

## Patentansprüche

1. Verbindung, die durch die Formel (1) dargestellt wird, 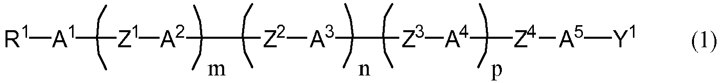 wobei in der Formel (1) das R¹ ein C₄₋₂₀-Alkyl oder -Alkenyl ist, das an einem Kohlenstoff in einer Position 2 davon verzweigt ist; wobei die Ringe A¹, A², A³, A⁴ und A⁵ unabhängig voneinander ein 1,4-Phenylen, ein 1,3-Dioxan-2,5-Diyl, ein Tetrahydropyran-2,5-Diyl, ein Tetrahydropyran-3,6-Diyl, ein Pyrimidin-2,5-Diyl, ein Pyridin-2,5-Diyl oder ein Naphthalin-2,6-Diyl sind, und in diesen Ringen ein beliebiger von dem Wasserstoff gegebenenfalls durch ein Fluor oder ein Chlor ersetzt ist, wobei das Z¹, das Z², das Z³ und das Z⁴ unabhängig voneinander eine Einfachbindung oder ein C₁₋₄-Alkylen sind, bei dem ein beliebiger von dem -CH₂-gegebenenfalls durch ein -O-, ein -COO-, ein -OCO- oder ein -CF₂O- ersetzt ist, ein beliebiger von dem -CH₂-CH₂- gegebenenfalls durch ein -CH=CH-, ein -CF=CF- oder ein -C≡C- ersetzt ist und ein beliebiger von dem Wasserstoff gegebenenfalls durch ein Halogen ersetzt ist; wobei das Y¹ ein Fluor, ein Chlor, ein -SF₅, ein -C≡N, ein -N=C=S oder ein C₁₋₃-Alkyl, wobei ein beliebiger von dem Wasserstoff durch ein Halogen ersetzt ist und in dem Alkyl ein beliebiger von dem -CH2- gegebenenfalls durch ein -O- ersetzt ist und ein beliebiger von dem -CH₂-CH₂- gegebenenfalls durch ein -CH=CH- oder ein -C≡C- ersetzt ist; wobei das m 1 ist, das n 1 ist und das p 0 oder 1 ist.

2. Verbindung nach Anspruch 1, wobei in der Formel (1) ein beliebiger der Ringe A¹, A², A³ und A⁴ ein 1,4-Phenylen ist, bei dem ein oder zwei Wasserstoffe durch ein Fluor ersetzt sind.

3. Verbindung nach Anspruch 1, die durch eine beliebige der Formeln (1-1) bis (1-2) dargestellt wird, 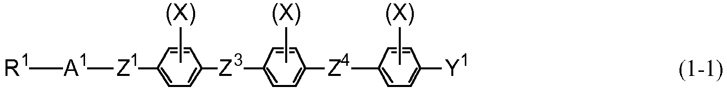 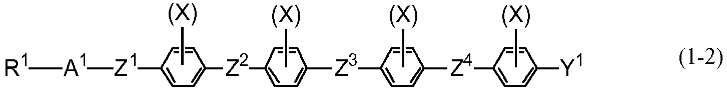 wobei in den Formeln (1-1) bis (1-2) das R¹ ein C₄₋₂₀-Alkyl- oder -Alkenyl ist, das an einem Kohlenstoff in einer Position 2 davon verzweigt ist; wobei der Ring A¹, ein 1,4-Phenylen, ein 1,3-Dioxan-2,5-Diyl, ein Tetrahydropyran-2,5-Diyl, ein Tetrahydropyran-3,6-Diyl, ein Pyrimidin-2,5-Diyl oder ein Pyridin-2,5-Diyl ist, und in diesen Ringen gegebenenfalls ein beliebiger von dem Wasserstoff durch ein Fluor ersetzt ist; wobei das Z¹, das Z², das Z³ und das Z⁴ unabhängig voneinander eine Einfachbindung, ein -CH₂-CH₂-, ein -COO- oder ein -CF₂O- mit der Maßgabe sind, dass ein beliebiger von dem Z¹, dem Z², dem Z³ und dem Z⁴ ein -COO- oder ein -CF₂O- ist; wobei das Y¹ ein Fluor, ein Chlor, ein -C≡N oder ein C₁₋₃-Alkyl ist, wobei ein beliebiger von dem Wasserstoff durch ein Fluor ersetzt ist und in dem Alkyl ein beliebiger von dem -CH₂- gegebenenfalls durch ein -O- ersetzt ist und ein beliebiger von dem -CH₂-CH₂- gegebenenfalls durch ein -CH=CH- ersetzt ist; wobei das X ein Fluor oder ein Chlor ist, wobei ein Symbol für das 1,4-Phenylen und das (X) durch eine gerade Linie verbunden ist, 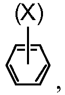 das ein 1,4-Phenylen darstellt, bei dem ein oder zwei Wasserstoffatome gegebenenfalls durch ein X ersetzt sind.

4. Verbindung nach Anspruch 3, die durch eine beliebige der Formeln (1-1-1) und (1-1-2) und der Formeln (1-2-1), (1-2-1), (1-2-9) und (1-2-10) dargestellt wird, 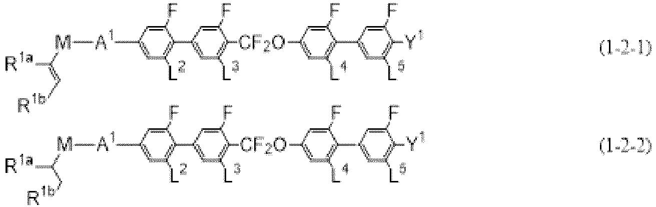 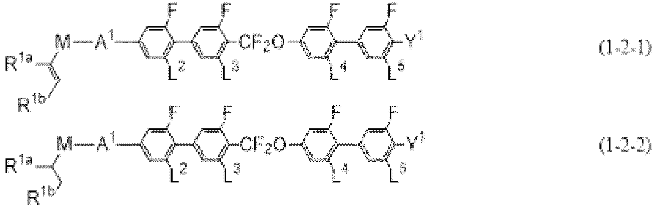 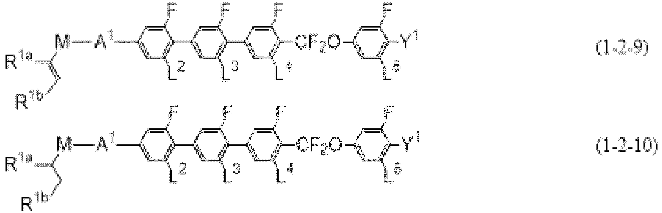 wobei in den Formeln (1-1-1) und (1-1-2) und (1-2-1) (1-2-2), (1-2-9) und (1-2-10) das R^{1a} ein C₁₋₁₀-Alkyl ist, in dem ein beliebiger von dem CH₂-CH₂- gegebenenfalls durch ein -CH=CH- ersetzt ist; wobei das R^{1b} ein Wasserstoff oder ein C₁₋₁₀-Alkyl ist, in dem ein beliebiger von dem -CH₂-CH₂- gegebenenfalls durch ein - CH=CH- ersetzt ist; wobei das M ein -CH₂- ist; wobei das L², das L³, das L⁴ und das L⁵ unabhängig voneinander ein Wasserstoff, ein Fluor oder ein Chlor sind; wobei der Ring A¹ ein 1,4-Phenylen, ein 1,3-Dioxan-2,5-Diyl, ein Tetrahydropyran-2,5-Diyl, ein Tetrahydropyran-3,6-Diyl, ein Pyrimidin-2,5-Diyl oder ein Pyridin-2,5-Diyl ist, und in diesen Ringen gegebenenfalls ein beliebiger von dem Wasserstoff durch ein Fluor ersetzt ist; wobei das Y¹ ein Fluor, ein Chlor, ein -C≡N, ein C₁₋₃-Alkyl, in dem ein beliebiger von dem Wasserstoff durch ein Fluor ersetzt ist, oder ein Alkenyl, in dem ein beliebiger von dem Wasserstoff durch ein Fluor ersetzt ist, oder ein Alkoxy ist, in dem ein beliebiger von dem Wasserstoff durch ein Fluor ersetzt ist.

5. Flüssigkristallzusammensetzung, welche die Verbindung nach einem der Ansprüche 1 bis 4 und einen chiralen Dotierstoff aufweist und welche eine optische isotrope Flüssigkristallphase zeigt.

6. Flüssigkristallzusammensetzung nach Anspruch 5, welche weiterhin mindestens eine Verbindung aufweist, die aus der Gruppe ausgewählt worden ist, die die Verbindungen umfasst, die durch die Formeln (2), (3) und (4) dargestellt werden, 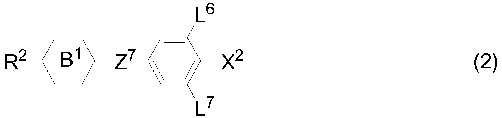 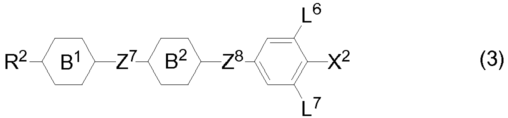 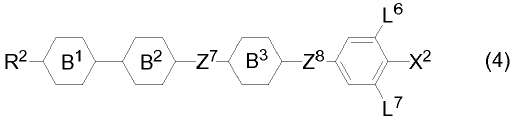 wobei das R² ein geradkettiges C₁₋₁₀-Alkyl oder ein geradkettiges C₂₋₁₀-Alkenyl ist, und in dem Alkyl und dem Alkenyl ein beliebiger von dem Wasserstoff gegebenenfalls durch ein Fluor ersetzt ist und ein beliebiger von dem -CH₂- gegebenenfalls durch ein -O- ersetzt ist; wobei das X² ein Fluor, ein Chlor, ein -OCF₃, ein -OCHF₂, ein -CF₃, ein -CHF₂, ein -CH₂F, ein -OCF₂CHF₂ oder ein -OCF₂CHFCF₃ ist; wobei der Ring B¹, der Ring B² und der Ring B³ unabhängig voneinander ein 1,4-Cyclohexylen, ein 1,3-Dioxan-2,5-Diyl, ein Pyrimidin-2,5-Diyl, ein Tetrahydropyran-2,5-Diyl, ein 1,4-Phenylen, ein Naphthalin-2,6-Diyl, ein 1,4-Phenylen sind, in dem beliebiger Wasserstoff durch Fluor ersetzt ist, oder Naphthalin-2,6-diyl, in dem ein beliebiger von dem Wasserstoff durch ein Fluor oder ein Chlor ersetzt ist; wobei das Z⁷ und das Z⁸ unabhängig voneinander ein -(CH₂)₂-, ein -(CH₂)₄-, ein -COO-, ein-CF₂O-, ein -OCF₂-, ein -CH=CH-, ein -C≡C-, ein -CH₂O- oder eine Einfachbindung sind; wobei das L⁶ und das L⁷ unabhängig voneinander ein Wasserstoff oder ein Fluor sind.

7. Flüssigkristallzusammensetzung nach Anspruch 5 oder 6, welche weiterhin mindestens eine Verbindung, die aus der Gruppe ausgewählt worden ist, die die Verbindungen umfasst, die durch die Formel (5) dargestellt werden, oder mindestens eine Verbindung aufweist, die aus der Gruppe ausgewählt worden ist, die die Verbindungen umfasst, die durch die Formel (6) werden, 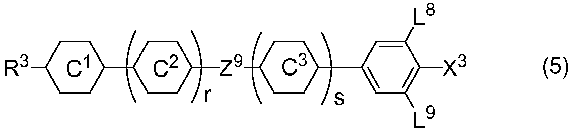 wobei das R³ ein geradkettiges C₁₋₁₀-Alkyl oder ein geradkettiges C₂₋₁₀-Alkenyl ist und in dem Alkyl und dem Alkenyl ein beliebiger von dem Wasserstoff gegebenenfalls durch ein Fluor und ein beliebiger von dem -CH₂- gegebenenfalls durch ein -O- ersetzt ist; wobei das X³ ein -C≡N oder ein -C≡C-C≡N ist; wobei der Ring C¹, der Ring C² und der Ring C³ unabhängig voneinander ein 1,4-Cyclohexylen, ein 1,4-Phenylen, ein 1,4-Phenylen sind, wobei ein beliebiger von dem Wasserstoff durch ein Fluor, ein Naphthalin-2,6-Diyl, ein Naphthalin-2,6-Diyl ersetzt ist, in dem ein beliebiger von dem Wasserstoff durch ein Fluor oder ein Chlor, ein 1,3-Dioxan-2,5-Diyl, ein Tetrahydropyran-2,5-Diyl oder ein Pyrimidin-2,5-Diyl ersetzt ist; wobei das Z⁹ ein -(CH₂)₂-, ein -COO-, ein -CF₂O-, ein -OCF₂-, ein -C≡C-, ein -CH₂O- oder eine Einfachbindung ist; wobei das L⁸ und das L⁹ unabhängig voneinander ein Wasserstoff oder ein Fluor sind; und wobei das r 1 oder 2 ist, das s 0 oder 1 ist und r+s 0, 1 oder 2 ist, 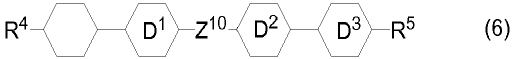 wobei das R⁴ und das R⁵ unabhängig voneinander ein geradkettiges C₁₋₁₀-Alkyl oder ein geradkettiges C₂₋₁₀-Alkenyl sind und in dem Alkyl und dem Alkenyl ein beliebiger von dem Wasserstoff gegebenenfalls durch ein Fluor ersetzt ist und ein beliebiger von dem -CH₂- gegebenenfalls durch ein -O- ersetzt ist; wobei der Ring D¹, der Ring D² und der Ring D³ unabhängig voneinander ein 1,4-Cyclohexylen, ein Pyrimidin-2,5-Diyl, 1,4-Phenylen, ein 2-Fluor-1,4-Phenylen, ein 3-Fluor-1,4-Phenylen oder ein 2,5-Difluor-1,4-Phenylen sind; und wobei das Z¹⁰ ein -C≡C-, ein -COO-, ein -(CH₂)₂-, ein -CH=CH- oder eine Einfachbindung ist.

8. Flüssigkristallzusammensetzung nach Anspruch 5, welche ferner mindestens eine Verbindung, die aus der Gruppe ausgewählt worden ist, die die Verbindungen umfasst, die durch die Formel (5) des Anspruches 7 dargestellt werden, und mindestens eine Verbindung aufweist, die aus der Gruppe ausgewählt worden ist, die die Verbindungen umfasst, die durch die Formel (6) des Anspruches 7 dargestellt werden.

9. Flüssigkristallzusammensetzung nach einem der Ansprüche 5 bis 8, welche ferner mindestens eine Verbindung, die aus der Gruppe ausgewählt worden ist, die die Verbindungen umfasst, die durch die Formeln (7), (8), (9) und (10) dargestellt werden, und mindestens eine Verbindung aufweist, die aus der Gruppe ausgewählt worden ist, die die Verbindungen umfasst, die durch die Formel (11) dargestellt werden, oder welche mindestens eine Verbindung, die aus der Gruppe ausgewählt worden ist, die die Verbindungen umfasst, die durch die Formeln (7), (8), (9) und (10) dargestellt werden, und mindestens eine Verbindung aufweist, die aus der Gruppe ausgewählt worden ist, die die Verbindungen umfasst, die durch die Formel (11) dargestellt werden, 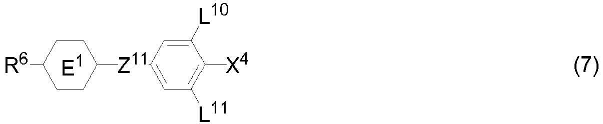 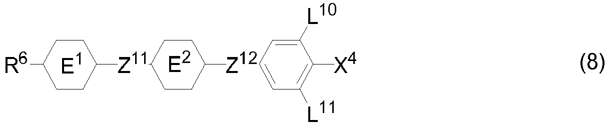 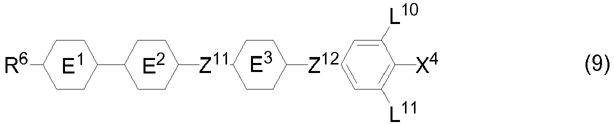 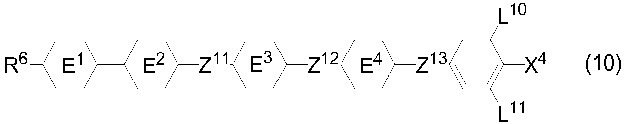 wobei das R⁶ ein geradkettiges C₁₋₁₀-Alkyl, ein geradkettiges C₂₋₁₀-Alkenyl oder ein geradkettiges C₂₋₁₀-Alkinyl ist und in dem Alkyl, dem Alkenyl und dem Alkinyl ein beliebiger von dem Wasserstoff gegebenenfalls durch ein Fluor ersetzt ist und ein beliebiger von dem -CH₂- durch ein -O- ersetzt ist; wobei das X⁴ ein Fluor, ein Chlor, ein -SF₅, ein -OCF₃, ein -OCHF₂, ein -CF₃, ein -CHF₂, ein -CH₂F, ein -OCF₂CHF₂ oder ein -OCF2CHFCF3 ist; wobei der Ring E¹, der Ring E², der Ring E³ und der Ring E⁴ unabhängig voneinander ein 1,4-Cyclohexylen, ein 1,3-Dioxan-2,5-Diyl, ein Pyrimidin-2,5-Diyl, ein Tetrahydropyran-2,5-Diyl, ein 1,4-Phenylen, ein Naphthalin-2,6-Diyl, ein 1,4-Phenylen sind, wobei ein beliebiger von dem Wasserstoff durch ein Fluor oder ein Chlor oder ein Naphthalin-2,6-Diyl ersetzt ist, in dem ein beliebiger von dem Wasserstoff durch ein Fluor oder ein Chlor ersetzt ist; wobei das Z¹¹, das Z¹² und das Z¹³ unabhängig voneinander ein -(CH₂)₂-, ein -(CH₂)₄-, ein -COO-, ein -CF₂O-, ein -OCF₂-, ein -CH=CH-, ein -C≡C-, ein -CH₂O- oder eine Einfachbindung sind; und das L¹⁰ und das L¹¹ unabhängig voneinander ein Wasserstoff oder ein Fluor sind; 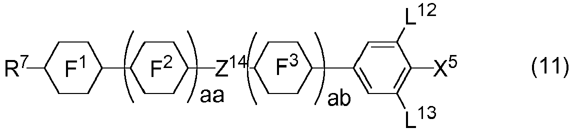 wobei das R⁷ ein geradkettiges C₁₋₁₀-Alkyl, ein geradkettiges C₂₋₁₀-Alkenyl oder ein geradkettiges C₂₋₁₀-Alkinyl ist und in dem Alkyl, dem Alkenyl und dem Alkinyl ein beliebiger von dem Wasserstoff gegebenenfalls durch ein Fluor ersetzt ist und ein beliebiger von dem -CH₂- durch ein -O- ersetzt ist; wobei das X⁵ ein -C≡N, ein -N=C=S oder ein -C≡C-C≡N ist; wobei der Ring F¹, der Ring F² und der Ring F³ unabhängig voneinander ein 1,4-Cyclohexylen, ein 1,4-Phenylen, ein 1,4-Phenylen, in dem ein beliebiger von dem Wasserstoff durch ein Fluor oder ein Chlor ersetzt ist, ein Naphthalin-2,6-Diyl, ein Naphthalin-2,6-Diyl, in dem ein beliebiger von dem Wasserstoff durch ein Fluor oder ein Chlor ersetzt ist, ein 1,3-Dioxan-2,5-Diyl, ein Tetrahydropyran-2,5-Diyl oder ein Pyrimidin-2,5-Diyl sind; wobei das Z¹⁴ ein -(CH₂)₂-, ein -COO-, ein -CF₂O-, ein -OCF₂-, ein -C≡C-, -CH₂O- oder eine Einfachbindung ist; wobei das L¹² und das L¹³ unabhängig voneinander ein Wasserstoff oder ein Fluor sind; und das aa 0, 1 oder 2 ist, das ab 0 oder 1 ist und aa+ab ist 0, 1 oder 2.

10. Flüssigkristallzusammensetzung nach Anspruch 5 oder 9, welche eine chirale nematische bei jeder Temperatur in einem Bereich von 70 °C bis -20 °C Phase aufweist und welche bei einer Temperatur in mindestens einem Teil des Bereichs von 70 °C bis -20 °C eine helikale Ganghöhe von 700 nm oder weniger aufweist.

11. Flüssigkristallzusammensetzung nach einem der Ansprüche 5 bis 10, wobei der chirale Dotierstoff mindestens eine Verbindung enthält, die aus der Gruppe ausgewählt worden ist, die aus den Verbindungen besteht, die durch die Formeln (K1) bis (K5) dargestellt werden, 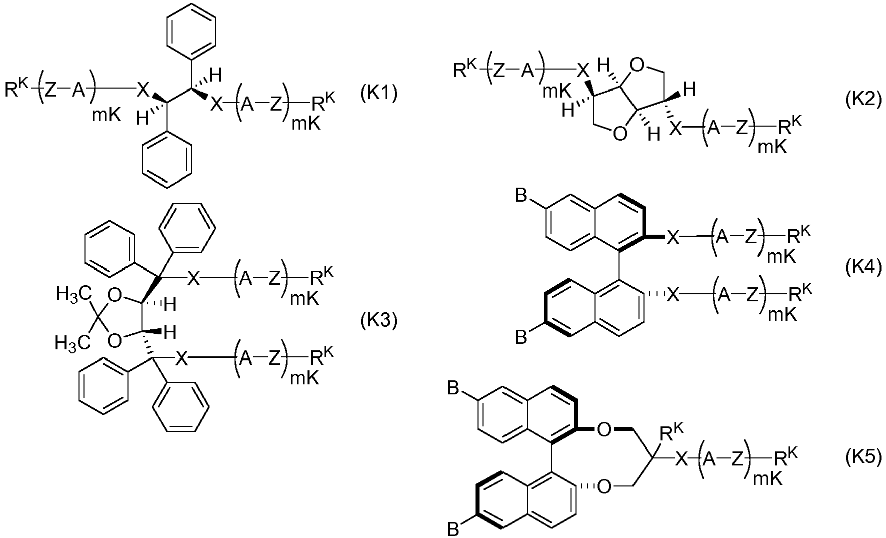
wobei jedes R^{K} unabhängig voneinander ein Wasserstoff, ein Halogen, ein -C≡N, ein -N=C=O, ein -NC=S oder ein C₁₋₂₀-Alkyl ist, in dem ein beliebiger von dem -CH₂- gegebenenfalls durch ein -O-, ein -S-, ein -COO- oder ein -OCO- ersetzt ist, ein beliebiger von dem -CH₂-CH₂- gegebenenfalls durch ein -CH=CH-, ein -CF=CF- oder ein -C≡C- ersetzt ist, und ein beliebiger von dem Wasserstoff gegebenenfalls durch ein Halogen ersetzt ist; wobei jedes A unabhängig voneinander ein aromatischer oder nichtaromatischer, drei- bis achtgliedriger Ring oder ein kondensierter Ring mit 9 oder mehr Kohlenstoffen ist, und in diesen Ringen ein beliebiger von dem Wasserstoff gegebenenfalls durch ein Halogen, ein C₁₋₃-Alkyl oder ein C₁₋₃-Haloalkyl ersetzt ist, das -CH2- gegebenenfalls durch ein -O-, ein -S- oder ein -NH- ersetzt ist und das -CH= gegebenenfalls durch ein -N= ersetzt ist; wobei jedes B unabhängig voneinander ein Wasserstoff, ein Halogen, ein C₁₋₃-Alkyl, ein C₁₋₃-Haloalkyl, ein aromatischer oder nichtaromatischer, drei- bis achtgliedriger Ring oder ein kondensierter Ring mit 9 oder mehr Kohlenstoffen ist, und in diesen Ringen ein beliebiger von dem Wasserstoff gegebenenfalls durch ein Halogen, ein C₁₋₃-Alkyl oder ein C₁₋₃-Haloalkyl ersetzt ist, das -CH₂- gegebenenfalls durch ein -O-, ein -S- oder ein -NH- ersetzt ist und das -CH= gegebenenfalls durch ein -N= ersetzt ist; wobei jedes Z unabhängig voneinander eine Einfachbindung oder ein C₁₋₈-Alkylen ist, in dem ein beliebiger von dem -CH₂- gegebenenfalls durch ein -O-, ein -S-, ein - COO-, ein -OCO-, ein -CSO-, ein -OCS-, ein -N=N-, ein -CH=N- oder ein -N=CH-ersetzt ist, ein beliebiger von dem -CH₂-CH₂- gegebenenfalls durch ein -CH=CH-, ein -CF=CF- oder ein -C≡C- ersetzt ist und ein beliebiger von dem Wasserstoff gegebenenfalls durch ein Halogen ersetzt ist;
wobei das X eine Einfachbindung, ein -COO-, ein -OCO-, ein -CH₂O-, ein -OCH₂-, ein -CF₂O-, -OCF₂- oder ein -CH₂CH₂- ist; und
wobei das mK eine ganze Zahl von 1 bis 4 ist.

12. Mischung, die die Flüssigkristallzusammensetzung nach einem der Ansprüche 5 bis 11 und ein polymerisierbares Monomer aufweist.

13. Polymer/Flüssigkristall-Verbundmaterial, das durch ein Polymerisieren der Mischung nach Anspruch 12 in einer nicht-flüssigen kristallinen isotropen Phase oder in einer optisch isotropen Flüssigkristallphase erhalten wird und in einem Element verwendet wird, das in einer optisch isotropen Flüssigkristallphase betrieben wird.

14. Polymer/Flüssigkristall-Verbundmaterial nach Anspruch 13, wobei ein Anteil der Flüssigkristallzusammensetzung 70 Gewichtsprozent bis 99 Gewichtsprozent und ein Anteil des Polymers 1 Gewichtsprozent bis 30 Gewichtsprozent beträgt.

15. Optisches Element, das ein Flüssigkristallmedium, das zwischen zwei Substraten angeordnet ist, wobei Elektroden auf einer Oberfläche eines oder beider Substrate angeordnet sind, und eine Einrichtung zum Anlegen eines elektrischen Feldes an das Flüssigkristallmedium über die Elektroden aufweist, wobei das Flüssigkristallmedium die Flüssigkristallzusammensetzung nach einem der Ansprüche 5 bis 11 oder das Polymer/Flüssigkristall-Verbundmaterial nach einem der Ansprüche 13 oder 14 ist.

## Revendications

1. Composé représenté par la formule (1), 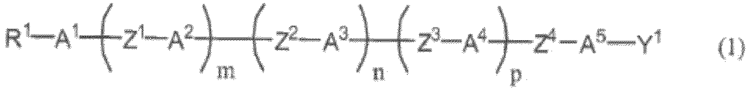 dans lequel dans la formule (1), R¹ est un alkyle C₄₋₂₀ ou un alcényle ramifié sur un carbone en position 2 de celui-ci ; les anneaux A¹, A², A³, A⁴ et A⁵ sont de façon indépendante le 1,4-phénylène, le 1,3-dioxane-2,5-diyl, le tétrahydropyrane-2,5-diyl, le tétrahydropyrane-3,6-diyl, la pyrimidine-2,5-diyl, la pyridine-2,5-diyl ou le naphtalène-2,6-diyl, et dans ces anneaux l'hydrogène arbitraire est remplacé optionnellement par le fluor ou le chlore, Z¹, Z², Z³ et Z⁴ sont de façon indépendante une liaison unique, ou un alkylène C₁₋₄ dans lequel le -CH₂- arbitraire est optionnellement remplacé par -O-, -COO-, -OCO- ou -CF₂O-, le -CH₂-CH₂- arbitraire est optionnellement remplacé par -CH=CH-, -CF=CF- ou -C≡C-, et l'hydrogène arbitraire est optionnellement remplacé par un halogène ; Y¹ est le fluor, le chlore, -SF5, -C≡N, -N=C=S, ou un alkyle C₁₋₃ dans lequel dans lequel l'hydrogène arbitraire est remplacé par un halogène, et dans l'alkyle le -CH₂- arbitraire est optionnellement remplacé par -O-, et le -CH₂-CH₂- arbitraire est optionnellement remplacé par -CH=CH- ou -C≡C- ; m est 1, n est 1, et p est 0 ou 1.

2. Composé selon la revendication 1, dans lequel dans la formule (1), arbitrairement l'un des anneaux A¹, A², A³ et A⁴ est le 1,4-phénylène dans lequel un ou deux hydrogènes sont remplacés par le fluor.

3. Composé selon la revendication 1, représenté par arbitrairement l'une des formules (1-1) à (1-2), 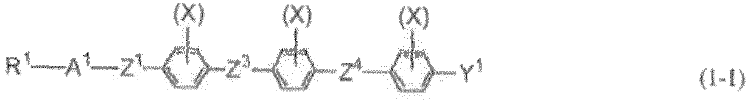 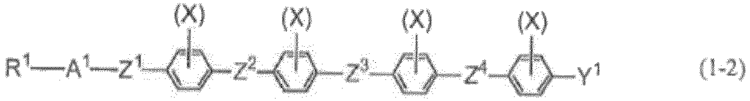 dans lequel dans les formules (1-1) à (1-2), R¹ est un alkyle C₄₋₂₀ ou un alcényle ramifié sur un carbone en position 2 de celui-ci ; l'anneau A¹ est le 1,4-phénylène, le 1,3-dioxane-2,5-diyl, le tétrahydropyrane-2,5-diyl, le tétrahydropyrane-3,6-diyl, la pyrimidine-2,5-diyl ou la pyridine-2,5-diyl, et dans ces anneaux l'hydrogène arbitraire est optionnellement remplacé par le fluor ; Z¹, Z², Z³ et Z⁴ sont de façon indépendante une liaison unique, -CH₂-CH₂-, -COO- ou -CF₂O-, sachant que arbitrairement l'un de Z¹, Z², Z³ et Z⁴ est -COO- ou -CF₂O- ; Y¹ est le fluor, le chlore, -C≡N, ou un alkyle C₁₋₃ dans lequel l'hydrogène arbitraire est remplacé par le fluor, et dans l'alkyle le -CH₂- arbitraire est optionnellement remplacé par -O- et le -CH₂-CH₂- arbitraire est optionnellement remplacé par -CH=CH- ; X est le fluor ou le chlore, dans lequel un symbole du 1,4-phénylène et (X) sont connectés par un trait droit, 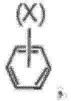 représente le 1,4-phénylène dans lequel un ou deux hydrogènes sont optionnellement remplacés par X.

4. Composé selon la revendication 3, représenté par arbitrairement l'une des formules (1-1-1) et (1-1-2) et les formules (1-2-1), (1-2-1), (1-2-9) et (1-2-10), 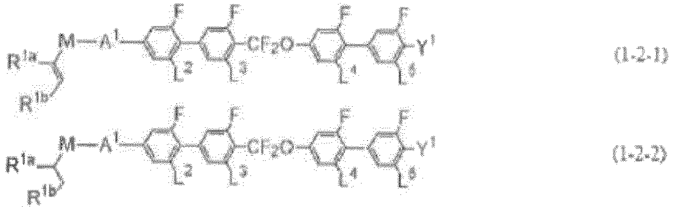 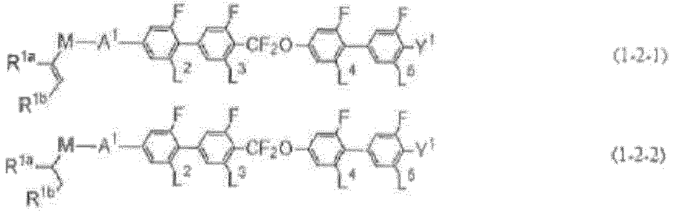 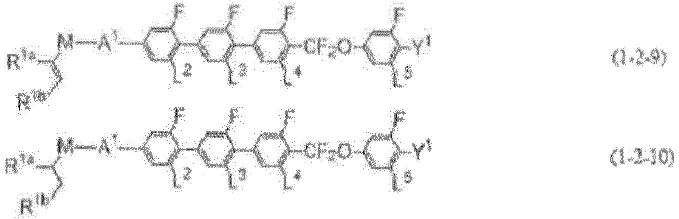 dans lequel dans les formules (1-1-1) et (1-1-2) et les formules (1-2-1), (1-2-2), (1-2-9) et (1-2-10), R^{1a} est un alkyle C₁₋₁₀ dans lequel le -CH₂-CH₂- arbitraire est optionnellement remplacé par -CH=CH- ; R^{1b} est l'hydrogène, ou un alkyle C₁₋₁₀ dans le -CH₂-CH₂- est optionnellement remplacé par -CH=CH- ; M est -CH₂- ; L², L³, L⁴ et L⁵ sont de façon indépendante l'hydrogène, le fluor ou le chlore ; l'anneau A¹ est le 1,4-phénylène, le 1,3-dioxane-2,5-diyl, le tétrahydropyrane-2,5-diyl, le tétrahydropyrane-3,6-diyl, la pyrimidine-2,5-diyl ou la pyridine-2,5-diyl, et dans ces anneaux, l'hydrogène arbitraire est optionnellement remplacé par le fluor ; Y¹ est le fluor, le chlore, -C≡N, un alkyle C₁₋₃ dans lequel l'hydrogène arbitraire est remplacé par le fluor, un alcényle dans lequel l'hydrogène arbitraire est remplacé par le fluor, ou un alkoxy dans lequel l'hydrogène arbitraire est remplacé par le fluor.

5. Composition de cristaux liquides, comprenant le composé selon l'une quelconque des revendications 1 à 4 et un dopant chiral, et présentant une phase de cristal liquide optiquement isotropique.

6. Composition de cristaux liquides selon la revendication 5, comprenant en outre au moins un composé sélectionné dans le groupe consistant en les composés représentés par les formules (2), (3) et (4), 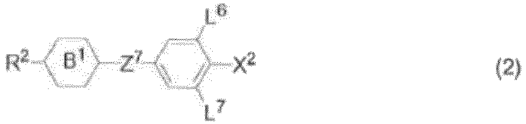 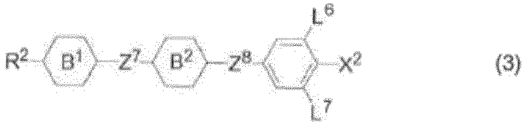 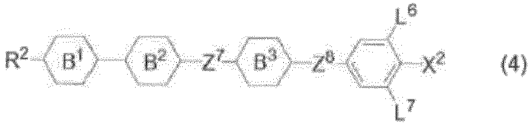 dans laquelle R² est un alkyle droit de C₁₋₁₀ ou un alcényle droit de C₂₋₁₀, et dans l'alkyle et l'alcényle, l'hydrogène arbitraire est optionnellement remplacé par le fluor et le -CH₂- arbitraire est optionnellement remplacé par -O- ; X² est le fluor, le chlore, -OCF₃, -OCHF₂, -CF₃, -CHF₂, -CH₂F, -OCF₂CHF₂ ou -OCF₂CHFCF₃ ; l'anneau B¹, l'anneau B² et l'anneau B³ sont de façon indépendante le 1,4-cyclohexylène, le 1,3-dioxane-2,5-diyl, la pyrimidine-2,5-diyl, le tétrahydropyrane-2,5-diyl, le 1,4-phénylène, le naphtalène-2,6-diyl, le 1,4-phénylène dans lequel l'hydrogène arbitraire est remplacé par le fluor, ou le naphtalène-2,6-diyl dans lequel l'hydrogène arbitraire est remplacé par le fluor ou le chlore ; Z⁷ et Z⁸ sont de façon indépendante -(CH₂)₂-, -(CH₂)₄-, -COO-, -CF₂O-, -OCF₂-, -CH=CH-, -C≡C-, -CH₂O- ou une liaison unique ; L⁶ et L⁷ sont de façon indépendante l'hydrogène ou le fluor.

7. Composition de cristaux liquides selon les revendications 5 ou 6, comprenant en outre au moins un composé sélectionné dans le groupe consistant en les composés représentés par la formule (5), ou au moins un composé sélectionné dans le groupe consistant en les composés représentés par la formule (6), 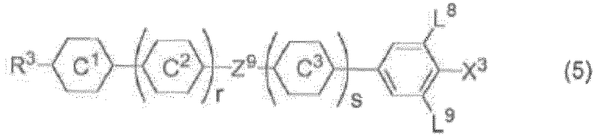 dans laquelle R³ est un alkyle droit de C₁₋₁₀ ou un alcényle droit de C₂₋₁₀ et dans l'alkyle et l'alcényle, l'hydrogène arbitraire est optionnellement remplacé par le fluor et le -CH₂- arbitraire est optionnellement remplacé par -O- ; X³ est -C≡N ou -C≡C-C≡N ; l'anneau C¹, l'anneau C² et l'anneau C³ sont de façon indépendante le 1,4-cyclohexylène, le 1,4-phénylène, le 1,4-phénylène dans lequel l'hydrogène arbitraire est remplacé par le fluor, le naphtalène-2,6-diyl, le naphtalène-2,6-diyl dans lequel l'hydrogène arbitraire est remplacé par le fluor ou le chlore, le 1,3-dioxane-2,5-diyl, le tétrahydropyrane-2,5-diyl ou la pyrimidine-2,5-diyl ; Z⁹ est -(CH₂)₂-, -COO-, -CF₂O-, -OCF₂-, -C≡C-, -CH₂O- ou une liaison unique ; L⁸ et L⁹ sont de façon indépendante l'hydrogène ou le fluor ; et r est 1 ou 2, s est 0 ou 1, et r+s est 0, 1 ou 2, 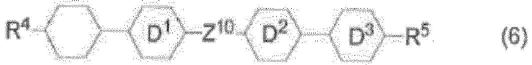 dans laquelle R⁴ et R⁵ sont de façon indépendante un alkyle droit de C₁₋₁₀ ou un alcényle droit de C₂₋₁₀, et dans l'alkyle et l'alcényle, l'hydrogène arbitraire est optionnellement remplacé par le fluor et le -CH₂- arbitraire est optionnellement remplacé par -O- ; l'anneau D¹, l'anneau D² et l'anneau D³ sont de façon indépendante le 1,4-cyclohexylène, la pyrimidine-2,5-diyl, le 1,4-phénylène, le 2-fluoro-1,4-phénylène, le 3-fluoro-1,4-phénylène ou le 2,5-difluoro-1,4-phénylène ; et Z¹⁰ est -C≡C-, -COO-, -(CH₂)₂-, -CH=CH- ou une liaison unique.

8. Composition de cristaux liquides selon la revendication 5, comprenant en outre au moins un composé sélectionné dans le groupe consistant en les composés représentés par la formule (5) de la revendication 7, et au moins un composé sélectionné dans le groupe consistant en les composés représentés par la formule (6) de la revendication 7.

9. Composition de cristaux liquides selon l'une quelconque des revendications 5 à 8, comprenant en outre au moins un composé sélectionné à partir du groupe consistant en les composés représentés par les formules (7), (8), (9) et (10), au moins un composé sélectionné dans le groupe consistant en les composés représentés par la formule (11), ou au moins un composé sélectionné dans le groupe consistant en les composés représentés par les formules (7), (8), (9) et (10) et au moins un composé sélectionné dans le groupe consistant en les composés représentés par la formule (11), 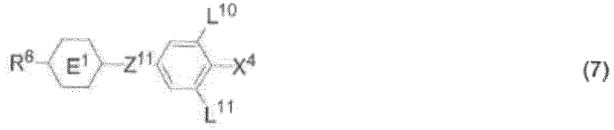 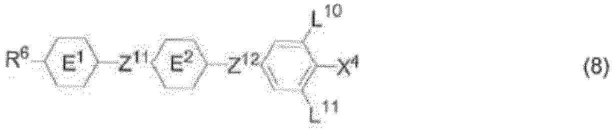 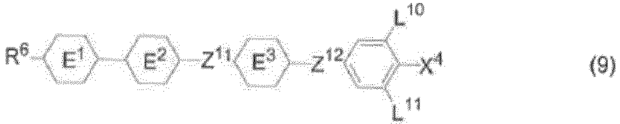 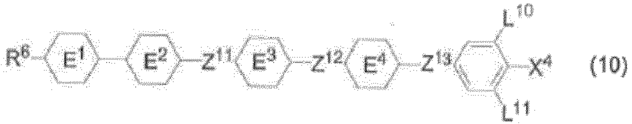 dans laquelle R⁶ est un alkyle droit de C₁₋₁₀, un alcényle droit de C₂₋₁₀ ou un alkynyle droit de C₂₋₁₀, et dans l'alkyle, l'alcényle et l'alkynyle, l'hydrogène arbitraire est optionnellement remplacé par le fluor et le -CH₂- arbitraire est optionnellement remplacé par -O- ; X⁴ est le fluor, le chlore, -SF₅, -OCF₃, -OCHF₂, -CF₃, -CHF₂, -CH₂F, -OCF₂CHF₂ ou -OCF₂CHFCF₃ ; l'anneau E¹, l'anneau E², l'anneau E³ et l'anneau E⁴ sont de façon indépendante le 1,4-cyclohexylène, le 1,3-dioxane-2,5-diyl, la pyrimidine-2,5-diyl, le tétrahydropyrane-2,5-diyl, le 1,4-phénylène, le naphtalène-2,6-diyl, le 1,4-phénylène dans lequel l'hydrogène arbitraire est remplacé par le fluor ou le chlore, ou le naphtalène-2,6-diyl dans lequel l'hydrogène arbitraire est remplacé par le fluor ou le chlore ; Z¹¹, Z¹² et Z¹³ sont de façon indépendante -(CH₂)₂-, -(CH₂)₄-, -COO-, -CF₂O-, -OCF₂-, -CH=CH-, -C≡C-, -CH₂O- ou une liaison unique ; et L¹⁰ et L¹¹ sont de façon indépendante l'hydrogène ou le fluor ; 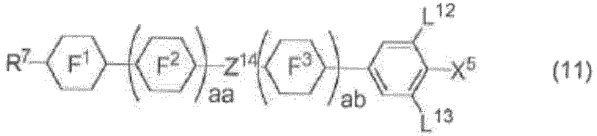 dans laquelle R⁷ est un alkyle droit de C₁₋₁₀, un alcényle droit de C₂₋₁₀ ou un alkynyle droit de C₂₋₁₀, et dans l'alkyle, l'alcényle et l'alkynyle, l'hydrogène arbitraire est optionnellement remplacé par le fluor et le -CH₂- arbitraire est optionnellement remplacé par -O- ; X⁵ est -C≡N, -N=C=S ou -C≡C-C≡N ; l'anneau F¹, l'anneau F² et l'anneau F³ sont de façon indépendante le 1,4-cyclohexylène, le 1,4-phénylène, le 1,4-phénylène dans lequel l'hydrogène arbitraire est remplacé par le fluor ou le chlore, le naphtalène-2,6-diyl, le naphtalène-2,6-diyl dans lequel l'hydrogène arbitraire est remplacé par le fluor ou le chlore, le 1,3-dioxane-2,5-diyl, le tétrahydropyrane-2,5-diyl, ou la pyrimidine-2,5-diyl ; Z¹⁴ est -(CH₂)₂-, -COO-, -CF₂O-, -OCF₂-, -C≡C-, -CH₂O- ou une liaison unique ; L¹² et L¹³ sont de façon indépendante l'hydrogène ou le fluor ; et aa est 0, 1 ou 2, ab est 0 ou 1, et aa+ab est 0, 1 ou 2.

10. Composition de cristaux liquides selon les revendications 5 ou 9, qui présente une phase nématique chirale à toute température dans une plage de 70°C à -20°C et a un pas hélicoïdal de 700 nm ou moins à une température dans au moins une partie de la plage de 70°C à -20°C.

11. Composition de cristaux liquides selon l'une quelconque des revendications 5 à 10, dans laquelle le dopant chiral inclut au moins un composé sélectionné dans le groupe consistant en les composés représentés par les formules (K1)-(K5), 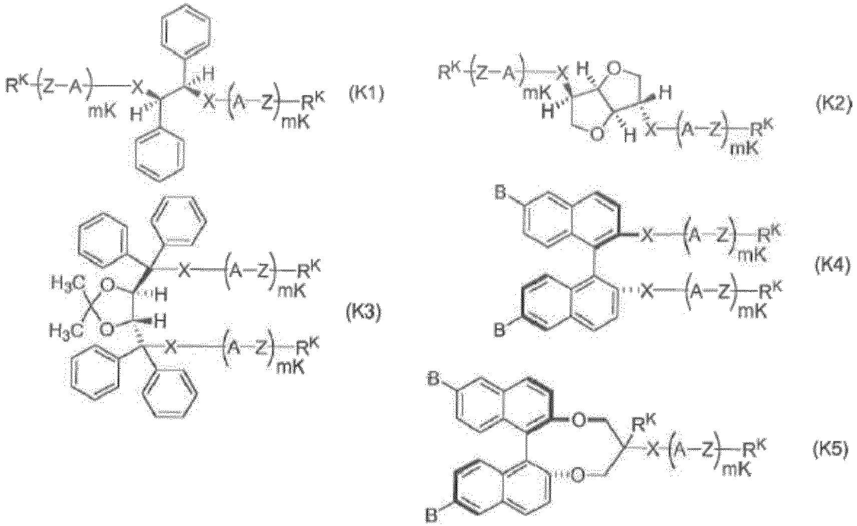
dans laquelle chaque R^{K} est de façon indépendante l'hydrogène, un halogène, -C≡N, -N=C=O, -N-C=S, ou un alkyle C₁₋₂₀ dans lequel le -CH₂- arbitraire est optionnellement remplacé par -O-, -S-, -COO- ou -OCO-, le -CH₂-CH₂- arbitraire est optionnellement remplacé par -CH=CH-, -CF=CF- ou -C≡C-, et un hydrogène arbitraire est optionnellement remplacé par un halogène ; chaque A est de façon indépendante un anneau aromatique ou non aromatique, de trois à huit membrures, ou un anneau fusionné de 9 carbones ou plus, et dans ces anneaux l'hydrogène arbitraire est optionnellement remplacé par l'halogène, un alkyle C₁₋₃ ou un haloalkyle C₁₋₃, -CH₂- est optionnellement remplacé par -O-, -S- ou -NH-, et -CH= est optionnellement remplacé par -N= ; chaque B est de façon indépendante l'hydrogène, un halogène, un alkyle C₁₋₃, un haloalkyle C₁₋₃, un anneau aromatique ou non aromatique, de trois à huit membrures, ou un anneau fusionné de 9 carbones ou plus, et dans ces anneaux l'hydrogène arbitraire est optionnellement remplacé par un halogène, un alkyle C₁₋₃ ou un haloalkyle C₁₋₃, -CH₂- est optionnellement remplacé par -O-, -S- ou -NH-, et -CH= est optionnellement remplacé par -N= ; chaque Z est de façon indépendante une liaison unique, ou un alkylène C₁₋₈ dans lequel le -CH₂- arbitraire est optionnellement remplacé par -O-, -S-, -COO-, -OCO-, -CSO-, -OCS-, -N=N-, -CH=N- ou -N= CH-, le -CH₂-CH₂- arbitraire est optionnellement remplacé par -CH=CH-, -CF=CF- ou -C≡C-, et l'hydrogène arbitraire est optionnellement remplacé par un halogène ;
X est une liaison unique, -COO-, -OCO-, -CH₂O-, -OCH₂-, -CF₂O-, -OCF₂- ou -CH₂CH₂- ; et
mK est un entier de 1-4.

12. Mélange, comprenant la composition de cristaux liquides selon l'une quelconque des revendications 5 à 11, et monomère polymérisable.

13. Matériau composite polymère/à cristaux liquides, étant obtenu en polymérisant le mélange selon la revendication 12 en une phase isotropique sans cristaux liquides ou en une phase à cristaux liquides optiquement isotropique, et étant utilisé dans un élément entraîné dans une phase à cristaux liquides optiquement isotropique.

14. Matériau composite polymère/à cristaux liquides selon la revendication 13, dans lequel un contenu de la composition de cristaux liquides est de 70% en poids à 99% en poids et un contenu du polymère est de 1% en poids à 30% en poids.

15. Élément optique, comprenant : un médium à cristaux liquides disposé entre deux substrats avec des électrodes disposées sur une surface d'un des substrats ou des deux, et des moyens d'application d'un champ électrique appliquant un champ électrique au médium de cristaux liquides via les électrodes, dans lequel le médium de cristaux liquides est la composition de cristaux liquides selon l'une quelconque des revendications 5 à 11, ou le matériau composite polymère/à cristaux liquides selon l'une quelconque des revendications 13 ou 14.
